# EUROPEAN PATENT APPLICATION

(11) **EP 4 046 631 A1**
(43) Date of publication of application: **24.08.2022**
(21) Application number: 21382133.3
(22) Date of filing: 18.02.2021
(51) Int. Cl.: A61K 31/00, A61K 31/7105, A61K 31/711, A61K 31/7125, A61K 31/713, C07H 21/02, C07H 21/04

(54) **MSI2 AS A THERAPEUTIC TARGET FOR THE TREATMENT OF MYOTONIC DYSTROPHY**

(71) Applicant: Universitat de València, 46010 Valencia (ES); Fundación para la Investigación del Hospital Clínico de la Comunidad Valenciana (INCLIVA), 46010 Valencia (ES)
(72) Inventor: SABATER ARCÍS, María, 46100 Burjassot (ES); ARTERO ALLEPUZ, Rubén Darío, 46100 Burjassot (ES); BARGIELA SCHÖNBRUNN, Ariadna, 46100 Burjassot (ES)
(74) Representative: Hoffmann Eitle

(57) **Abstract**

The invention relates to inhibitors of MSI2 functions that are able to decrease MSI2 activity, MSI2 transcript levels or MSI2 protein levels in myotonic dystrophy cells or cells with MD phenotype, wherein said inhibitor is for use in the treatment of myotonic dystrophy in a mammal.

SEQUENCE LISTING

<110> Universitat de València
      Fundación para la Investigación del Hospital Clínico de la Comunidad
Valenciana (INCLIVA)

<120> MSI2 AS A THERAPEUTIC TARGET FOR THE TREATMENT OF MYOTONIC DYSTROPHY

<130> 905 209

<140> EP21382133.3
<141> 2021-02-18

<160> 69

<170> BiSSAP 1.3.6

<210> 1
<211> 6379
<212> DNA
<213> Homo sapiens


<220>
<223> Homo sapiens musashi RNA binding protein 2 (MSI2), transcript
      variant 1 (NCBI Reference Sequence: NM_138962.4)

<400> 1
gtgcgaggca gcggggctga gctaagccga gcccacgtgt gacggctctc gccgctgccc      60

cggctccgcc gctcgcagag agattcggag gagcccgggc gggggggagg aggaggggga     120

ggagggagcg gagatctcgg ggctcggagc cggccgccgc tccgctccga tcgctgtggg     180

gcttggtttt ttgggggtgg gggggcgggg gggctcagat atggaggcaa atgggagcca     240

aggcacctcg ggcagcgcca acgactccca gcacgacccc ggtaaaatgt ttatcggtgg     300

actgagctgg cagacctcac cagatagcct tagagactat tttagcaaat ttggagaaat     360

tagagaatgt atggtcatga gagatcccac tacgaaacgc tccagaggct tcggtttcgt     420

cacgttcgca gacccagcaa gtgtagataa agtattaggt cagccccacc atgagttaga     480

ttccaagacg attgacccca aagttgcatt tcctcgtcga gcgcaaccca agatggtcac     540

aagaacaaag aaaatatttg taggcgggtt atctgcgaac acagtagtgg aagatgtaaa     600

gcaatatttc gagcagtttg gcaaggtgga agatgcaatg ctgatgtttg ataaaactac     660

caacaggcac agagggtttg gctttgtcac ttttgagaat gaagatgttg tggagaaagt     720

ctgtgagatt catttccatg aaatcaataa taaaatggta gaatgtaaga aagctcagcc     780

gaaagaagtc atgttcccac ctgggacaag aggccgggcc cggggactgc cttacaccat     840

ggacgcgttc atgcttggca tggggatgct gggatatccc aacttcgtgg cgacctatgg     900

ccgtggctac cccggatttg ctccaagcta tggctatcag ttcccaggct tcccagcagc     960

ggcttatgga ccagtggcag cagcggcggt ggcggcagca agaggatcag gctccaaccc    1020

ggcgcggccc ggaggcttcc cgggggccaa cagcccagga cctgtcgccg atctctacgg    1080

ccctgccagc caggactccg gagtggggaa ttacataagt gcggccagcc cacagccggg 1140

ctcgggcttc ggccacggca tagctggacc tttgattgca acggccttta caaatggata 1200

ccattgagca ggtgctttcg ttgccatctc actctgagag catacctgga tgtccaggca 1260

agactgggcg aagtttctga gtggcccttt gtttaggtga tgtcctcaga cctggacccc 1320

caccagcctc actccccatc ccaaccagag atggctcact tcggatcgag ggttgactac 1380

atctcatcat ctcacgaatc tgctgtaata taagacaaca gcttttaaat gtgtatataa 1440

cccatgattt cggttttgtt ttgttttgtt tttcttgatg gtttccctct ccctccctct 1500

cttcccattc tccttttaaa tctctttgaa tcacatttgg tagtgatttt gacttagtcc 1560

agtagtcaca tagctttaat atctagttca aagctaacca tagtataatt gttatattaa 1620

ggagttattt tttcttaaaa catttttttt tgcttgtttt ggttctgttc tcacttttaa 1680

aggatgctga gatggtaata tgactctcca tattttggta ccaattctga gactgtatga 1740

attttcaggt ggaactttag cacacactga agcaaagttg tgaagtgcag ggcgggaggt 1800

gggcgtgagc tttctatttt gcgttgtaga agaagtgaga tgtagtaagc taattaacag 1860

actttctagc agttcttttt gtgatgtctc tttgttaatc tgagtctatc tattttcggc 1920

aataaggtaa ggacgacagt gttttgagtg tcctcctttt ctataagtgc tttttttctg 1980

ttgaaagagg tgatattata aggttttttg aaattgtgaa ttctaaaaaa gaaatgttgt 2040

aaatacaatt ccattaacta catagaaact attaagaaag agagaatcaa aaatattttt 2100

gtgagggagt cggtcccagg cagtttgatg ctctgtggaa ggaggcggga agggaacgtt 2160

ggccaagtca gttactgaga tgaagatcgc ccagctgcca ggaccacccc aggacaagtt 2220

agagcactgt ttagctcctt tgtctgtgtg atagacctaa gaactgtatt agtgttgtac 2280

cagcctatta acctcttgtc tgtgcacagc ttcaaatgtt accgtctagt tagattttta 2340

tttaaaatat gaaaaactgc ttttcccaag atgtttttta aaaacaaagc tacaatttta 2400

atatttaaca tatttaaagt ttcaaagcac acctgtttgg cttgggtggg ggtggggtgg 2460

gggggacatt ctttttcagt cttaattttt aaatatttga tcattttcta ttgtccaatc 2520

atttcagcac ctccaaaggt ccctaggaca ctttgcctct cttctccccc tgccccccac 2580

cctgctccca catctggggg cccatgggcc aggagtggat aagcctgcat taatacaacc 2640

tttctccatt cactttctat ttacaaatta ggaaagcaac cttttggttt atatatattt 2700

tttttaatac ctcagtgctg caagtatcac cagagaggct atggaagaat ttttttttaa 2760

tttattgtag atgtaaacag aattttaaaa ataaaaagta taaacatcac tgcactgtga 2820

ctggtgggaa aaactgacag tttcctcttt gcacatgttt aacatttggc tgttataata 2880

tatggtcctc ggttggggaa agatacttat gatgaaggat attttttaat ttaacttttt 2940

tttaaatatt ggtaataggt cggcaacagc aactatagaa gtacaactca atagatggca 3000

ttaaaacata ttgtagtgtg gatatatatt ttttcttttt taaaatgtga tattgacgtt 3060

ttattaatat tttttaaatt gttacgttta taaatttggt acttaaggca cagccagtat 3120

gagacactga atgcgacatt tattataaag agctgctgca ctcctatttt tataaatttt 3180

actaacaaag tagactaatg tagacattca cagacatggt agggcaaaag catcttcaaa 3240

ctaaagactc caaaatgcta actcagaaag aaagaaaaaa cccgttttca attctaatga 3300

aacagcaaca acattttttt taattaaaaa aaaaatcatg ttctttgttt ttctaataaa 3360

atgttaggtt gtttggtgag gtttttttgt tgtttttttc ctttctttgt ttcttttctt 3420

ttcccccaaa caaccagatt aaatgctgag cgcttttaaa atatcaaaac ttgttcaagt 3480

cataaaacaa caaaacataa gttttattta aaaaaaaaaa aagaaagaaa aaatagtaaa 3540

ttcccagaaa ttcagtgttt agacgaggga atttaattcc tattttgtcc atgttggtga 3600

tgtactgtac ttcccttcct tttctctgca tcccccatca cctcatagaa gactctttgt 3660

tgatcattgt atgttaataa tgtataaaat ggctatcttg taagcgtgct gtcctggtac 3720

tagtgtagcg actttttttc tcctctttct tctagtacat attgataggt ataacataat 3780

taaggtttaa aaaaaattag acatagttat tcagatttag gaccagtaag gatagaactt 3840

tctcttattt atgaaaaaaa atgctaataa ttttgggcca gttttttcct ttaattattt 3900

ttttcaattt caagtttaat tttattttag ctgatctgat gtggtttcaa ctaacccaag 3960

gtctcaccat gttaaaatgc cggcggactc tacggcgttt tgtagatccc ccccccccca 4020

cccactgtga aggggtgcca tactacctta aatgctaatg ctagatatgc aaaactggat 4080

ttttttaatt tattttttaa aagagggagg catggtatat taaaatgatt ttactaagag 4140

aaaaaatatt tttttaagaa tgctcagaag aaattgataa tctgtgtgaa tatgttttag 4200

atgtttatat accttttgaa gagacccagt agcccatagc acaaatcttg tggaaatccg 4260

atatgtttta atgtggctac ctaggtttaa ggttcacgtt agtcccccca ttccatctag 4320

aagtccattt tgaaagattt ttgtaaattc ttttaacact gatgtttcag cctcgtcttt 4380

gtttcagtta agctcaatgg cgaacatggg aaccaccttt cgccttccct gggggagaaa 4440

ccctcttggc tgatggcttt tccccggaat tatcaaacag ccaccgggtg actttctggc 4500

ttccagatcc atctgcctga gacccccgaa ctcctctcct cccaagcaga ggcgagtgag 4560

tggcattagc tcccggaccc attcccggtc ctagctgggc atggggctga cggagatgac 4620

caagccttgg tctgctctct agcagcttcc acagacttgg ctcgtggcct tccttatatc 4680

cactgggaac aaacagcctc gcgctctata ccaaacagcc tcgcgctcta tcccagtcgc 4740

ccattagctt gattcaaaca aagccccagc aggcctttgc gtttttatcc ttcataacct 4800

tcatcttaat ttgaacttgt agcttggact ttaaggtagc atggctctat tgctgtcaat 4860

ttactgtttc actgcacagc aatcacagcc agtgaatgtt acacacatct tgctagacta 4920

gtataaaaat cattgggtaa ttgttggttc taatgacctg aaaggtgttc agtttttgtt 4980

cttggttttg ttttgttttt gatttcttgg gggtgggttt tgcttgttgt tccttttcat 5040

ttgggggttt ttggggaaaa aatttatttt tggttccaaa tagaaaaaca aaacctattt 5100

tgatctttag tgcaaacgag ggctagggac ttagcctcct ccaccacctc cacactgctt 5160

cattctgcca ttcactcact gcagcatatt caagaataaa gcaatatcgt ttactacatt 5220

ttttattgaa ggtcagccat gctttctgta ttatattgca tatgaaattg tttacaaaag 5280

aaacactaac tcatacttct ctttattggt tgcaagtggc acgcaggaac agagggagag 5340

tggggggctg gtgggggagg ggagattttt tttttttttt cttgaatgtg cttcgcaagc 5400

caggctatct tccaaggaag gcagacagtg ggagagcaga gggactgact gcaggcaagc 5460

acattgaaga aagacactgg cgggtttccc caccctcacc ccaaagcaga aaactagcag 5520

acgtcagctc agccccgtcc tgggcacaga cactacacaa ggagacgctg gaagttaagc 5580

aatactttaa tactgtaata tgtttgtttt ctttttcttt cttttttttc taccaaaaaa 5640

aagtaagtaa actaaaacac aaaaacatat aaataaaatc catccctctt gtcggggacc 5700

tgcagggggg caaccttaat ccaaacacct ggctatcaaa taatcagaat gtattgtctc 5760

agacaggatt tcagttccgg gaggcagggg catgatgggg gagggggccg gaggctgaga 5820

gacaaaagtt ccagagcctc cctcgaaggt tctctactac tgtattctgt acataatgta 5880

ccatcccatg tggaatctgt gagtgtcctc ttaagtagcg tgggctagcc aatctgccgt 5940

tcatggtgta ttgtaaactc cgaattccat atgtaatagg atgcaagtct aagcgtttca 6000

tgtggacata aatgtatcta aataaaactt tccctagcac tgtggctgac ctcaccctta 6060

cttttatact ttagtatgaa actgatgaga actttggtag tgagtatttt ttttatatat 6120

atacatatat atgtactatc tatatatata tctcaagcat ctttcaggtc tttgtgtgtg 6180

gctttcttaa agccctgttg taaaaaatta ctatgtggat ggcagtctct cacatcacag 6240

atgtggaaag tataatttta tatttgtatt ttcaaataaa taagtttgtg aaaggtttcc 6300

atcctctact gtggtccaga aatcaatgtg tttgtctgac aaaaaaaaaa ataaaataaa 6360

ataaactgtt ttgaacaga 6379

<210> 2
<211> 1292
<212> DNA
<213> Artificial Sequence

<220>
<223> Partial MSI2 transcript (nucleotides 327-1618)

<400> 2
gccttagaga ctattttagc aaatttggag aaattagaga atgtatggtc atgagagatc      60

ccactacgaa acgctccaga ggcttcggtt tcgtcacgtt cgcagaccca gcaagtgtag     120

ataaagtatt aggtcagccc caccatgagt tagattccaa gacgattgac cccaaagttg     180

catttcctcg tcgagcgcaa cccaagatgg tcacaagaac aaagaaaata tttgtaggcg     240

ggttatctgc gaacacagta gtggaagatg taaagcaata tttcgagcag tttggcaagg     300

tggaagatgc aatgctgatg tttgataaaa ctaccaacag gcacagaggg tttggctttg     360

tcacttttga gaatgaagat gttgtggaga aagtctgtga gattcatttc catgaaatca     420

ataataaaat ggtagaatgt aagaaagctc agccgaaaga agtcatgttc ccacctggga     480

caagaggccg ggcccgggga ctgccttaca ccatggacgc gttcatgctt ggcatgggga     540

tgctgggata tcccaacttc gtggcgacct atggccgtgg ctaccccgga tttgctccaa     600

gctatggcta tcagttccca ggcttcccag cagcggctta tggaccagtg gcagcagcgg     660

cggtggcggc agcaagagga tcaggctcca acccggcgcg gcccggaggc ttcccggggg     720

ccaacagccc aggacctgtc gccgatctct acggccctgc cagccaggac tccggagtgg     780

ggaattacat aagtgcggcc agcccacagc cgggctcggg cttcggccac ggcatagctg     840

gacctttgat tgcaacggcc tttacaaatg gataccattg agcaggtgct ttcgttgcca     900

tctcactctg agagcatacc tggatgtcca ggcaagactg ggcgaagttt ctgagtggcc     960

ctttgtttag gtgatgtcct cagacctgga cccccaccag cctcactccc catcccaacc    1020

agagatggct cacttcggat cgagggttga ctacatctca tcatctcacg aatctgctgt    1080

aatataagac aacagctttt aaatgtgtat ataacccatg atttcggttt tgttttgttt    1140

tgtttttctt gatggtttcc ctctccctcc ctctcttccc attctccttt taaatctctt    1200

tgaatcacat ttggtagtga ttttgactta gtccagtagt cacatagctt taatatctag    1260

ttcaaagcta accatagtat aattgttata tt                                  1292

<210> 3
<211> 23
<212> DNA
<213> Artificial Sequence

<220>
<223> potential target region

<400> 3
gccttagaga ctattttagc aaa                                             23

<210> 4
<211> 23
<212> DNA
<213> Artificial Sequence

<220>
<223> potential target region

<400> 4
tagagactat tttagcaaat ttg                                               23


<210> 5
<211> 23
<212> DNA
<213> Artificial Sequence


<220>
<223> potential target nucleotide

<400> 5
gactatttta gcaaatttgg aga                                               23


<210> 6
<211> 23
<212> DNA
<213> Artificial Sequence


<220>
<223> potential target region

<400> 6
agcaaatttg gagaaattag aga                                               23


<210> 7
<211> 23
<212> DNA
<213> Artificial Sequence


<220>
<223> potential target region

<400> 7
agcaagtgta gataaagtat tag                                               23


<210> 8
<211> 23
<212> DNA
<213> Artificial Sequence


<220>
<223> potential target region

<400> 8
cacaagaaca aagaaaatat ttg                                               23


<210> 9
<211> 23

<212> DNA
<213> Artificial Sequence

<220>
<223> potential target region

<400> 9
aagaacaaag aaaatatttg tag                                              23

<210> 10
<211> 23
<212> DNA
<213> Artificial Sequence

<220>
<223> potential target region

<400> 10
gtggaagatg taaagcaata ttt                                              23

<210> 11
<211> 23
<212> DNA
<213> Artificial Sequence

<220>
<223> potential target region

<400> 11
tggaagatgt aaagcaatat ttc                                              23

<210> 12
<211> 23
<212> DNA
<213> Artificial Sequence

<220>
<223> potential target region

<400> 12
tgctgatgtt tgataaaact acc                                              23

<210> 13
<211> 23
<212> DNA
<213> Artificial Sequence

<220>
<223> potential target region

<400> 13
tggctttgtc acttttgaga atg                                              23

<210> 14
<211> 23
<212> DNA
<213> Artificial Sequence

<220>
<223> potential target region

<400> 14
ggctttgtca cttttgagaa tga                                              23

<210> 15
<211> 23
<212> DNA
<213> Artificial Sequence

<220>
<223> potential target region

<400> 15
cacttttgag aatgaagatg ttg                                              23

<210> 16
<211> 23
<212> DNA
<213> Artificial Sequence

<220>
<223> potential target region

<400> 16
aagatgttgt ggagaaagtc tgt                                              23

<210> 17
<211> 23
<212> DNA
<213> Artificial Sequence

<220>
<223> potential target region

<400> 17
gagaaagtct gtgagattca ttt                                              23

<210> 18
<211> 23
<212> DNA
<213> Artificial Sequence

<220>
<223> potential target region

<400> 18
ttcatttcca tgaaatcaat aat                                              23

<210> 19
<211> 23
<212> DNA
<213> Artificial Sequence

<220>
<223> potential target region

<400> 19
ttccatgaaa tcaataataa aat                                               23

<210> 20
<211> 23
<212> DNA
<213> Artificial Sequence

<220>
<223> potential target region

<400> 20
tccaagctat ggctatcagt tcc                                               23

<210> 21
<211> 23
<212> DNA
<213> Artificial Sequence

<220>
<223> potential target region

<400> 21
ttgattgcaa cggcctttac aaa                                               23

<210> 22
<211> 23
<212> DNA
<213> Artificial Sequence

<220>
<223> potential target region

<400> 22
cacgaatctg ctgtaatata aga                                               23

<210> 23
<211> 23
<212> DNA
<213> Artificial Sequence

<220>
<223> potential target region

<400> 23
aacagctttt aaatgtgtat ata                                               23

<210> 24
<211> 23
<212> DNA
<213> Artificial Sequence

<220>
<223> potential target region

<400> 24
cagcttttaa atgtgtatat aac                                               23

<210> 25
<211> 23
<212> DNA
<213> Artificial Sequence

<220>
<223> potential target region

<400> 25
agcttttaaa tgtgtatata acc                                               23

<210> 26
<211> 23
<212> DNA
<213> Artificial Sequence

<220>
<223> potential target region

<400> 26
tcccattctc cttttaaatc tct                                               23

<210> 27
<211> 23
<212> DNA
<213> Artificial Sequence

<220>
<223> potential target region

<400> 27
ctccttttaa atctctttga atc                                               23

<210> 28
<211> 23
<212> DNA
<213> Artificial Sequence

<220>

<223> potential target region

<400> 28
tccttttaaa tctctttgaa tca                                              23


<210> 29
<211> 23
<212> DNA
<213> Artificial Sequence


<220>
<223> potential target region

<400> 29
ctctttgaat cacatttggt agt                                              23


<210> 30
<211> 23
<212> DNA
<213> Artificial Sequence


<220>
<223> potential target region

<400> 30
atcacatttg gtagtgattt tga                                              23


<210> 31
<211> 23
<212> DNA
<213> Artificial Sequence


<220>
<223> potential target region

<400> 31
tggtagtgat tttgacttag tcc                                              23


<210> 32
<211> 23
<212> DNA
<213> Artificial Sequence


<220>
<223> potential target region

<400> 32
cacatagctt taatatctag ttc                                              23


<210> 33
<211> 23
<212> DNA
<213> Artificial Sequence

<220>
<223> potential target region

<400> 33
tagctttaat atctagttca aag                                               23


<210> 34
<211> 23
<212> DNA
<213> Artificial Sequence


<220>
<223> potential target region

<400> 34
agctttaata tctagttcaa agc                                               23


<210> 35
<211> 23
<212> DNA
<213> Artificial Sequence


<220>
<223> potential target region

<400> 35
ttcaaagcta accatagtat aat                                               23


<210> 36
<211> 23
<212> DNA
<213> Artificial Sequence


<220>
<223> potential target region

<400> 36
agctaaccat agtataattg tta                                               23


<210> 37
<211> 23
<212> DNA
<213> Artificial Sequence


<220>
<223> potential target region

<400> 37
aaccatagta taattgttat att                                               23


<210> 38
<211> 16
<212> DNA

<213> Artificial Sequence

<220>
<223> ASO 1

<400> 38
tgacttcttt cggctg                                                    16

<210> 39
<211> 16
<212> DNA
<213> Artificial Sequence

<220>
<223> ASO 3

<400> 39
ttggattaag gttgcc                                                    16

<210> 40
<211> 21
<212> DNA
<213> Artificial Sequence

<220>
<223> siRNA 1

<400> 40
tgacttcttt cggctgagct t                                              21

<210> 41
<211> 23
<212> RNA
<213> Artificial Sequence

<220>
<223> Sense AgomiR-7 (mimic of miR-107)

<400> 41
agcagcauug uacagggcua uca                                            23

<210> 42
<211> 23
<212> RNA
<213> Artificial Sequence

<220>
<223> Antisense AgomiR-7 (mimic of miR-107)

<400> 42
ugauagcccu guacaaugcu gcu                                            23

<210> 43

<211> 21
<212> DNA
<213> Artificial Sequence

<220>
<223> siRNA 2

<400> 43
agttttatca aacatcagca t                                                21

<210> 44
<211> 16
<212> DNA
<213> Artificial Sequence

<220>
<223> ASOS SCRAMBLE (control)

<400> 44
ttccctgaag gttcct                                                      16

<210> 45
<211> 20
<212> DNA
<213> Artificial Sequence

<220>
<223> GAPDH fwd

<400> 45
catcttccag gagcgagatc                                                  20

<210> 46
<211> 21
<212> DNA
<213> Artificial Sequence

<220>
<223> GAPDH rev

<400> 46
gttcacaccc atgacgaaca t                                                21

<210> 47
<211> 20
<212> DNA
<213> Artificial Sequence

<220>
<223> GPI fwd

<400> 47
cagggcatca tctgggacat                                                  20

<210> 48
<211> 20
<212> DNA
<213> Artificial Sequence

<220>
<223> GPI rev

<400> 48
tcttagccag ctgctttccc                                                    20

<210> 49
<211> 21
<212> DNA
<213> Artificial Sequence

<220>
<223> HPRT1 fwd

<400> 49
tgacactggc aaaacaatgc a                                                  21

<210> 50
<211> 21
<212> DNA
<213> Artificial Sequence

<220>
<223> HPRT1 REV

<400> 50
ggtccttttc accagcaagc t                                                  21

<210> 51
<211> 23
<212> DNA
<213> Artificial Sequence

<220>
<223> IGF1 fwd

<400> 51
ctcttcagtt cgtgtgtgga gac                                                23

<210> 52
<211> 22
<212> DNA
<213> Artificial Sequence

<220>
<223> IGF1 rev

<400> 52

cagcctcctt agatcacagc tc                                                22

<210> 53
<211> 22
<212> DNA
<213> Artificial Sequence

<220>
<223> MSI2 fwd

<400> 53
gcagacctca ccagatagcc tt                                                22

<210> 54
<211> 22
<212> DNA
<213> Artificial Sequence

<220>
<223> MSI2 rev

<400> 54
aagcctctgg agcgtttcgt ag                                                22

<210> 55
<211> 24
<212> DNA
<213> Artificial Sequence

<220>
<223> MSTN fwd

<400> 55
tgagaatggt catgatcttg ctgt                                              24

<210> 56
<211> 24
<212> DNA
<213> Artificial Sequence

<220>
<223> MSTN rev

<400> 56
   tcatcacagt caagaccaaa atcc                                             24

<210> 57
<211> 22
<212> DNA
<213> Artificial Sequence

<220>
<223> mTOR fwd

<400> 57
agcatcggat gcttaggagt gg                                                22

<210> 58
<211> 22
<212> DNA
<213> Artificial Sequence

<220>
<223> mTOR rev

<400> 58
cagccagtca tctttggaga cc                                                22

<210> 59
<211> 24
<212> DNA
<213> Artificial Sequence

<220>
<223> NFIX fwd

<400> 59
gagccctgtt gatgacgtgt tcta                                              24

<210> 60
<211> 24
<212> DNA
<213> Artificial Sequence

<220>
<223> NFIX rev

<400> 60
ctgcacaaac tccttcagtg agtc                                              24

<210> 61
<211> 22
<212> DNA
<213> Artificial Sequence

<220>
<223> P21 fwd

<400> 61
aggtggacct ggagactctc ag                                                22

<210> 62
<211> 22
<212> DNA
<213> Artificial Sequence

<220>
<223> P21 rev

<400> 62
tcctcttgga gaagatcagc cg                                                22

<210> 63
<211> 20
<212> DNA
<213> Artificial Sequence

<220>
<223> PKM fwd

<400> 63
ctgaaggcag tgatgtcgcc                                                   20

<210> 64
<211> 19
<212> DNA
<213> Artificial Sequence

<220>
<223> PKM rev

<400> 64
acccggaggt ccacgtctc                                                    19

<210> 65
<211> 21
<212> DNA
<213> Artificial Sequence

<220>
<223> ATP2A fwd

<400> 65
gatgatcttc aagctccggg c                                                 21

<210> 66
<211> 20
<212> DNA
<213> Artificial Sequence

<220>
<223> ATP2A rev

<400> 66
cagctctgcc tgaagatgtg                                                   20

<210> 67
<211> 22
<212> DNA
<213> Artificial Sequence

<220>
<223> TGFBR1 fwd

<400> 67
gacaacgtca ggttctggct ca                                                22


<210> 68
<211> 22
<212> DNA
<213> Artificial Sequence


<220>
<223> TGFBR1 rev

<400> 68
ccgccacttt cctctccaaa ct                                                22


<210> 69
<211> 2619
<212> DNA
<213> Artificial Sequence


<220>
<223> AAV9-hDES-mMSI2(V4)-V5-T2A-Nanoluc

<400> 69
gctagcgcgg ccgcacccat gcctcctcag gtaccccctg ccccccacag ctcctctcct      60

gtgccttgtt tcccagccat gcgttctcct ctataaatac ccgctctggt atttggggtt      120

ggcagctgtt gctgccaggg agatggttgg gttgacatgc ggctcctgac aaaacacaaa      180

cccctggtgt gtgtgggcgt gggtggtgtg agtaggggga tgaatcaggg agggggcggg      240

ggacccaggg ggcaggagcc acacaaagtc tgtgcggggg tgggagcgca catagcaatt      300

ggaaactgaa agcttatcag accctttctg gaaatcagcc cactgtttat aaacttgagg      360

ccccaccctc gagataacca gggctgaaag aggcccgcct gggggctgca gacatgcttg      420

ctgcctgccc tggcgaagga ttggcaggct tgcccgtcac aggacccccg ctggctgact      480

caggggcgca ggcctcttgc gggggagctg gcctccccgc ccccacggcc acgggccgcc      540

ctttcctggc aggacagcgg gatcttgcag ctgtcagggg aggggaggcg ggggctgatg      600

tcaggaggga tacaaatagt gccgacggct gggggccctg tctcccctcg ccgcatccac      660

tctccggccg gccgcctgcc cgccgcctcc tccgtgcgcc cgccagcctc gcccgcgccg      720

tcaccggttc gaacaggtaa gcgcccctaa aatccctttg gcacaatgtg tcctgagggg      780

agaggcagcg acctgtagat gggacggggg cactaaccct cagggtttgg ggttctgaat      840

gtgagtatcg ccatctaagc ccagtatttg gccaatctca gaaagctcct ggctccctgg      900

aggatggaga gagaaaaaca aacagctcct ggagcaggga gagtgttggc ctcttgctct      960

ccggctccct ctgttgccct ctggtttctc cccaggttcg aaggatccat ggaggcaaat 1020

gggagcccag gcacctcggg cagcgccaac gactcccagc acgaccccgg taaaatgttt 1080

atcggtggac tgagctggca gacctcacca gatagcctta gagactattt tagcaaattt 1140

ggagaaatta gagaatgtat ggtcatgaga gatcccacaa cgaaacgctc cagaggcttc 1200

ggtttcgtca ccttcgcaga cccagcaagt gtagataaag tattaggtca gccccaccat 1260

gagttagatt ccaagacgat tgacccaaaa gttgcatttc ctcgtcgagc gcaacctaag 1320

atggtcacaa gaacaaagaa aatcttcgta ggaggattgt ctgcaaacac agtagtggaa 1380

gatgtaaagc agtatttcga gcagtttggc aaggtagagg atgcgatgct gatgttcgac 1440

aaaaccacca acaggcacag agggtttggc tttgtcacct ttgagaatga agacgttgtg 1500

gagaaagtct gtgagattca tttccatgaa atcaataata aaatggtaga atgtaagaaa 1560

gctcagccga aagaagtcat gttcccacct gggacaagag gccgggcccg gggcctgcca 1620

tacaccatgg atgcgttcat gcttggcatg gggatgctgg gctaccccaa ctttgtggca 1680

acctatggca gaggctaccc cggatttgct cctagctatg gctaccagtt cccaggcttc 1740

ccggcggcag cttatggacc agtggcagcg gcagctgtgg cagcggctcg aggatcaggc 1800

tccaacccgg cgcggcccgg aggcttcccg ggggccaaca gcccaggacc tgtcgccgat 1860

ctctacggcc ctgccagcca ggactccgga gtggggaatt acataagcgc ggccagccca 1920

cagccgggct ccggcttcgg ccacggcata gctggacctt tgattgcaac ggcctttaca 1980

aatggatacc actgaggtaa gcctatccct aaccctctcc tcggtctcga ttctacggag 2040

ggcagaggaa gtctgctaac atgcggtgac gtcgaggaga atcctggccc aggatccatg 2100

gtcttcacac tcgaagattt cgttggggac tggcgacaga cagccggcta caacctggac 2160

caagtccttg aacagggagg tgtgtccagt ttgtttcaga atctcggggt gtccgtaact 2220

ccgatccaaa ggattgtcct gagcggtgaa aatgggctga agatcgacat ccatgtcatc 2280

atcccgtatg aaggtctgag cggcgaccaa atgggccaga tcgaaaaaat ttttaaggtg 2340

gtgtaccctg tggatgatca tcactttaag gtgatcctgc actatggcac actggtaatc 2400

gacggggtta cgccgaacat gatcgactat ttcggacggc cgtatgaagg catcgccgtg 2460

ttcgacggca aaaagatcac tgtaacaggg accctgtgga acggcaacaa aattatcgac 2520

gagcgcctga tcaaccccga cggctccctg ctgttccgag taaccatcaa cggagtgacc 2580

ggctggcggc tgtgcgaacg cattctggcg taagtatac 2619

SEQUENCE LISTING

<110> Universitat de València
      Fundación para la Investigación del Hospital Clínico de la
      Comunidad Valenciana (INCLIVA)

<120> MSI2 AS A THERAPEUTIC TARGET FOR THE TREATMENT OF MYOTONIC DYSTROPHY

<130> 905 209

<140> EP21382133.3
<141> 2021-02-18

<160> 70

<170> BiSSAP 1.3.6

<210> 1
<211> 6379
<212> DNA
<213> Homo sapiens


<220>
<223> Homo sapiens musashi RNA binding protein 2 (MSI2), transcript
      variant 1 (NCBI Reference Sequence: NM_138962.4)

<400> 1
gtgcgaggca gcggggctga gctaagccga gcccacgtgt gacggctctc gccgctgccc      60

cggctccgcc gctcgcagag agattcggag gagcccgggc gggggggagg aggaggggga     120

ggagggagcg gagatctcgg ggctcggagc cggccgccgc tccgctccga tcgctgtggg     180

gcttggtttt ttgggggtgg gggggcgggg gggctcagat atggaggcaa atgggagcca     240

aggcacctcg ggcagcgcca acgactccca gcacgacccc ggtaaaatgt ttatcggtgg     300

actgagctgg cagacctcac cagatagcct tagagactat tttagcaaat ttggagaaat     360

tagagaatgt atggtcatga gagatcccac tacgaaacgc tccagaggct tcggtttcgt     420

cacgttcgca gacccagcaa gtgtagataa agtattaggt cagccccacc atgagttaga     480

ttccaagacg attgacccca aagttgcatt tcctcgtcga gcgcaaccca agatggtcac     540

aagaacaaag aaaatatttg taggcgggtt atctgcgaac acagtagtgg aagatgtaaa     600

gcaatatttc gagcagtttg gcaaggtgga agatgcaatg ctgatgtttg ataaaactac     660

caacaggcac agagggtttg gctttgtcac ttttgagaat gaagatgttg tggagaaagt     720

ctgtgagatt catttccatg aaatcaataa taaaatggta gaatgtaaga aagctcagcc     780

gaaagaagtc atgttcccac ctgggacaag aggccgggcc cggggactgc cttacaccat     840

ggacgcgttc atgcttggca tggggatgct gggatatccc aacttcgtgg cgacctatgg     900

ccgtggctac cccggatttg ctccaagcta tggctatcag ttcccaggct tcccagcagc     960

ggcttatgga ccagtggcag cagcggcggt ggcggcagca agaggatcag gctccaaccc    1020

ggcgcggccc ggaggcttcc cgggggccaa cagcccagga cctgtcgccg atctctacgg    1080

ccctgccagc caggactccg gagtggggaa ttacataagt gcggccagcc cacagccggg 1140

ctcgggcttc ggccacggca tagctggacc tttgattgca acggccttta caaatggata 1200

ccattgagca ggtgctttcg ttgccatctc actctgagag catacctgga tgtccaggca 1260

agactgggcg aagtttctga gtggcccttt gtttaggtga tgtcctcaga cctggacccc 1320

caccagcctc actccccatc ccaaccagag atggctcact tcggatcgag ggttgactac 1380

atctcatcat ctcacgaatc tgctgtaata taagacaaca gcttttaaat gtgtatataa 1440

cccatgattt cggttttgtt ttgttttgtt tttcttgatg gtttccctct ccctccctct 1500

cttcccattc tccttttaaa tctctttgaa tcacatttgg tagtgatttt gacttagtcc 1560

agtagtcaca tagctttaat atctagttca aagctaacca tagtataatt gttatattaa 1620

ggagttattt tttcttaaaa catttttttt tgcttgtttt ggttctgttc tcacttttaa 1680

aggatgctga gatggtaata tgactctcca tattttggta ccaattctga gactgtatga 1740

attttcaggt ggaactttag cacacactga agcaaagttg tgaagtgcag ggcgggaggt 1800

gggcgtgagc tttctatttt gcgttgtaga agaagtgaga tgtagtaagc taattaacag 1860

actttctagc agttcttttt gtgatgtctc tttgttaatc tgagtctatc tattttcggc 1920

aataaggtaa ggacgacagt gttttgagtg tcctcctttt ctataagtgc tttttttctg 1980

ttgaaagagg tgatattata aggttttttg aaattgtgaa ttctaaaaaa gaaatgttgt 2040

aaatacaatt ccattaacta catagaaact attaagaaag agagaatcaa aaatattttt 2100

gtgagggagt cggtcccagg cagtttgatg ctctgtggaa ggaggcggga agggaacgtt 2160

ggccaagtca gttactgaga tgaagatcgc ccagctgcca ggaccacccc aggacaagtt 2220

agagcactgt ttagctcctt tgtctgtgtg atagacctaa gaactgtatt agtgttgtac 2280

cagcctatta acctcttgtc tgtgcacagc ttcaaatgtt accgtctagt tagattttta 2340

tttaaaatat gaaaaactgc ttttcccaag atgtttttta aaaacaaagc tacaatttta 2400

atatttaaca tatttaaagt ttcaaagcac acctgtttgg cttgggtggg ggtggggtgg 2460

gggggacatt ctttttcagt cttaattttt aaatatttga tcattttcta ttgtccaatc 2520

atttcagcac ctccaaaggt ccctaggaca ctttgcctct cttctccccc tgccccccac 2580

cctgctccca catctggggg cccatgggcc aggagtggat aagcctgcat taatacaacc 2640

tttctccatt cactttctat ttacaaatta ggaaagcaac cttttggttt atatatattt 2700

tttttaatac ctcagtgctg caagtatcac cagagaggct atggaagaat ttttttttaa 2760

tttattgtag atgtaaacag aattttaaaa ataaaaagta taaacatcac tgcactgtga 2820

ctggtgggaa aaactgacag tttcctcttt gcacatgttt aacatttggc tgttataata 2880

tatggtcctc ggttggggaa agatacttat gatgaaggat attttttaat ttaacttttt 2940

tttaaatatt ggtaataggt cggcaacagc aactatagaa gtacaactca atagatggca 3000

ttaaaacata ttgtagtgtg gatatatatt ttttcttttt taaaatgtga tattgacgtt 3060

ttattaatat tttttaaatt gttacgttta taaatttggt acttaaggca cagccagtat 3120

gagacactga atgcgacatt tattataaag agctgctgca ctcctatttt tataaatttt 3180

actaacaaag tagactaatg tagacattca cagacatggt agggcaaaag catcttcaaa 3240

ctaaagactc caaaatgcta actcagaaag aaagaaaaaa cccgttttca attctaatga 3300

aacagcaaca acattttttt taattaaaaa aaaaatcatg ttctttgttt ttctaataaa 3360

atgttaggtt gtttggtgag gtttttttgt tgtttttttc ctttctttgt ttcttttctt 3420

ttcccccaaa caaccagatt aaatgctgag cgcttttaaa atatcaaaac ttgttcaagt 3480

cataaaacaa caaaacataa gttttattta aaaaaaaaaa aagaaagaaa aaatagtaaa 3540

ttcccagaaa ttcagtgttt agacgaggga atttaattcc tattttgtcc atgttggtga 3600

tgtactgtac ttcccttcct tttctctgca tcccccatca cctcatagaa gactctttgt 3660

tgatcattgt atgttaataa tgtataaaat ggctatcttg taagcgtgct gtcctggtac 3720

tagtgtagcg actttttttc tcctctttct tctagtacat attgataggt ataacataat 3780

taaggtttaa aaaaaattag acatagttat tcagatttag gaccagtaag gatagaactt 3840

tctcttattt atgaaaaaaa atgctaataa ttttgggcca gttttttcct ttaattattt 3900

ttttcaattt caagtttaat tttattttag ctgatctgat gtggtttcaa ctaacccaag 3960

gtctcaccat gttaaaatgc cggcggactc tacggcgttt tgtagatccc ccccccccca 4020

cccactgtga aggggtgcca tactacctta aatgctaatg ctagatatgc aaaactggat 4080

ttttttaatt tattttttaa aagagggagg catggtatat taaaatgatt ttactaagag 4140

aaaaaatatt tttttaagaa tgctcagaag aaattgataa tctgtgtgaa tatgttttag 4200

atgtttatat accttttgaa gagacccagt agcccatagc acaaatcttg tggaaatccg 4260

atatgtttta atgtggctac ctaggtttaa ggttcacgtt agtcccccca ttccatctag 4320

aagtccattt tgaaagattt ttgtaaattc ttttaacact gatgtttcag cctcgtcttt 4380

gtttcagtta agctcaatgg cgaacatggg aaccaccttt cgccttccct gggggagaaa 4440

ccctcttggc tgatggcttt tccccggaat tatcaaacag ccaccgggtg actttctggc 4500

ttccagatcc atctgcctga gacccccgaa ctcctctcct cccaagcaga ggcgagtgag 4560

tggcattagc tcccggaccc attcccggtc ctagctgggc atggggctga cggagatgac 4620

caagccttgg tctgctctct agcagcttcc acagacttgg ctcgtggcct tccttatatc 4680

cactgggaac aaacagcctc gcgctctata ccaaacagcc tcgcgctcta tcccagtcgc 4740

ccattagctt gattcaaaca aagccccagc aggcctttgc gtttttatcc ttcataacct 4800

tcatcttaat ttgaacttgt agcttggact ttaaggtagc atggctctat tgctgtcaat 4860

ttactgtttc actgcacagc aatcacagcc agtgaatgtt acacacatct tgctagacta 4920

gtataaaaat cattgggtaa ttgttggttc taatgacctg aaaggtgttc agtttttgtt 4980

cttggttttg ttttgttttt gatttcttgg gggtgggttt tgcttgttgt tccttttcat 5040

ttgggggttt ttggggaaaa aatttatttt tggttccaaa tagaaaaaca aaacctattt 5100

tgatctttag tgcaaacgag ggctagggac ttagcctcct ccaccacctc cacactgctt 5160

cattctgcca ttcactcact gcagcatatt caagaataaa gcaatatcgt ttactacatt 5220

ttttattgaa ggtcagccat gctttctgta ttatattgca tatgaaattg tttacaaaag 5280

aaacactaac tcatacttct ctttattggt tgcaagtggc acgcaggaac agagggagag 5340

tggggggctg gtgggggagg ggagattttt tttttttttt cttgaatgtg cttcgcaagc 5400

caggctatct tccaaggaag gcagacagtg ggagagcaga gggactgact gcaggcaagc 5460

acattgaaga aagacactgg cgggtttccc caccctcacc ccaaagcaga aaactagcag 5520

acgtcagctc agccccgtcc tgggcacaga cactacacaa ggagacgctg gaagttaagc 5580

aatactttaa tactgtaata tgtttgtttt ctttttcttt cttttttttc taccaaaaaa 5640

aagtaagtaa actaaaacac aaaaacatat aaataaaatc catccctctt gtcggggacc 5700

tgcagggggg caaccttaat ccaaacacct ggctatcaaa taatcagaat gtattgtctc 5760

agacaggatt tcagttccgg gaggcagggg catgatgggg gagggggccg gaggctgaga 5820

gacaaaagtt ccagagcctc cctcgaaggt tctctactac tgtattctgt acataatgta 5880

ccatcccatg tggaatctgt gagtgtcctc ttaagtagcg tgggctagcc aatctgccgt 5940

tcatggtgta ttgtaaactc cgaattccat atgtaatagg atgcaagtct aagcgtttca 6000

tgtggacata aatgtatcta aataaaactt tccctagcac tgtggctgac ctcaccctta 6060

cttttatact ttagtatgaa actgatgaga actttggtag tgagtatttt ttttatatat 6120

atacatatat atgtactatc tatatatata tctcaagcat ctttcaggtc tttgtgtgtg 6180

gctttcttaa agccctgttg taaaaaatta ctatgtggat ggcagtctct cacatcacag 6240

atgtggaaag tataatttta tatttgtatt ttcaaataaa taagtttgtg aaaggtttcc 6300

atcctctact gtggtccaga aatcaatgtg tttgtctgac aaaaaaaaaa ataaaataaa 6360

ataaactgtt ttgaacaga 6379

<210> 2
<211> 1292
<212> DNA
<213> Artificial Sequence

<220>
<223> Partial MSI2 transcript (nucleotides 327-1618)

<400> 2
gccttagaga ctattttagc aaatttggag aaattagaga atgtatggtc atgagagatc      60

ccactacgaa acgctccaga ggcttcggtt tcgtcacgtt cgcagaccca gcaagtgtag     120

ataaagtatt aggtcagccc caccatgagt tagattccaa gacgattgac cccaaagttg     180

catttcctcg tcgagcgcaa cccaagatgg tcacaagaac aaagaaaata tttgtaggcg     240

ggttatctgc gaacacagta gtggaagatg taaagcaata tttcgagcag tttggcaagg     300

tggaagatgc aatgctgatg tttgataaaa ctaccaacag gcacagaggg tttggctttg     360

tcacttttga gaatgaagat gttgtggaga aagtctgtga gattcatttc catgaaatca     420

ataataaaat ggtagaatgt aagaaagctc agccgaaaga agtcatgttc ccacctggga     480

caagaggccg ggcccgggga ctgccttaca ccatggacgc gttcatgctt ggcatgggga     540

tgctgggata tcccaacttc gtggcgacct atggccgtgg ctaccccgga tttgctccaa     600

gctatggcta tcagttccca ggcttcccag cagcggctta tggaccagtg gcagcagcgg     660

cggtggcggc agcaagagga tcaggctcca acccggcgcg gcccggaggc ttcccggggg     720

ccaacagccc aggacctgtc gccgatctct acggccctgc cagccaggac tccggagtgg     780

ggaattacat aagtgcggcc agcccacagc cgggctcggg cttcggccac ggcatagctg     840

gacctttgat tgcaacggcc tttacaaatg gataccattg agcaggtgct ttcgttgcca     900

tctcactctg agagcatacc tggatgtcca ggcaagactg ggcgaagttt ctgagtggcc     960

ctttgtttag gtgatgtcct cagacctgga cccccaccag cctcactccc catcccaacc    1020

agagatggct cacttcggat cgagggttga ctacatctca tcatctcacg aatctgctgt    1080

aatataagac aacagctttt aaatgtgtat ataacccatg atttcggttt tgttttgttt    1140

tgtttttctt gatggtttcc ctctccctcc ctctcttccc attctccttt taaatctctt    1200

tgaatcacat ttggtagtga ttttgactta gtccagtagt cacatagctt taatatctag    1260

ttcaaagcta accatagtat aattgttata tt                                  1292

<210> 3
<211> 23
<212> DNA
<213> Artificial Sequence

<220>
<223> potential target region

<400> 3
gccttagaga ctattttagc aaa                                             23

<210> 4
<211> 23
<212> DNA
<213> Artificial Sequence

<220>
<223> potential target region

<400> 4
tagagactat tttagcaaat ttg                                               23


<210> 5
<211> 23
<212> DNA
<213> Artificial Sequence


<220>
<223> potential target nucleotide

<400> 5
gactatttta gcaaatttgg aga                                               23


<210> 6
<211> 23
<212> DNA
<213> Artificial Sequence


<220>
<223> potential target region

<400> 6
agcaaatttg gagaaattag aga                                               23


<210> 7
<211> 23
<212> DNA
<213> Artificial Sequence


<220>
<223> potential target region

<400> 7
agcaagtgta gataaagtat tag                                               23


<210> 8
<211> 23
<212> DNA
<213> Artificial Sequence


<220>
<223> potential target region

<400> 8
cacaagaaca aagaaaatat ttg                                               23


<210> 9
<211> 23

<212> DNA
<213> Artificial Sequence

<220>
<223> potential target region

<400> 9
aagaacaaag aaaatatttg tag                                              23

<210> 10
<211> 23
<212> DNA
<213> Artificial Sequence

<220>
<223> potential target region

<400> 10
gtggaagatg taaagcaata ttt                                              23

<210> 11
<211> 23
<212> DNA
<213> Artificial Sequence

<220>
<223> potential target region

<400> 11
tggaagatgt aaagcaatat ttc                                              23

<210> 12
<211> 23
<212> DNA
<213> Artificial Sequence

<220>
<223> potential target region

<400> 12
tgctgatgtt tgataaaact acc                                              23

<210> 13
<211> 23
<212> DNA
<213> Artificial Sequence

<220>
<223> potential target region

<400> 13
tggctttgtc acttttgaga atg                                              23

<210> 14
<211> 23
<212> DNA
<213> Artificial Sequence

<220>
<223> potential target region

<400> 14
ggctttgtca cttttgagaa tga                                              23

<210> 15
<211> 23
<212> DNA
<213> Artificial Sequence

<220>
<223> potential target region

<400> 15
cacttttgag aatgaagatg ttg                                              23

<210> 16
<211> 23
<212> DNA
<213> Artificial Sequence

<220>
<223> potential target region

<400> 16
aagatgttgt ggagaaagtc tgt                                              23

<210> 17
<211> 23
<212> DNA
<213> Artificial Sequence

<220>
<223> potential target region

<400> 17
gagaaagtct gtgagattca ttt                                              23

<210> 18
<211> 23
<212> DNA
<213> Artificial Sequence

<220>
<223> potential target region

<400> 18
ttcatttcca tgaaatcaat aat                                              23

<210> 19
<211> 23
<212> DNA
<213> Artificial Sequence

<220>
<223> potential target region

<400> 19
ttccatgaaa tcaataataa aat                                               23

<210> 20
<211> 23
<212> DNA
<213> Artificial Sequence

<220>
<223> potential target region

<400> 20
tccaagctat ggctatcagt tcc                                               23

<210> 21
<211> 23
<212> DNA
<213> Artificial Sequence

<220>
<223> potential target region

<400> 21
ttgattgcaa cggcctttac aaa                                               23

<210> 22
<211> 23
<212> DNA
<213> Artificial Sequence

<220>
<223> potential target region

<400> 22
cacgaatctg ctgtaatata aga                                               23

<210> 23
<211> 23
<212> DNA
<213> Artificial Sequence

<220>
<223> potential target region

<400> 23
aacagctttt aaatgtgtat ata                                               23

<210> 24
<211> 23
<212> DNA
<213> Artificial Sequence

<220>
<223> potential target region

<400> 24
cagcttttaa atgtgtatat aac                                               23

<210> 25
<211> 23
<212> DNA
<213> Artificial Sequence

<220>
<223> potential target region

<400> 25
agcttttaaa tgtgtatata acc                                               23

<210> 26
<211> 23
<212> DNA
<213> Artificial Sequence

<220>
<223> potential target region

<400> 26
tcccattctc cttttaaatc tct                                               23

<210> 27
<211> 23
<212> DNA
<213> Artificial Sequence

<220>
<223> potential target region

<400> 27
ctccttttaa atctctttga atc                                               23

<210> 28
<211> 23
<212> DNA
<213> Artificial Sequence

<220>

<223> potential target region

<400> 28
tccttttaaa tctctttgaa tca                                              23


<210> 29
<211> 23
<212> DNA
<213> Artificial Sequence


<220>
<223> potential target region

<400> 29
ctctttgaat cacatttggt agt                                              23


<210> 30
<211> 23
<212> DNA
<213> Artificial Sequence


<220>
<223> potential target region

<400> 30
atcacatttg gtagtgattt tga                                              23


<210> 31
<211> 23
<212> DNA
<213> Artificial Sequence


<220>
<223> potential target region

<400> 31
tggtagtgat tttgacttag tcc                                              23


<210> 32
<211> 23
<212> DNA
<213> Artificial Sequence


<220>
<223> potential target region

<400> 32
cacatagctt taatatctag ttc                                              23


<210> 33
<211> 23
<212> DNA
<213> Artificial Sequence

<220>
<223> potential target region

<400> 33
tagctttaat atctagttca aag                                               23


<210> 34
<211> 23
<212> DNA
<213> Artificial Sequence


<220>
<223> potential target region

<400> 34
agctttaata tctagttcaa agc                                               23


<210> 35
<211> 23
<212> DNA
<213> Artificial Sequence


<220>
<223> potential target region

<400> 35
ttcaaagcta accatagtat aat                                               23


<210> 36
<211> 23
<212> DNA
<213> Artificial Sequence


<220>
<223> potential target region

<400> 36
agctaaccat agtataattg tta                                               23


<210> 37
<211> 23
<212> DNA
<213> Artificial Sequence


<220>
<223> potential target region

<400> 37
aaccatagta taattgttat att                                               23


<210> 38
<211> 16
<212> DNA

<213> Artificial Sequence

<220>
<223> ASO 1

<400> 38
tgacttcttt cggctg                                                    16

<210> 39
<211> 16
<212> DNA
<213> Artificial Sequence

<220>
<223> ASO 3

<400> 39
ttggattaag gttgcc                                                    16

<210> 40
<211> 21
<212> DNA
<213> Artificial Sequence

<220>
<223> siRNA 1

<400> 40
tgacttcttt cggctgagct t                                              21

<210> 41
<211> 23
<212> RNA
<213> Artificial Sequence

<220>
<223> Sense AgomiR-7 (mimic of miR-107)

<400> 41
agcagcauug uacagggcua uca                                            23

<210> 42
<211> 23
<212> RNA
<213> Artificial Sequence

<220>
<223> Antisense AgomiR-7 (mimic of miR-107)

<400> 42
ugauagcccu guacaaugcu gcu                                            23

<210> 43

<211> 21
<212> DNA
<213> Artificial Sequence

<220>
<223> siRNA 2

<400> 43
agttttatca aacatcagca t                                                21

<210> 44
<211> 16
<212> DNA
<213> Artificial Sequence

<220>
<223> ASOS SCRAMBLE (control)

<400> 44
ttccctgaag gttcct                                                      16

<210> 45
<211> 20
<212> DNA
<213> Artificial Sequence

<220>
<223> GAPDH fwd

<400> 45
catcttccag gagcgagatc                                                  20

<210> 46
<211> 21
<212> DNA
<213> Artificial Sequence

<220>
<223> GAPDH rev

<400> 46
gttcacaccc atgacgaaca t                                                21

<210> 47
<211> 20
<212> DNA
<213> Artificial Sequence

<220>
<223> GPI fwd

<400> 47
cagggcatca tctgggacat                                                  20

<210> 48
<211> 20
<212> DNA
<213> Artificial Sequence

<220>
<223> GPI rev

<400> 48
tcttagccag ctgctttccc                                                    20

<210> 49
<211> 21
<212> DNA
<213> Artificial Sequence

<220>
<223> HPRT1 fwd

<400> 49
tgacactggc aaaacaatgc a                                                  21

<210> 50
<211> 21
<212> DNA
<213> Artificial Sequence

<220>
<223> HPRT1 REV

<400> 50
ggtccttttc accagcaagc t                                                  21

<210> 51
<211> 23
<212> DNA
<213> Artificial Sequence

<220>
<223> IGF1 fwd

<400> 51
ctcttcagtt cgtgtgtgga gac                                                23

<210> 52
<211> 22
<212> DNA
<213> Artificial Sequence

<220>
<223> IGF1 rev

<400> 52

cagcctcctt agatcacagc tc                                                22

<210> 53
<211> 22
<212> DNA
<213> Artificial Sequence

<220>
<223> MSI2 fwd

<400> 53
gcagacctca ccagatagcc tt                                                22

<210> 54
<211> 22
<212> DNA
<213> Artificial Sequence

<220>
<223> MSI2 rev

<400> 54
aagcctctgg agcgtttcgt ag                                                22

<210> 55
<211> 24
<212> DNA
<213> Artificial Sequence

<220>
<223> MSTN fwd

<400> 55
tgagaatggt catgatcttg ctgt                                              24

<210> 56
<211> 24
<212> DNA
<213> Artificial Sequence

<220>
<223> MSTN rev

<400> 56
   tcatcacagt caagaccaaa atcc                                             24

<210> 57
<211> 22
<212> DNA
<213> Artificial Sequence

<220>
<223> mTOR fwd

<400> 57
agcatcggat gcttaggagt gg                                                22

<210> 58
<211> 22
<212> DNA
<213> Artificial Sequence

<220>
<223> mTOR rev

<400> 58
cagccagtca tctttggaga cc                                                22

<210> 59
<211> 24
<212> DNA
<213> Artificial Sequence

<220>
<223> NFIX fwd

<400> 59
gagccctgtt gatgacgtgt tcta                                              24

<210> 60
<211> 24
<212> DNA
<213> Artificial Sequence

<220>
<223> NFIX rev

<400> 60
ctgcacaaac tccttcagtg agtc                                              24

<210> 61
<211> 22
<212> DNA
<213> Artificial Sequence

<220>
<223> P21 fwd

<400> 61
aggtggacct ggagactctc ag                                                22

<210> 62
<211> 22
<212> DNA
<213> Artificial Sequence

<220>
<223> P21 rev

<400> 62
tcctcttgga gaagatcagc cg                                                22

<210> 63
<211> 20
<212> DNA
<213> Artificial Sequence

<220>
<223> PKM fwd

<400> 63
ctgaaggcag tgatgtcgcc                                                   20

<210> 64
<211> 19
<212> DNA
<213> Artificial Sequence

<220>
<223> PKM rev

<400> 64
acccggaggt ccacgtctc                                                    19

<210> 65
<211> 21
<212> DNA
<213> Artificial Sequence

<220>
<223> ATP2A fwd

<400> 65
gatgatcttc aagctccggg c                                                 21

<210> 66
<211> 20
<212> DNA
<213> Artificial Sequence

<220>
<223> ATP2A rev

<400> 66
cagctctgcc tgaagatgtg                                                   20

<210> 67
<211> 22
<212> DNA
<213> Artificial Sequence

<220>
<223> TGFBR1 fwd

<400> 67
gacaacgtca ggttctggct ca                                                22


<210> 68
<211> 22
<212> DNA
<213> Artificial Sequence


<220>
<223> TGFBR1 rev

<400> 68
ccgccacttt cctctccaaa ct                                                22


<210> 69
<211> 2619
<212> DNA
<213> Artificial Sequence


<220>
<223> AAV9-hDES-mMSI2(V4)-V5-T2A-Nanoluc

<400> 69
gctagcgcgg ccgcacccat gcctcctcag gtaccccctg ccccccacag ctcctctcct      60

gtgccttgtt tcccagccat gcgttctcct ctataaatac ccgctctggt atttggggtt      120

ggcagctgtt gctgccaggg agatggttgg gttgacatgc ggctcctgac aaaacacaaa      180

cccctggtgt gtgtgggcgt gggtggtgtg agtaggggga tgaatcaggg agggggcggg      240

ggacccaggg ggcaggagcc acacaaagtc tgtgcggggg tgggagcgca catagcaatt      300

ggaaactgaa agcttatcag accctttctg gaaatcagcc cactgtttat aaacttgagg      360

ccccaccctc gagataacca gggctgaaag aggcccgcct gggggctgca gacatgcttg      420

ctgcctgccc tggcgaagga ttggcaggct tgcccgtcac aggacccccg ctggctgact      480

caggggcgca ggcctcttgc gggggagctg gcctccccgc ccccacggcc acgggccgcc      540

ctttcctggc aggacagcgg gatcttgcag ctgtcagggg aggggaggcg ggggctgatg      600

tcaggaggga tacaaatagt gccgacggct gggggccctg tctcccctcg ccgcatccac      660

tctccggccg gccgcctgcc cgccgcctcc tccgtgcgcc cgccagcctc gcccgcgccg      720

tcaccggttc gaacaggtaa gcgcccctaa aatccctttg gcacaatgtg tcctgagggg      780

agaggcagcg acctgtagat gggacggggg cactaaccct cagggtttgg ggttctgaat      840

gtgagtatcg ccatctaagc ccagtatttg gccaatctca gaaagctcct ggctccctgg      900

aggatggaga gagaaaaaca aacagctcct ggagcaggga gagtgttggc ctcttgctct      960

ccggctccct ctgttgccct ctggtttctc cccaggttcg aaggatccat ggaggcaaat 1020

gggagcccag gcacctcggg cagcgccaac gactcccagc acgaccccgg taaaatgttt 1080

atcggtggac tgagctggca gacctcacca gatagcctta gagactattt tagcaaattt 1140

ggagaaatta gagaatgtat ggtcatgaga gatcccacaa cgaaacgctc cagaggcttc 1200

ggtttcgtca ccttcgcaga cccagcaagt gtagataaag tattaggtca gccccaccat 1260

gagttagatt ccaagacgat tgacccaaaa gttgcatttc ctcgtcgagc gcaacctaag 1320

atggtcacaa gaacaaagaa aatcttcgta ggaggattgt ctgcaaacac agtagtggaa 1380

gatgtaaagc agtatttcga gcagtttggc aaggtagagg atgcgatgct gatgttcgac 1440

aaaaccacca acaggcacag agggtttggc tttgtcacct ttgagaatga agacgttgtg 1500

gagaaagtct gtgagattca tttccatgaa atcaataata aaatggtaga atgtaagaaa 1560

gctcagccga aagaagtcat gttcccacct gggacaagag gccgggcccg ggggctgcca 1620

tacaccatgg atgcgttcat gcttggcatg gggatgctgg gctaccccaa ctttgtggca 1680

acctatggca gaggctaccc cggatttgct cctagctatg gctaccagtt cccaggcttc 1740

ccggcggcag cttatggacc agtggcagcg gcagctgtgg cagcggctcg aggatcaggc 1800

tccaacccgg cgcggcccgg aggcttcccg ggggccaaca gcccaggacc tgtcgccgat 1860

ctctacggcc ctgccagcca ggactccgga gtggggaatt acataagcgc ggccagccca 1920

cagccgggct ccggcttcgg ccacggcata gctggacctt tgattgcaac ggcctttaca 1980

aatggatacc actgaggtaa gcctatccct aaccctctcc tcggtctcga ttctacggag 2040

ggcagaggaa gtctgctaac atgcggtgac gtcgaggaga atcctggccc aggatccatg 2100

gtcttcacac tcgaagattt cgttggggac tggcgacaga cagccggcta caacctggac 2160

caagtccttg aacagggagg tgtgtccagt ttgtttcaga atctcggggt gtccgtaact 2220

ccgatccaaa ggattgtcct gagcggtgaa aatgggctga agatcgacat ccatgtcatc 2280

atcccgtatg aaggtctgag cggcgaccaa atgggccaga tcgaaaaaat ttttaaggtg 2340

gtgtaccctg tggatgatca tcactttaag gtgatcctgc actatggcac actggtaatc 2400

gacggggtta cgccgaacat gatcgactat ttcggacggc cgtatgaagg catcgccgtg 2460

ttcgacggca aaaagatcac tgtaacaggg accctgtgga acggcaacaa aattatcgac 2520

gagcgcctga tcaaccccga cggctccctg ctgttccgag taaccatcaa cggagtgacc 2580

ggctggcggc tgtgcgaacg cattctggcg taagtatac 2619

<210> 70
<211> 39
<212> RNA
<213> Artificial Sequence

<220>
<223> Fig. 1 Seq pri-miR-7-1

<400> 70
gugauuuugu uguuuuuagc uaaaucgaca acaaaucac                              39

## Description

### TECHNICAL FIELD OF THE INVENTION

The present invention relates to the medical field. In particular, the present invention relates to an inhibitor of RNA-binding protein Musashi (MSI2) or an analogue thereof for use in the treatment of myotonic dystrophy.

### BACKGROUND OF THE INVENTION

Myotonic dystrophy (MD) is characterized by progressive skeletal muscle weakness. People with this disorder often have prolonged muscle contractions (myotonia) and are not able to relax certain muscles after use. Also, affected people may have slurred speech or temporary locking of their jaw. The Myotonic dystrophy foundation describes in their website (https://www.myotonic.org/what-dm/how-dm-affects-your-body) the symptoms of DM at different ranges of severity.

There are two major types of myotonic dystrophy: type 1 (MD1) and type 2 (MD2). Their signs and symptoms are similar, although type 2 tends to be milder than type 1. The two types of myotonic dystrophy are caused by mutations in different genes. MD1 is a life-threatening and chronically debilitating disease caused by an expansion of the CTG triplet repeat in the 3' untranslated region of the DM1 protein kinase (DMPK) gene. MD1 is autosomal dominant and can affect from newborns to elderly people. Adult-onset patients may become physically disabled and may have a shortened life-span. Most symptoms are neuromuscular, including muscle weakness (myopathy), muscle stiffness and trouble relaxing muscles (myotonia), and progressive muscle wasting (atrophy), but MD1 is characteristically multisystemic and also affects the heart, the brain, and the smooth musculature. Pneumonia and respiratory distress due to muscle weakness and atrophy, and heart failure, are the most frequent causes of death. MD2 is also characterized by progressive muscle wasting and weakness. Symptoms typically begin in a person's twenties, and patients with this condition often have prolonged muscle contractions and are not able to relax certain muscles after use. Further, affected people may have slurred speech, temporary locking of their jaw and muscle pain and weakness that mainly affects the neck, shoulders, elbows, and hips. Less common symptoms in MD2 include abnormalities of the electrical signals that control the heartbeat (cardiac conduction defects), clouding of the lens in the eyes (cataracts), and diabetes.

In MD1, mutant DMPK transcripts in skeletal muscle, heart, and brain tissue get retained in the cell nucleus into microscopically visible ribonuclear foci. CTG trinucleotide expansions fold into stable double-stranded stem-loop that sequester the splicing factors Muscleblind-like (MBNL) proteins. CUGBP Elav-like family member 1 (CELF1) regulates alternative splicing antagonistically to MBNL1. In contrast to MBNL1, CELF1 is not sequestered into ribonuclear foci, but is hyper-activated and stabilized in the cell nucleus in MD1.

As mentioned, muscle wasting is a key symptom in MD1 patients. Multiple alterations have been described to explain muscle dysfunction in MD1, including an increased autophagy. Muscle wasting and histological defects were observed in an inducible mouse model that express 960 CUG repeats that showed an imbalance between anabolic and catabolic pathways that normally regulate muscle mass. Specifically, the upregulation of proteins involved in autophagic pathway has been described. Similarly, in a Drosophila MD1 model and in MD1 myotubes it was demonstrated that autophagy flux was overactivated and genetic or chemical blockade of this pathway, by mTOR upregulation or chloroquine treatment, resulted in an improvement of muscle function and molecular markers of muscle atrophy. Similarly, involvement of Muscleblind proteins with autophagy was demonstrated. In a Drosophila inducible model, overexpression of mbl isoform C (the ortholog in Drosophila of MBNL) was enough to recover muscle area and expression of autophagy related genes. Moreover, in MD1 myotubes and MD1 model mice, treatment with chloroquine, an autophagy blocker, resulted in increased levels of MBNL1 and 2.

Additional studies demonstrated that miRNAs levels are also altered in skeletal muscle, heart, and blood samples from MD1 patients and MD1 muscle cells and mouse models. Specifically, it was reported that miR-7 levels are impaired in human biopsies from skeletal muscle and MD1 muscle cells. Independent studies demonstrated that this miRNA represses autophagy through up-regulation of mTOR signalling and direct inhibition of some autophagy genes (ATG7, ULK2 and ATG4A). miR-7 biogenesis is regulated by Musashi homolog 2 (MSI2) and Hu antigen R (HuR). However, MSI2 and HuR are proteins involved in complex and numerous cellular pathways, and their direct implications in miR-7 levels as well as their contribution to MD1 pathology in different tissues remains unknown.

In order to shed some light into the molecular pathways that regulate miR-7 biogenesis, we study herein the impact of MSI2 levels and its overexpression in DM cells and animal model, demonstrating that the intracellular concentration of MSI2 protein is an important factor directly related to MD1 pathology and thus we propose the use of inhibitors directed against MSI2 protein to increase miR-7 intracellular levels for the treatment of myotonic dystrophy.

### DESCRIPTION OF THE FIGURES

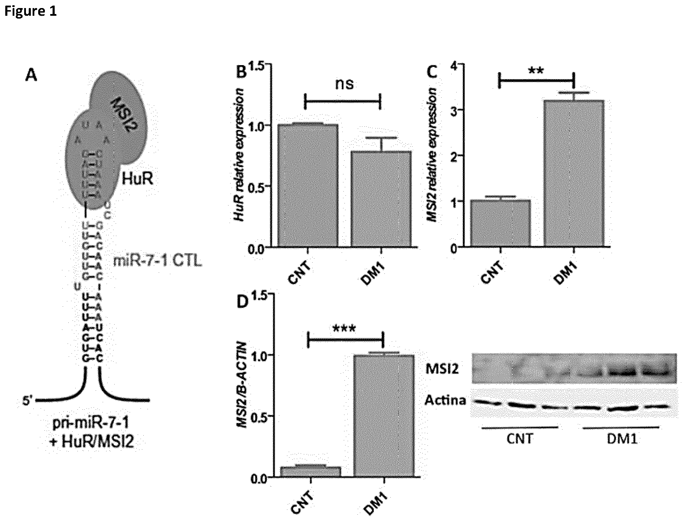

**Figure 1****. The processing machinery of pri-miR-7 is altered in MD1 myotubes.** (A) Schematic representation of the secondary structure of pri-miR-7 with the conserved terminal loop of miR-7 showing the complex formed by pre-miR-7-1/HuR/MSI2 (obtained from Kumar et al., 2017). (B, C) Relative expression levels of HuR and MSI2 transcripts in healthy control and MD1 myotubes. GAPDH expression was used as internal reference. (D) Western blot quantification of total levels of MSI2 in control and MD1 cells. β-ACTIN was used as an endogenous control to normalize protein levels (n=3).

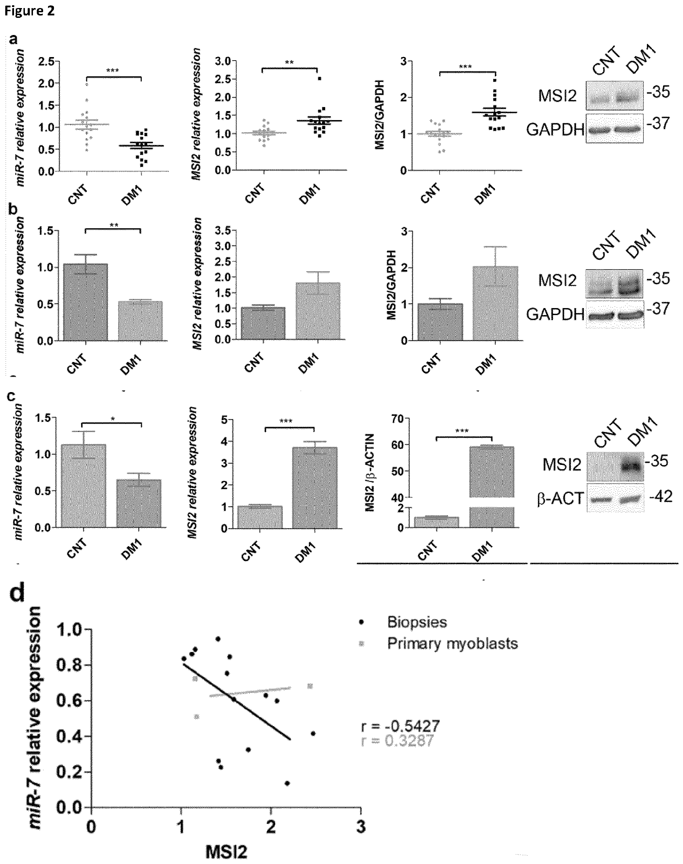

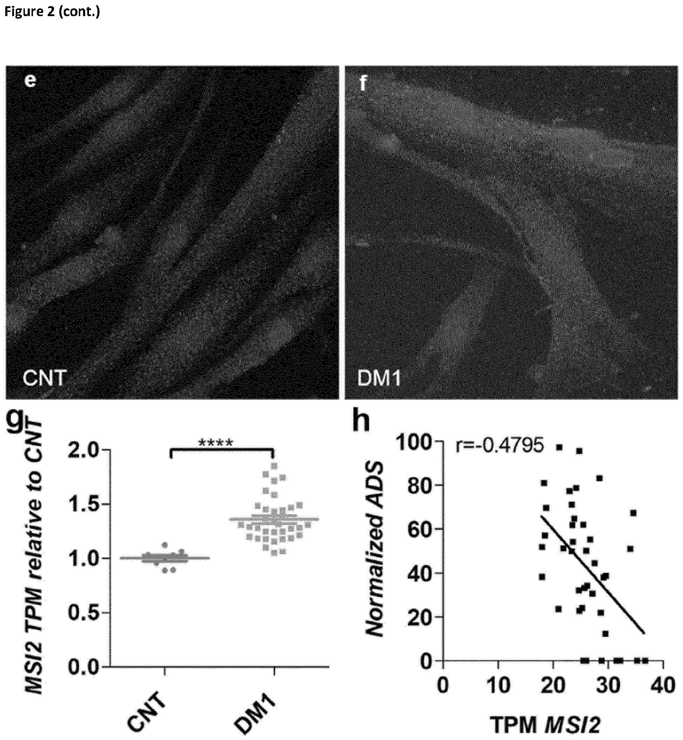

**Figure 2****. MSI2 is upregulated in MD1 samples.** Quantification of relative expression of miR-7 (left), MSI2 transcripts (middle), and MSI2 protein levels (right), and representative blots from patient-derived biopsies **(A),** primary myoblasts **(B)** and myotubes **(C).** In quantifications by RT-qPCR, U1 and U6 were used as endogenous controls of miR-7 while MSI2 levels are relative to GADPH and GPI expression. Data were obtained using the 2-ΔΔCt method (n=3). In western blots, β-ACTIN or GAPDH were used as endogenous controls to normalize protein levels (n=3). The graphs show the median with interquartile range (A) and mean±s.e.m (B and C). **(D)** Pearson's correlation between miR-7 relative expression and MSI2 protein levels in biopsies and transdifferentiated myotubes (TDMs). Representative confocal images of MSI2 immunostained healthy controls (CNT) **(E)** and MD1 myotubes **(F).** Nuclei were counterstained with DAPI. Scale bar=20 µm. Statistical analyses are Student's t-tests. * P<0.05, ** P<0.01, *** P<0.001, **** P<0.0001. **(G)** The graph represents MSI2 transcripts per million in RNA-seq experiments from biopsies from 40 MD1 patients and 10 controls according to Wang et al. 2019 [Wang, E. T. et al. Transcriptome alterations in myotonic dystrophy skeletal muscle and heart. Hum Mol Genet 28, 1312-1321, doi:10.1093/hmg/ddy432 (2019)]. The graph shows the median with interquartile range **(H)** Pearson's correlations between MSI2 transcripts per million in MD1 biopsies and ankle dorsiflexion strength (ADS) measured in biopsies donors. Data according to Wang et al. 2019.

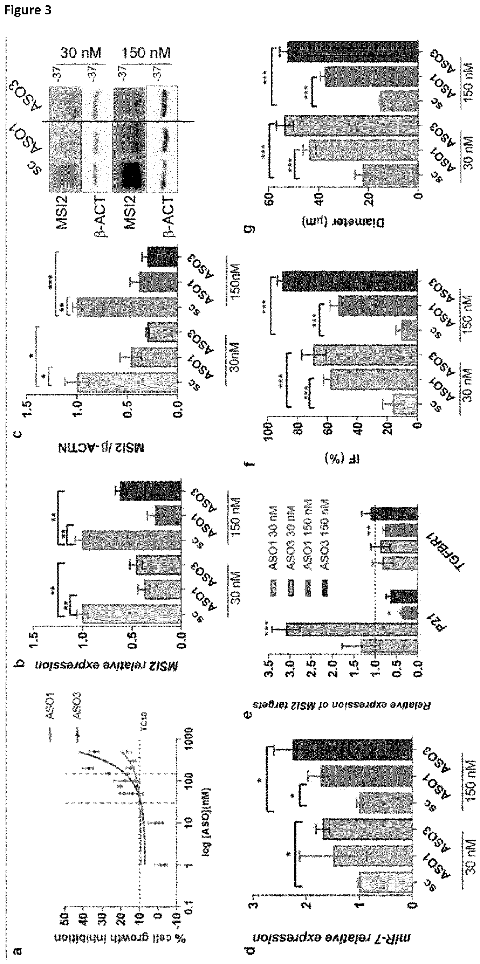

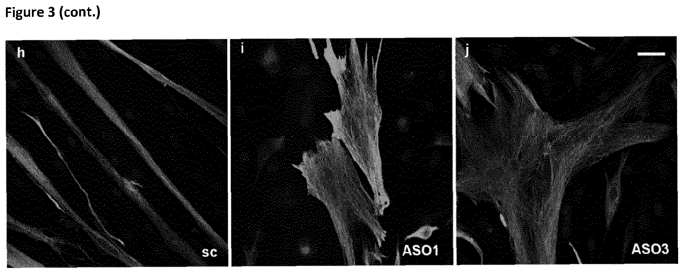

**Figure 3****. Atrophic muscle markers ameliorate after MSI2 reduction using a gapmer in MD1 TDMs. (A)** Cell growth inhibition assay by MTS method. Human CNT myotubes were transfected with increasing concentrations of AS01, 2 and 3 (n =4). TC10 (31.2 nM, 110 nM and 33.45 nM for ASO1, 2 and 3, respectively) were obtained using the least squares non-lineal regression model. Vertical dashed lines indicate used concentrations in the following experiments: 30 and 150 nM. **(B)** MSI2 quantification by RT-qPCR relative to GAPDH and GPI and **(C)** MSI2 quantification by Western blot. Graph and representative blot image of MSI2 immunodetection are shown. β-ACTIN expression was used as an endogenous control. **(D)** Quantification of miR-7 relative to U1 and U6 in MD1 TDMs transfected with ASOs. Data were obtained using the 2^{-ΔΔCt} method (n=3). **(E)** Quantification by RT-qPCR relative to GAPDH and GPI of the expression levels of P21 and TGFBR1 after treatment of the muscle cells with the indicated concentrations of gapmers targeting MSI2. The statistical analysis was performed comparing values from treated cells with their respective scramble (black dashed line). Data were obtained using the 2^{-ΔΔCt} method (n=3). **(F)** Analysis of myogenic fusion index and **(G)** myotube diameter of MD1 myoblasts transfected with the indicated concentrations of ASOs or scramble (n=10-15). **(H-J)** Representative confocal images of Desmin-immunostained human fibroblasts transdifferentiated for 7 days after ASOs transfection into MD1 cells and their respective scramble control at 150 nM. Nuclei were counterstained with DAPI. Scale bar, 40 µm. The statistical analysis was performed comparing values from treated cells with their respective scramble. The bar graphs show mean±s.e.m. * P<0.05, ** P<0.01, *** P<0.001, according to Student's t-test.

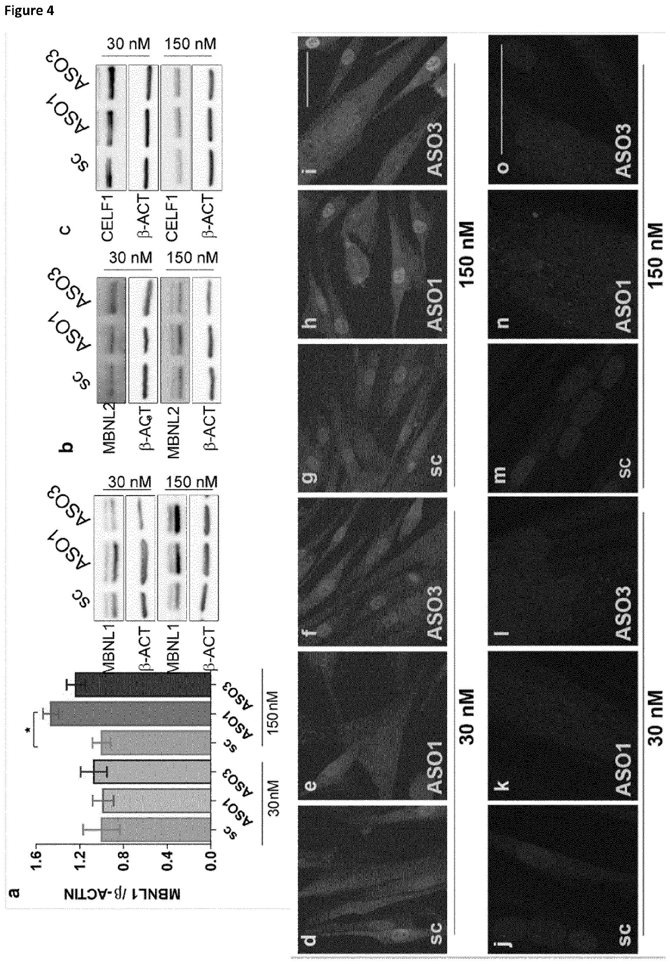

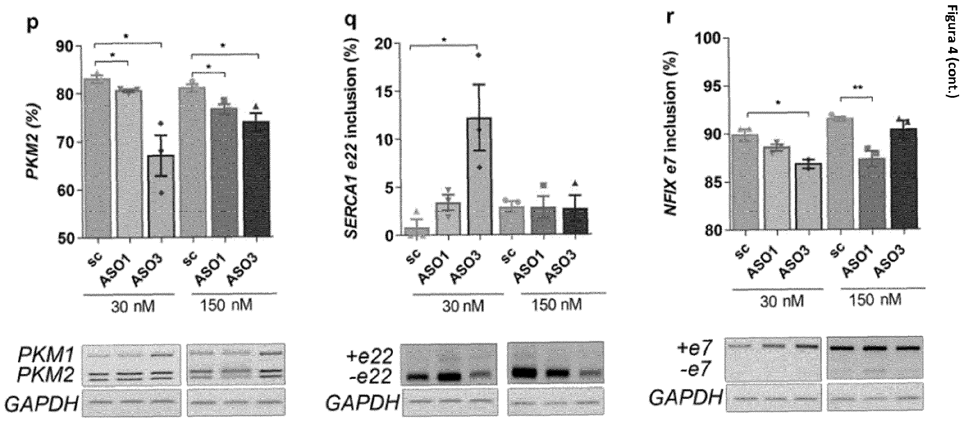

**Figure 4** **Degradation of MSI2 transcripts enhances MBNL1 levels in 7-days differentiated TDMs. (A)** Quantification and representative blots of MBNL1 levels in protein extracts obtained from MD1 TDMs treated with the indicated concentrations of MSI2 or scramble ASOs. **(B, C)** Representative blots used for quantitative analyses of MBNL2 and CELF1 levels. β-ACTIN expression was used as an endogenous control (n=3). Representative confocal images of **(D-I)** MBNL1 or **(J-O)** MBNL2 immunodetection in MD1 TDMs treated for 48 h with scramble (sc) **(D, J, G, M),** ASO1 **(E,K,H,N)** or ASO3 **(F,L,I,O)** at the indicated concentration. Nuclei were counterstained with DAPI (scale bar, 50 µm). **(P-R)** Semiquantitative RT-PCR analyses and representative gels of splicing events altered in **(P)** PKM isoforms represented as the % of PKM2 and **(Q)** SERCA1 (exon 13), and **(R)** NFIX (exon 7) represented as the % of exon inclusion in MD1 cells. GAPDH was used as internal control (n=3). In all cases, the statistical analyses were performed comparing values from treated cells with those obtained for cells treated with the scramble ASO at the same concentration. The bar graphs show mean±s.e.m. * P<0.05, ** P<0.01, *** P<0.001, according to Student's t-test.

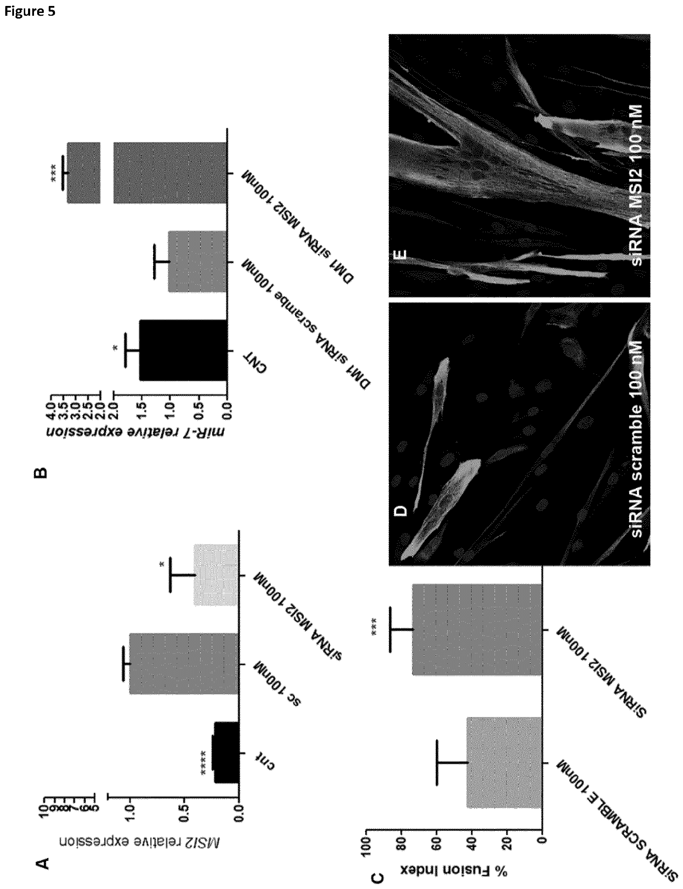

**Figure 5****. Silencing MSI2 with a combination of two siRNAs is effective in MD1 muscle cells. (A)** MSI2 relative expression analyzed by RT-qPCR. GAPDH and GPI were used as reference genes (n=3). **(B)** Relative expression levels of miR-7 measured by RT-qPCR (calculated using the 2^{-ΔΔCt} method). Data were normalized to U1 and U6 levels. **(C)** Quantification of the fusion index of MD1 myotubes treated with the indicated siRNA. **(D, E)** Representative confocal images of Desmin-immunostained human fibroblasts transdifferentiated for 7 days after siRNA transfection into MD1 cells. Nuclei were counterstained with DAPI. Error bars indicate s.e.m. * P<0.05, *** P<0.001, **** P<0.0001 according to Student's t-test.

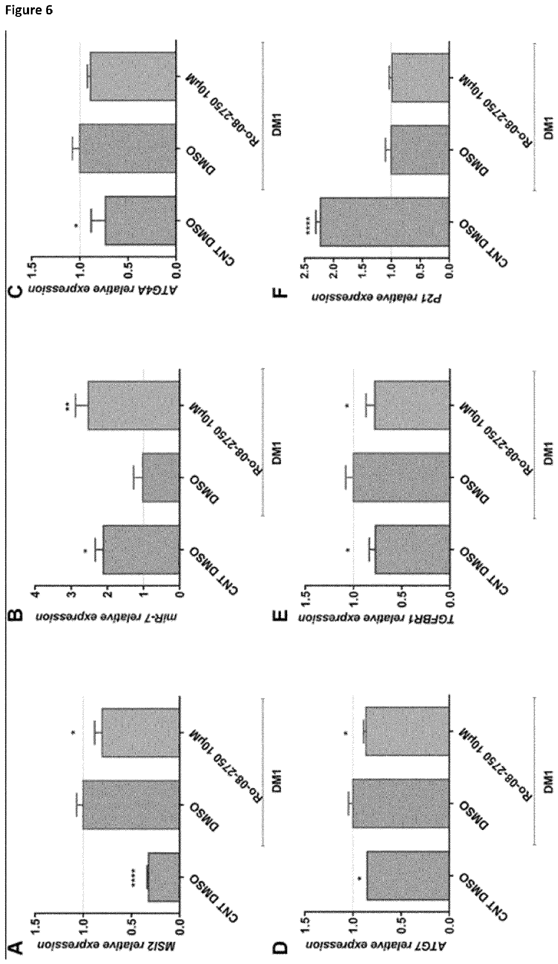

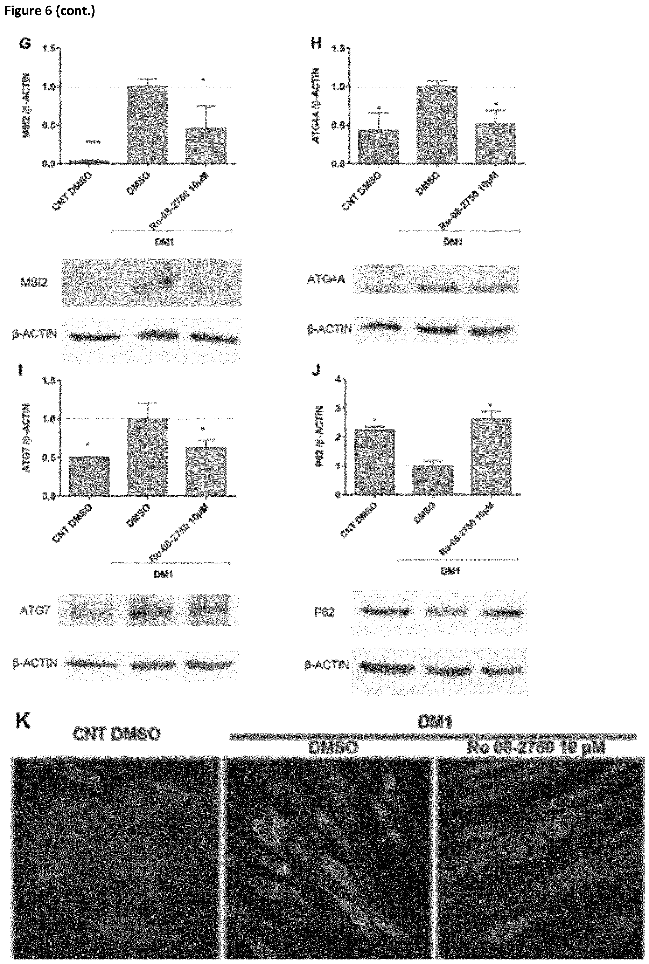

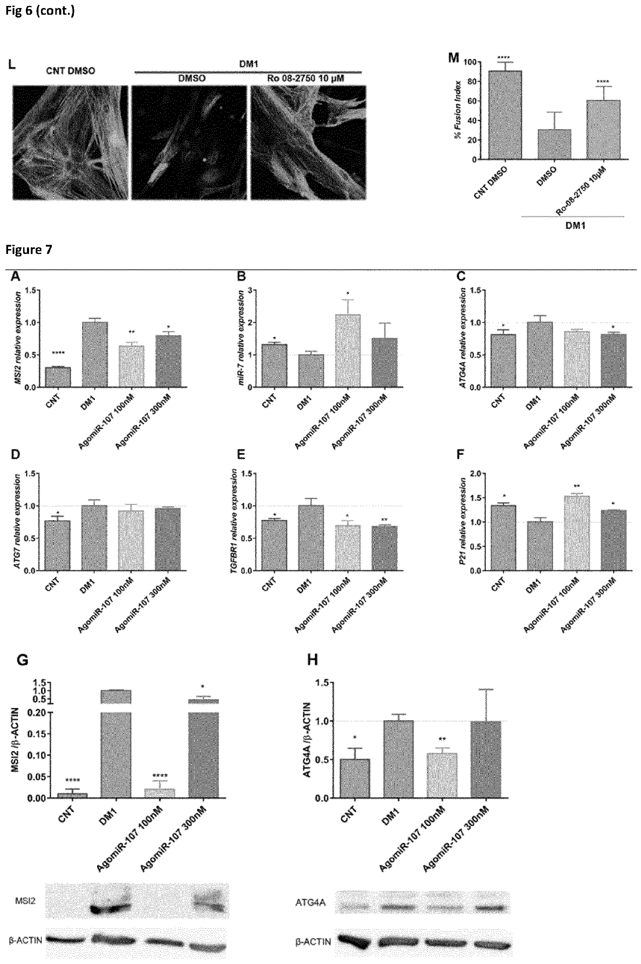

**Figure 6****. Downregulation of MSI2 by treatment with a small molecule improves MD1-related phenotypes. (A)** MSI2 relative expression analyzed by RT-qPCR. GAPDH, HPRT1 and GPI were used as reference genes (n=3). **(B)** Relative expression levels of miR-7 measured by RT-qPCR (calculated using the 2^{-ΔΔCt} method). Data were normalized to U1 and U6 levels. Relative expression of **(C)** ATG4A, **(D)** ATG7, **(E)** TGFBR1 and **(F)** P21 analyzed by RT-qPCR. GAPDH and GPI were used as reference genes (n=3). Quantification by western blot of **(G)** MSI2, **(H)** ATG4A, **(I)** ATG7 and **(J)** P62. Representative blots used for quantification are shown below the bar graphs. β-ACTIN was used as endogenous control to normalize protein levels (n=3). **(K)** Representative confocal images of LysoTracker staining of control and MD1 cells treated with the indicated concentrations of the small molecules. Nuclei were counterstained with DAPI. **(L)** Representative confocal images of Desmin-immunostained human fibroblasts transdifferentiated for 7 days into myotubes. Nuclei were counterstained with DAPI. **(M)** Quantification of the fusion index of MD1 TDMs treated with the indicated compounds and concentrations. Error bars indicate s.e.m. * P<0.05, ** P<0.01 *** P<0.001, **** P<0.0001 according to Student's t-test. Legend: **CNT DMSO:** control healthy cells (CNT) treated with DMSO. **DMSO:** MD1 cells treated with DMSO. **Ro-08-2750 10 µM:** MD1 cells treated with Ro-08-2750 at 10 µM.

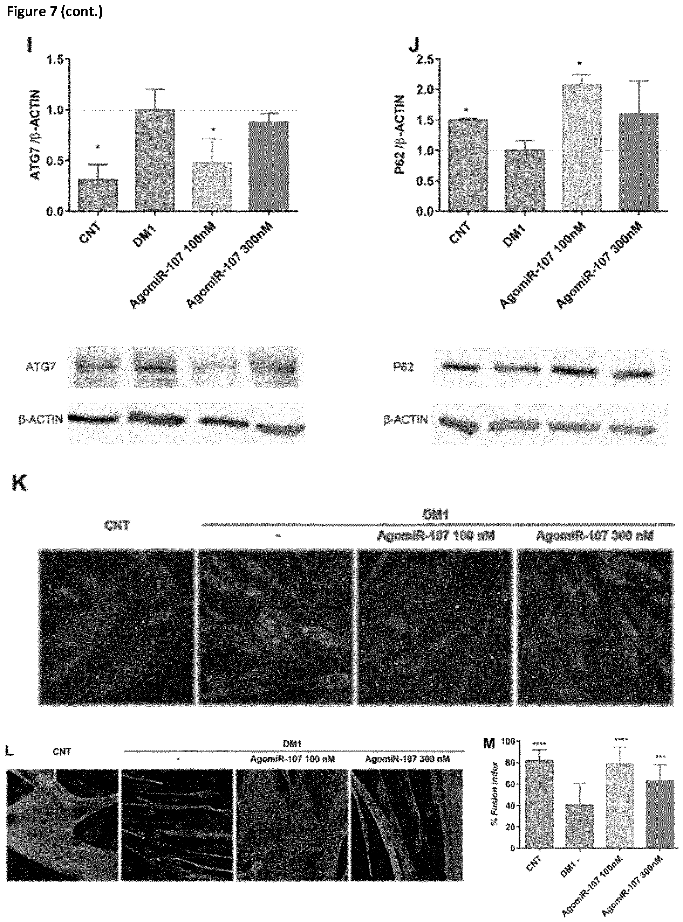

**Figure 7****. Inhibition of MSI2 expression by agomiR-107 addition is beneficial for MD1 cells. (A)** MSI2 relative expression analyzed by RT-qPCR. GAPDH, HPRT1 and GPI were used as reference genes (n=3). **(B)** Relative expression levels of miR-7 measured by RT-qPCR (calculated using the 2-^{ΔΔCt}method). Data were normalized to U1 and U6 levels. Relative expression of **(C)** ATG4A, **(D)** ATG7, **(E)**TGFBR1 **(F)** and P21 analyzed by RT-qPCR. GAPDH, HPRT1 and GPI were used as reference genes (n=3). Quantification by western blot of **(G)** MSI2, **(H)** ATG4A, **(I)** ATG7 and **(J)** P62. Representative blots used for quantification are shown below the bar graphs. β-ACTIN was used as endogenous control to normalize protein levels (n=3). **(K)** Representative confocal images of LysoTracker staining of control and MD1 cells treated with the indicated concentrations of the small molecules. Nuclei were counterstained with DAPI. **(L)** Representative confocal images of Desmin-immunostained human fibroblasts transdifferentiated for 7 days. Nuclei were counterstained with DAPI . **(M)** Quantification of the fusion index of MD1 TDMs treated with the indicated compounds and concentrations. Error bars indicate s.e.m. * P<0.05, ** P<0.01 *** P<0.001, **** P<0.0001 according to Student's t-test. Legend: **CNT:** control healthy cells. **MD1:** untreated MD1 cells. **AgomiR-107 100 nM:** MD1 cells treated with agomiR-107 at 100 nM. **AgomiR-107 300 nM:** MD1 cells treated with agomiR-107 at 300 nM.

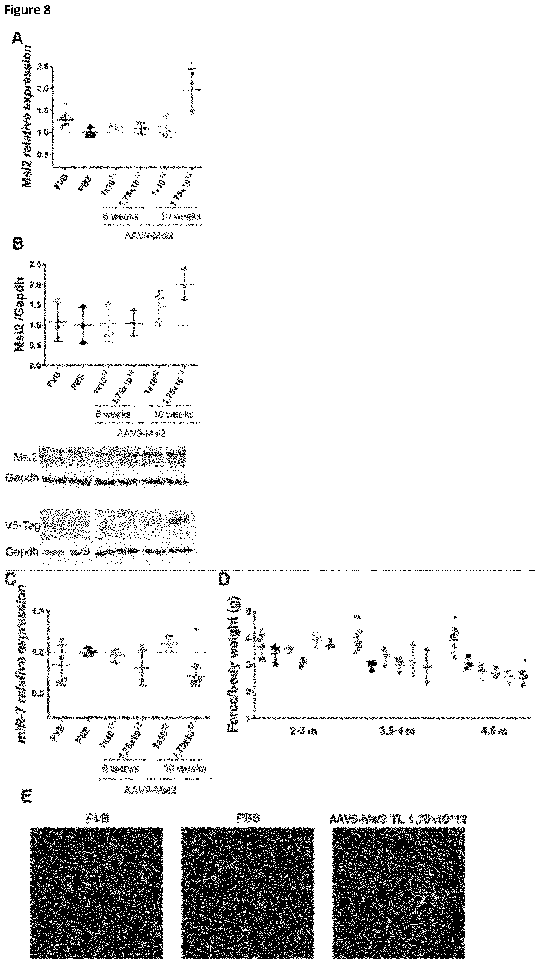

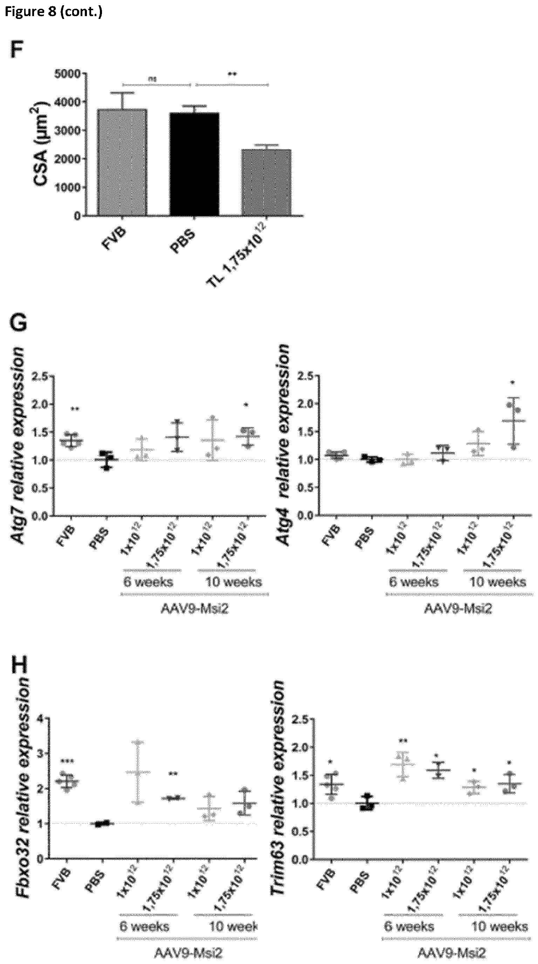

**Figure 8****. MSI2 overexpression has** a **deleterious effect in MD1 mouse muscles. (A)** MSI2 relative expression analyzed by RT-qPCR **(B)** and western blot, at the indicated post-injection times. Protein levels are relative to Gapdh, which was used as an internal control, and correspond to the signals obtained with the anti-MSI2 antibody (bar graph). **(B)** Representative blots used for quantification are shown below the bar graph using an anti-MSI2 (upper gels) or anti-V5-tag (lower gels). **(C)** Relative expression levels of miR-7 measured by RT-qPCR (calculated using the 2^{-ΔΔCt} method). Data were normalized to U1 and U6 levels. **(D)** Forelimb grip strength in 2-3 months-old mice (before injection), 3.5-4 months-old mice (intermediate time point) and in 4.5 months-old mice (before euthanasia). Data were normalized to body weight. **(E)** Representative confocal images of muscle fibers of the indicated mouse groups stained with WGA and **(F)** cross-sectional muscle area quantification. Nuclei were counterstained with DAPI. **(G-H)** Relative expression of the indicated genes analyzed by RT-qPCR. The average of Gapdh, Gtfb2, and Hprt1 expression levels was used as reference in A, G, and H (n=3). Error bars indicate s.e.m. * P<0.05, ** P<0.01 according to Student's t-test. Legend: **FVB:** control mice treated with PBS; **PBS:** HSA^{LR} mice treated with PBS; **1x10¹² 6 weeks:** HSA^{LR} mice treated with AAV at a dose of 1x10¹² vg/mice and sacrificed 6 weeks after the injection. **1.75x10¹² 6 weeks:** HSA^{LR} mice treated with a dose of AAV **1.75x10¹²** vg/mice and sacrificed 6 weeks after the injection; **1x10¹² 10 weeks:** HSA^{LR} mice treated with AAV at a concentration of 1x10¹² vg/mice and sacrificed 10 weeks after the injection. **1.75x10¹² 10 weeks:** HSA^{LR} mice treated with AAV at a dose of **1.75x10¹²** vg/mice and sacrificed 10 weeks after the injection.

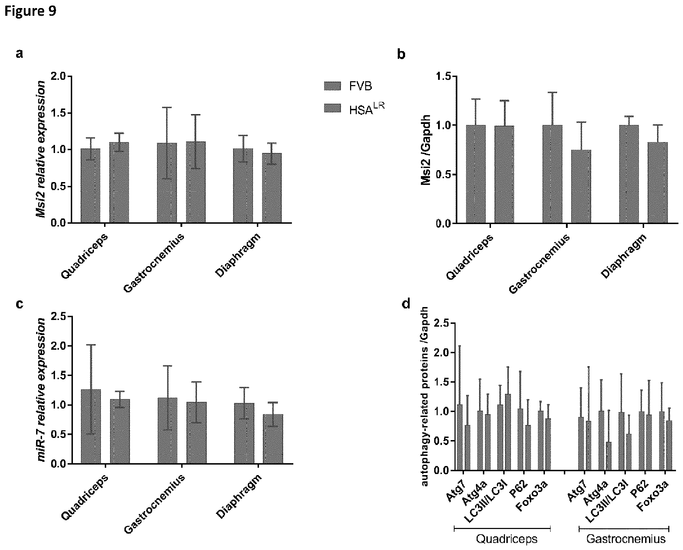

**Figure 9****. MSI2, miR-7 and autophagy are not altered in HSA^{LR} mice in comparison to control mice (FVB). (A)** Quantification of relative expression of MSI2 transcripts, **(B)** MSI2 protein levels, **(C)** miR-7 levels and (D) autophagy-related protein levels from diaphragm, gastrocnemius, and quadriceps muscles of 4.5-months HSA^{LR} and control (FVB) mice. U1 and U6 were used as endogenous controls of miR-7 while MSI2 levels are relative to GAPDH, Gtf2b and Hprt1 expression. Data were obtained using the 2^{-ΔΔCt} method (n=4-8). In western blots GAPDH was used as endogenous control to normalize protein levels (n=4-8). The graphs show the median with interquartile range and mean±s.e.m.

### BRIEF DESCRIPTION OF THE INVENTION

In a first aspect, the present invention relates to an inhibitor of RNA-binding protein Musashi (MSI2) function or an analogue thereof for use in the treatment of myotonic dystrophy in a mammal, wherein the inhibitor is capable of reducing in a myotonic dystrophy (MD) cell or in a cell with the phenotype of a MD cell, at least one of
i) MSI2 protein activity,
ii) intracellular transcript levels of MSI2, and/or
iii) intracellular protein levels of MSI2,
thereby causing in the same MD cell an increase in the intracellular miR-7 levels of at least 1.5-fold of the intracellular miR-7 levels of a reference cell, as measured by a gene expression quantification technique such as quantitative reverse transcription PCR (RT-qPCR), Northern blotting, or RNA-sequencing (RNA-Seq) techniques,
wherein the cell with the phenotype of a MD cell is selected from the group of muscular cells, nervous cell, or cardiac cells, and
wherein the reference cell is immortalized skin fibroblasts expressing conditional MyoD.

The MSI2 inhibitor for use according to the first aspect, wherein the increase in the intracellular miR-7 level is of at least 1.7-fold of the intracellular miR-7 levels of a reference cell.

The MSI2 inhibitor for use according to the first aspect or to any of the previous aspects, wherein the MD cell or a cell with the phenotype of a MD cell is a myocyte.

The MSI2 inhibitor for use according to the first aspect or to any of the previous aspects, wherein the increase in the intracellular miR-7 level is of at least 1.7-fold of the intracellular miR-7 levels of a reference cell and wherein the MD cell or a cell with the phenotype of a MD cell is a myocyte.

The MSI2 inhibitor for use according to the first aspect or to any of the previous aspects, wherein said inhibitor is for use in the treatment of myotonic dystrophy type 1.

The MSI2 inhibitor for use according the first aspect or to any of the previous aspects, wherein the mammal is a human being.

The MSI2 inhibitor for use according to the first aspect or to any of the previous aspects, wherein the inhibitor reduces the intracellular transcript levels of MSI2.

The MSI2 inhibitor for use according to the first aspect or to any of the previous aspects, wherein the inhibitor is an oligonucleotide molecule which comprises a fragment composed of a succession of nucleotide units, wherein the oligonucleotide is selected from the group consisting of small interfering RNAs, antisense oligonucleotides, gapmers, morpholino oligomers, FANA oligonucleotides, agomiRs, miRNA mimics, antagomiRs, blockmiRs, PNAs, LNAs (locked nucleic acid antisense oligonucleotides), splice-switching oligonucleotides (SSOs), LNA-based splice-switching oligonucleotides (LNA SSOs), LNA/DNA mixmers or miRNA sponges.

The oligonucleotide molecule for use according to the first aspect or to any of the previous aspects, wherein said oligonucleotide molecule comprises at least 5 consecutive nucleotides in length that have at least 80% identity over the complementary sequence of the full-length sequence of SEQ ID: 1 or, preferably, SEQ ID: 2.

The oligonucleotide molecule for use according to the first aspect or to any of the previous aspects, wherein said oligonucleotide comprises at least 7 consecutive nucleotides in length that have at least 90% identity over the complementary sequence of the full length sequence of SEQ ID: 1 or, preferably, SEQ ID: 2.

The oligonucleotide molecule for use according to the first aspect or to any of the previous aspects, wherein said oligonucleotide comprises at least 10-25 nucleotides in length, from which at least 5 consecutive nucleotides in length have at least 80% identity over the complementary sequence of the full-length sequence of SEQ ID: 1 or, preferably, SEQ ID: 2.

The oligonucleotide molecule for use according to the first aspect or to any of the previous aspects, wherein said oligonucleotide comprises at least 10-25 nucleotides in length, from which at least 7 consecutive nucleotides in length have at least 90% identity over the complementary sequence of the full length sequence of SEQ ID: 1 or, preferably SEQ ID: 2.

The oligonucleotide molecule for use according to the first aspect or to any of the previous aspects, wherein said oligonucleotide comprises at least 10-25 nucleotides in length, from which at least 7 consecutive nucleotides in length have at least 80% identity over the complementary sequence of the full sequence of any the oligonucleotides of SEQ ID NO:3 to SEQ ID NO:37.

The oligonucleotide molecule for use according to the first aspect or to any of the previous aspects, wherein said oligonucleotide comprises at least 10-25 nucleotides in length, from which at least 7 consecutive nucleotides in length have at least 90% identity over complementary sequence of the full sequence of any of the oligonucleotides of SEQ ID NO:3 to SEQ ID NO:37.

The oligonucleotide molecule for use according to the first aspect or to any of the previous aspects, wherein said oligonucleotide comprises at least 10-25 nucleotides in length, from which at least 7 consecutive nucleotides in length have at least 80% identity over the full sequence of any the oligonucleotides of SEQ ID NO:38 to 43.

The oligonucleotide molecule for use according to the first aspect or to any of the previous aspects, wherein said oligonucleotide comprises at least 10-25 nucleotides in length, from which at least 7 consecutive nucleotides in length have at least 90% identity over the full sequence of any of the oligonucleotides SEQ ID NO:38 to 43.

The oligonucleotide molecule for use according to the first aspect or to any of the previous aspects, wherein said oligonucleotide comprises at least 10-25 nucleotides in length, from which at least 7 consecutive nucleotides in length have at least 95%, preferably 98%, identity over the full sequence of any of the oligonucleotides SEQ ID NO:38 to 43.

The MSI2 inhibitor for use according to the first aspect or to any of the previous aspects, wherein the inhibitor reduces the activity of MSI2 protein, and wherein the inhibitor is a molecule such as 2,3,4,10-Tetrahydro-7,10-dimethyl-2,4-dioxobenzo[g]pteridine-8-carboxaldehyde, analogues and derivatives thereof.

In a second aspect, the present invention relates to a pharmaceutical composition comprising an inhibitor as defined in the first aspect, or a mixture of two or more of them, optionally further comprising a carrier and/or one or more pharmaceutically acceptable excipients.

The pharmaceutical composition according to the second aspect or to any of the previous aspects, wherein the pharmaceutical composition comprises an expression vector comprising the oligonucleotide sequence of at least one of the oligonucleotide molecules as defined in the first aspect or a designed protein or peptide that recognizes the same sequences that the oligonucleotide sequences defined in the first aspect bind to.

In a **third aspect,** the present invention relates to a pharmaceutical composition according to the second aspect, **for use** according to any of the previous aspects.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus five percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair, for instance, as in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage. The terms "sequence identity" or "percent identity" in the context of two or more polypeptides or proteins refers to two or more sequences or subsequences that are the same ("identical") or have a specified percentage of amino acid residues that are identical ("percent identity") when compared and aligned for maximum correspondence with a second molecule, as measured using a sequence comparison algorithm (e.g., by a BLAST alignment, or any other algorithm known to persons of skill), or alternatively, by visual inspection.

The term "hybridization" is used to refer to the structure formed by 2 or more independent strands of RNA or DNA that form a double- or triplex-stranded structure via base pairings from one strand to the other. These base pairs are considered to be G-C, A-U/T and G-U/t. (A-Adenine, C-Cytosine, G-Guanine, U-Uracil, T-Thymine). As in the case of the complementarity, the hybridization can be total or partial.

"Myotonic dystrophy" (MD) is part of a group of inherited disorders called muscular dystrophies. There are two major types of myotonic dystrophy: type 1 (MD1) and type 2 (MD2). Myotonic dystrophy type 1 (MD1) as defined herein is a multisystem disorder that affects skeletal and smooth muscle as well as other tissues such as the eye, heart, endocrine system, and central nervous system. The clinical manifestations of MD span a continuum from mild to severe and have been broadly categorized into three phenotypes: mild, classic, and congenital. Mild MD1 is characterized by cataracts and mild myotonia (sustained muscle contraction); life span is normal. Classic MD1 is characterized by muscle weakness and wasting, myotonia, cataracts, and often cardiac conduction abnormalities; adults may become physically disabled and may have a shortened life span. Congenital MD1 is characterized by hypotonia and severe generalized weakness at birth, often with respiratory insufficiency and early death; intellectual disability is common.

"Treatment of myotonic dystrophy" involves treatment of symptomatic or asymptomatic myotonic dystrophy, restoring at least partially the phenotype of a patient with said disease to a non-diseased state. "Prevention of myotonic dystrophy" involves measures taken to avoid development of myotonic dystrophy (i.e., prevents the onset of the histopathology and/or symptomatology associated myotonic dystrophy).

"RNA-binding proteins" (RBPs) are proteins that bind to the double or single stranded RNA in cells and participate in forming ribonucleoprotein complexes. "Musashi" proteins are a family of RNA binding proteins comprised of Musashi homolog 1 (MSI1) and Musashi homolog 2 (MSI2) proteins and their critical roles in multiple biological processes, include those relevant to cancer initiation and progression. "RNA-binding protein Musashi (MSI2) or an analogue thereof" refers to MSI2 protein or a structural analogue, such as a chemical analogue, that, despite of not having an identical structure as MSI2 protein, exerts similar function.

"MicroRNA" (miRNA, miR) are a class of small non-coding RNA molecules that function in RNA silencing and post-transcriptional regulation of gene expression, playing critical role in mRNA expression. miR-7 is a miRNA that plays important role in the occurrence and development of some pathologies, but it is also essential in a variety of normal tissues and it is involved in the development of multiple organs and biological functions of cells. The primary transcript of miR-7 (pri-mir-7) is cleaved into precursor pre-mir-7 and further processed to mature miR-7. The products of three different DNA sequences (termed as pri-mir-7-1, pri-mir-7-2, and pri-mir-7-3) can be processed into the same mature miR-7 sequence which comprising more or less 23 nucleotides. Human antigen R (HuR) protein mediates the binding of MSI2 to the conserved terminal loop of pri-miR-7.

"Small interfering RNAs (siRNA)", also referred to as short interfering RNA or silencing RNA, are a class of double-stranded non-coding RNA molecules, typically 20-27 base pairs in length, that operate within the RNA interference (RNAi) pathway by interfering with the expression of specific genes with complementary nucleotide sequences, causing the degradation of the target mRNA after transcription and therefore preventing its translation. By "small interfering RNAs" (siRNAs) is thus meant oligonucleotide molecules used for transient silencing of gene of interest by eliciting RNA interference response upon binding to their target transcript based on the sequence complementarity. "Asymmetric siRNAs" (asiRNAs) are a class of siRNA characterized by long guide and short passenger strands, and they represent a novel and efficient scaffold structure for RNAi duplexes that do not have symmetry in their structure but present similar mechanism of action as siRNAs.

"Antisense oligonucleotides" (ASOs) consist of single-stranded short fragments of RNA or DNA that are used to alter the function of target RNAs or DNAs to which they hybridize without necessarily producing target mRNA degradation but sterically blocking the binding of another biomolecule and/or preventing a given secondary structure formation. The goal of the antisense oligonucleotide is often the downregulation of a molecular target, usually achieved by induction of RNase H endonuclease activity that cleaves the RNA-DNA heteroduplex with a significant reduction of the target gene translation. Antisense oligonucleotides might present nucleotide modifications in order to improve their characteristics.

"Gapmers" are a class of an antisense oligonucleotide that contains a central block of deoxynucleotide monomers sufficiently long to induce RNAse H cleavage protected by flanking highly modified ribonucleotide blocks.

"2 -deoxy-2 -fluoro-Darabinonucleic acid analog" (FANA) are a fully 2' nucleotide that has both high-affinity RNA binding and retains RNAse H-compatible properties, being a class of antisense oligonucleotide highly useful for gene targeting applications.

"Morpholino oligomers" or "Phosphorodiamidate morpholino oligomers" (PMO) are short single-stranded DNA analogs that are built upon a backbone of morpholine rings connected by phosphorodiamidate linkages. PMOs bind to complementary sequences of target mRNA by Watson-Crick base pairing to block protein translation through steric blockade, that may be independently of RNase H, or modulate splicing events, among other potential mechanisms of action.

"AgomiR" or "miRNA mimetics" are a type of microRNA designed based on the mature microRNA sequence of interest which inhibits the expression of endogenous RNA by mimicking it.

"AntagomiRs", on the other hand, are generally used to silence endogenous miRNAs. Therefore, antagomiRs refer to small synthetic RNAs, chemically modified with respect to the corresponding RNA oligomer composed only of ribonucleotide units, and that are complementary to a target miRNA. Therefore, they can be considered oligonucleotide analogues that bind specifically to particular miRNAs and therefore act as miRNA inhibitors/blockers.

"BlockmiRs" are small RNAs with a special chemistry designed against the sequence that a particular miRNA detects in a particular messenger RNA (mRNA), so that, in principle, each one of them should only derepress the effect of that miRNA on that transcript, a very specific effect being expected. Therefore, they are designed so that they have a sequence that is complementary to that of a fragment of the mRNA sequence that serves as a binding site for a microRNA, in such a way that they usually bind at the 3' end of the untranslated region (UTR) of a mRNA, in other words, in the area where the endogenous microRNAs usually bind.

"Anti-miRNA oligonucleotides" (AMOS) are molecules that neutralize microRNAs (miRNAs or miRs) function to which they hybridize. "AntimiR" or "antagomiR" refer to a group of anti-miRNAs that are specific and complementary to a miRNA and interfere with its function in the cell, that can be repressive or activatory on certain mRNAs, by for instance sequestering the miRNAs in competition with their cellular target mRNA or degrading them (the miRNA), inducing miRNA repression.

"Splice-switching oligonucleotides" (SSOs) are molecules that bind to target sequences in pre-mRNA and prevent the interaction of said target with various splicing modulators. Thus, SSOs are able to modulate pre-mRNA splicing and repair defective RNA without inducing the RNase H-mediated cleavage of mRNA.

"Locked nucleic acids" (LNA), also known as 2'-O,4'-C-methylene-bridged nucleic acid (2',4'-BNA), are artificial nucleic acid derivatives that contain a methylene bridge connecting the 2'-O with the 4'-C position in the furanose rings, which enables it to form a strictly N-type conformation that offers high binding affinity against complementary RNA. Given these features, LNAs can be used for various gene silencing techniques, such as antisense, short interfering RNA, blocking of microRNA and triplex-forming oligonucleotides. Previous studies also showed that LNA could be used in SSOs and LNA-based SSOs (LNA SSOs) have been shown to be functional in vivo in mice models.

"LNA/DNA mixmers" are antisense oligonucleotides composed of alternating LNA and DNA nucleotides that are able to induce exon skipping in target mRNAs (LNA-based splice-switching oligonucleotide) or inhibit miRNAs. One of the advantages of using LNA/DNA mixmers for treatment is that they have negatively charged backbones, which may make delivery into cells more efficient than PMOs which are neutrally charged.

"Peptide nucleic acid" (PNA) are artificially synthesized polymers that mimic DNA or RNA and hybridize to either single-stranded DNA or RNA, or to double-stranded DNA, with high affinity and specificity, acting as antisense molecules and interfering with the post transcriptional regulation of the target molecule.

"MicroRNA sponges" or miRNA sponges are constructs either transiently or stably transfected or infected or delivered by gymnosis into mammalian cells containing multiple miR-binding sites for a chosen miRNA gene, interfering with their function.

A "derivative" as used herein may be a compound, molecule, oligonucleotide, protein, or analogue thereof that arises from a parent compound by replacement of a portion of said compound, without significantly altering its function.

By "substantially identical" is meant a nucleic acid or amino acid sequence that, when optimally aligned, share at least 60%, 65%, 70%, 75%, 80%, 85%, 90%, 95%, 96%, 97%, 98%, 99%, or 100% sequence identity with a second nucleic acid or amino acid sequence.

By "fragment" is meant a portion of a polypeptide or nucleic acid molecule that contains, preferably, at least 10%, 20%, 30%, 40%, 50%, 60%, 70%, 80%, 90%, 95%, or more of the entire length of the reference nucleic acid molecule or polypeptide. A fragment may contain at least 10, 20, 30, 40, 50, 60, 70, 80, 90, or at least 100, 200, 300, 400, 500, 600, 700, 800, 900, 1000, 1200, 1500, 1750, 1800 or more nucleotides or at least 10, 20, 30, 40, 50, 60, 70, 80, 90, 100, 150, 200, 250, 300, 350, 400, 450, 500, 550, 600, 640 amino acids or more.

An "effective dose" or "therapeutically effective dose" is a sufficient amount to achieve a beneficial or desired clinical outcome.

The expressions "pharmaceutically acceptable" or "pharmacologically acceptable" refer to molecular entities and compositions that do not produce any adverse, allergic or other reactions when administered to an animal or human being. As used herein, "pharmaceutically acceptable vehicle" includes solvents, cells, buffers, solutions, dispersion media, coatings, antibacterial and antifungal agents, isotonic and absorption retarding agents and similar acceptable for use in formulation pharmaceuticals, such as pharmaceutical products suitable for administration to human beings. The use of such media and agents for pharmaceutical-active substances is well known in the art. Except where any conventional medium or agent is incompatible with the active ingredients of this invention, their use in the pharmaceutical compositions of the invention is contemplated. Additional active ingredients may also be incorporated into the compositions, provided that they do not inactivate the molecules of this invention or their expression vectors. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the active ingredients of the present disclosure, its use in therapeutic compositions is contemplated. Supplementary active ingredients also can be incorporated into the compositions, provided they do not inactivate the inhibitors of the compositions.

"Expression vector" is a nucleic acid molecule, usually a plasmid, a virus, DNA molecule, minichromosomes, or cell, designed to endogenously express a specific biomolecule, natural or artificially designed, such as RNAs or proteins, including the CRISPR-associated systems such as Cas9, Cas12a, and Cas13 and their corresponding guide RNAs, in a target cell. The expression vector is introduced into a host cell using well-known techniques such as infection or transfection, including calcium phosphate transfection, liposome-mediated transfection, electroporation and sonoporation. Expression constructs and methods for their generation and use to express a desired protein are known in the art.

Other features and advantages of the invention will be apparent from the following description, the drawings, and the claims.

### DESCRIPTION

MSI2 and HuR proteins form a complex with pri-mir-7 and regulate the tissue-specific control of miR-7 biogenesis. Further, MSI2 is a transcriptional regulator that targets genes involved in development and cell cycle regulation, having an important role in the development of the nervous system, regulation of hematopoietic stem cells, cell division, cell cycle regulation and it has even been associated with poor prognosis in certain types of cancer. Thus, the function of MSI2 varies depending on the tissue where it is located. The authors of the present invention have surprisingly found that the function of MSI2 does not only depend on where and when the protein is located (which type of cells, tissue, and in which stage of development) but also depends on the concentration at which it is present in said cell. That is, the levels of MSI2 protein found in a cell also influence its regulation function and this is pivotal when it comes to the use of MSI2 as a therapeutic target to treat myotonic dystrophy (MD). It is shown in the present invention that MD can be treated by reducing the activity or levels of MSI2 protein or transcript in a myotonic dystrophy cell when said cells have reached a certain threshold of MSI2 protein concentration.

On the one hand, the present invention relates to inhibitors of MSI2 function by reducing MSI2 protein activity. Such inhibitors may be small molecules that are able to bind to or interact with MSI2 protein and avoid or compete for its RNA binding function, decreasing its activity in the cell. For instance, as shown in Example 5, a small molecule that binds directly to MSI2 protein was tested. This molecule, named Ro-08-2750 (2,3,4,10-Tetrahydro-7,10-dimethyl-2,4-dioxobenzo[g]pteridine-8-carboxaldehyde), is a compound that competes for RNA binding function of MSI2, causing a reduction in MSI2 activity and an increase in miR-7 (Figure 6). Thus, the present invention encompasses inhibitors that exert their function at a protein level, and even if they do not decrease the levels of MSI2 protein or transcript, they are able to reduce MSI2 activity, causing an increase in miR-7 levels.

On the other hand, the present invention also relates to inhibitors of MSI2 function by reducing the MSI2 intracellular transcript levels or MSI2 intracellular protein levels. Such inhibitors may be oligonucleotides, oligoribonucleotides, or other compounds that reduce the MSI2 intracellular transcript or protein levels. For instance, as shown in Example 4, antisense oligonucleotides ASO1 and ASO3 directed against MSI2 transcript significantly lowered the level of MSI2 transcripts in MD1-derived myotubes at two different concentrations, causing a strong rescue in two atrophy-related myotube parameters: fusion index and myotube diameter (see Figure 3). Other approaches to inhibit intracellular levels of MSI2 transcripts or protein were tested, such as small interfering RNA or miRNA mimetics: Figure 5 shows the co-transfection of two siRNAs designed to target MSI2 transcript, causing an increase of miR-7 levels, and eventually rescuing the fusion index in patient cells. In Example 5, miR-107 analogues (miR-107 is a microRNA that negatively regulates MSI2 transcript by directly binding to its 3' UTR region) were designed, and their use caused a downregulation of MSI2 that correlated with significant derepression of miR-7 (Figure 7).

All of the above indicates that MSI2 levels can be related to miR-7 levels and therefore the regulation of MSI2 has a direct impact in the phenotype of MD1 cells. Further, it has also been found that the relationship between MSI2 and MD1 phenotype is also influenced by the levels of MSI2 protein present in the cell. Example 6 shows that miR-7 and autophagy-related proteins were quantified in different tissues in the mouse model HSA^{LR}, but no differences were observed between the mouse model and control mice (FVB), see Figure 9. The authors of the present invention found that the reason why this mouse model did not present MD1 phenotype is because its cells did not have enough levels of overexpression of MSI2 protein. Therefore, when murine Msi2 protein was overexpressed in mice's tissues by administrating adeno-associated virus (AAV), it was observed that only in the group with the highest Msi2 levels (group administrated with the highest dose of AAV (1.75x10¹²)), miR-7 levels were significantly reduced, and the mice also showed decreased forelimb grip strength in comparison with control mice or mice administrated with a lower dose of AAV overexpressing murine Msi2 (Figures 8 and 9). These results demonstrate that when certain level of overexpression of MSI2 protein is achieved, the MD1 phenotype appears. In particular, as shown in Table 3 in the examples, the level of overexpression of MSI2 transcript and protein in MD1 patients are higher in at least 1.358 and 1.59-fold change, respectively, than those found in healthy human biopsies. Thus, if it is desired that a patient suffering from MD reaches the physiological levels of MSI2 present in healthy persons, the levels of MSI2 transcript and protein should be preferably reduced in about 35% and 60%, respectively.

Further, as also shown in Table 3, it was also found that MD1 patients have reduced levels of miR-7 in comparison to healthy controls. In particular, the miR-7 intracellular values of MD patients' biopsies were 0.58-fold reduced compared to healthy controls. Thus, for a MD patient to reach physiological or normal levels of miR-7, these levels should be preferably increased in about 70%.

Importantly, it was also found that, as shown in Table 5 in the examples, an increase of at least 1.68-fold (68%) in the intracellular miR-7 levels in treated cells compared to MD1 reference cells, particularly compared to immortalized skin fibroblasts expressing conditional MyoD, is enough to observe phenotypical changes in the cells such as fusion index and cell's diameters, indicating a partially rescued phenotype. These observations support the notion that the levels of miR-7 in a MD cell need to be increased in at least 1.68 times in order to treat MD in a mammal.

As can be noted, the adjustment between MSI2 levels and miR-7 levels can be compared to those of a healthy individual (Table 3), or to those in a reference cell (Table 5). For sake of simplicity, we herein define the changes in a MD1 cell treated with the inhibitor in terms of changes in miR-7 levels of a reference cell, particularly of immortalized skin fibroblasts expressing conditional MyoD. The reference cell will be defined below.

In view of the above, a **first aspect** of the present invention relates to an inhibitor of RNA-binding protein Musashi (MSI2) function or an analogue thereof for use in the treatment of myotonic dystrophy in a mammal, wherein the inhibitor is capable of reducing in a myotonic dystrophy (MD) cell or in a cell with the phenotype of a MD cell, at least one of
i) MSI2 protein activity,
ii) intracellular transcript levels of **MSI2,** and/or
iii) intracellular protein levels of **MSI2,**
thereby causing in the same MD cell an increase in the intracellular miR-7 levels of at least 1.5-fold of the intracellular miR-7 levels of a reference cell, as measured by a gene expression quantification technique such as quantitative reverse transcription PCR (RT-qPCR), Northern blotting, or RNA-sequencing (RNA-Seq) techniques.

By "inhibitor" is meant any substance or group of substances that reduce or suppress the activity and/or the amount of another substance. Particularly, in the present invention, the substance whose amount or activity is reduced or suppressed by the effect of the inhibitor is the MSI2 protein or transcript. At the time of defining the inhibitor of the first aspect, its function is defined in terms that it causes an increase in the intracellular levels of miR-7, since it has been shown in the present invention that when MSI2 function was reduced, the intracellular levels of miR-7 were increased. However, it is to be understood that the definition of the inhibitor of the present invention is not limited to only an effect over the levels of miR-7, but it also includes the inhibitors of MSI2 function that exert their effect by other mechanism of action not directly related to miR-7, for example on TGFBR1 levels, as shown in Table 3.

Further, as referred herein, an "inhibitor" is not necessarily limited to only one compound, but it can be formed by the interaction of one or more than one compound (referred from now on as a "system of one or more compounds"). The term "inhibitor" may thus also include those technical approaches requiring the combination of two or more elements for its repressive activity such as, for example, a Cas family protein requiring the presence of a given guide RNA to recognize a specific DNA or RNA complementary sequence to exert their inhibitory function. That is, the inhibitor according to the first aspect of the present invention may consists of only one molecule type, such as an oligonucleotide, or it may be a complex comprising several components, such as a CRISPR/Cas and gRNA system. In general terms, inhibitors as referred herein can be divided in A) Oligonucleotides for RNA therapies, B) Proteins and small molecules, and C) Compounds or systems of one or more compounds for gene therapies. Each of these groups are explained in detail below.

"A myotonic dystrophy (MD) cell" is a cell suffering from MD or a cell with the phenotype of a MD cell. This cell might come from a subject suffering from MD or from a cell that has been genetically engineered so that MD is artificially induced. "A myotonic dystrophy cell or a cell with the phenotype of a MD cell" may also be defined as a cell having more than 50 repetitions of the CTG triplet in its genome, which are expressed as an isolated non-coding RNA or in the context of a coding messenger. The MD cell treated with the inhibitor may be a cell from any organ or tissue of a mammal. In a preferred embodiment, the MD cell or a cell with the phenotype of a MD cell is selected from the group of muscular cells, nervous cell, or cardiac cells. Preferably, the cell with the phenotype of a MD cell is selected from the group consisting of muscle satellite cells, myoblast, myocyte, or myotubes. More preferably, the cell with the phenotype of a MD cell is a myocyte or one of its precursor cell types. The MD cell may be a cell obtained from human muscle biopsies from a person suffering from MD disease. The MD cell may be one or more cells from a primary culture or an established cell line derived from muscle tissue (including induced pluripotent stem cells, known as iPSCs) or stem cells of said tissue. The MD cell may also be a transdifferentiated or genetically engineered cell from a human being or an animal which has been modified to be phenotypically or genotypically similar to a different cell, such as skin fibroblasts transdifferentiated into myoblasts (TDM cells).

It is noted that the function inhibited by the inhibitor of the present invention may be the natural function of MSI2, or the function derived due to its overexpression in the MD cells.

By "reference cell" is meant a cell affected by MD and that has not been treated with the inhibitor of the present invention. The reference cell can be any cell in any organ or tissue in a mammal suffering from MD. A reference cell is a cell whose levels of MSI2 activity, and/or MSI2 intracellular levels of protein and transcripts are taken as reference in the present invention because they are affected by MD disease. As also explained below, the reference cell can serve as reference cell for the study of the increase of intracellular levels of miR-7 caused by the inhibitor according to the present invention.

In a particular embodiment, the reference cell is a standardized DM myoblast cell line. In a preferred embodiment, the reference cell is immortalized skin fibroblast cell line expressing conditional MyoD, as described by Arandel et al. 2017 [Arandel et al. (2017). Dis Model Mech. Apr 1;10(4):487-497. doi: 10.1242/dmm.027367. Epub 2017 Feb 10]. Immortalized skin fibroblasts expressing conditional MyoD, are a renewable and reliable source of converted human muscle cells that allow the assessment of therapeutic strategies against Muscular dystrophies. To obtain the reference cells, human primary fibroblasts isolated from skin biopsies obtained from a DM1 patient were used to establish immortalized myo-converted or transdifferentiated cell lines (also referred to as transdifferentiated myotubes) following the methodology described in Chaouch et al. [Chaouch, S., et al. (2009). Immortalized Skin Fibroblasts Expressing Conditional MyoD as a Renewable and Reliable Source of Converted Human Muscle Cells to Assess Therapeutic Strategies for Muscular Dystrophies: Validation of an Exon-Skipping Approach to Restore Dystrophin in Duchenne Muscular Dystrophy Cells. Human Gene Therapy, 20(7), 784-790. doi:10.1089/hum.2008.163]. Briefly, primary fibroblasts isolated from a skin biopsy are immortalized by the sole re-expression of TERT before a subsequent transduction with lentiviral vectors containing an inducible Tet-on MYOD1 construct. Under non-permissive conditions, immortalized cells can proliferate indefinitely, but when supplemented with doxycycline, cells express MYOD1 that activates the myogenic program and their fusion into myotubes. The forced expression of MYOD1 following doxycycline supplementation leads to the formation of large multinucleated DM1 transdifferentiated myotubes comparable to healthy-derived transdifferentiated myotubes. FISH and immunofluorescence experiments revealed that DM1 myotubes contain nuclear CUGexp-RNA aggregates that colocalized with MBNL1 splicing factor. DM1 transdifferentiated myotubes displayed splicing changes of many transcripts including ATP2A1, BIN1, INSR, LDB3, MBNL1 and TNNT2 that are also misregulated in skeletal muscles of DM1 patients. Immortalized DM1 cell lines, thereafter referred to as reference cells, exhibit the characteristic features of DM1 pathology, making them excellent cell lines to asses dystrophin rescue and molecular analyses to assess the effectiveness of therapeutic approaches to cure and/or treat MD.

By "healthy cell" is meant a cell not affected by MD, i.e having less than 37 CTG repeats in the DMPK locus. Healthy cells can be cells from any organ or tissue having physiological, i.e., normal, intracellular levels of MSI2. In an embodiment, the healthy cell is a muscular, cardiac, or nervous cell.

"MSI2 activity" is meant the activity or function of the MSI2 protein, which may be the regulation of target gene transcripts involved in cellular pathways, such as cell development and cell cycle regulation, including but not limited to those directed to regulate the biogenesis of miR-7.

By "MSI2 transcript" is meant the single-stranded ribonucleic acid product synthesized by transcription of MSI2 gene. The term MSI2 transcript includes all kind of transcripts produced after transcription, including mature and immature transcripts (such as pre-mRNAs or mature mRNAs). By "transcript levels" is meant the number, amount or quantity of total transcript molecules measured by a gene expression quantification technique. By "intracellular transcript levels" is meant the number, amount or quantity of total transcripts molecules located inside one or more cells.

By "protein levels" is meant the number, amount or quantity of the proteins measured by a protein quantification technique. "Intracellular protein levels" refers to the number, amount or quantity of the proteins located inside one or more cells.

By "reducing in a myotonic dystrophy cell at least one of i) MSI2 protein activity, ii) intracellular transcript levels of MSI2, or iii) intracellular protein levels of MSI2" is meant that the protein activity of MS2, its intracellular transcript levels or protein levels are decreased, i.e., lowered.

In the context of the first aspect of the present invention, "causing in the same MD cell an increase in the intracellular miR-7 levels" is meant that the intracellular levels of miR-7 of the MD cell or the cell with the phenotype of a MD cell treated with the inhibitor are increased in comparison to those present in a reference cell, wherein the reference cell is preferably a skin fibroblast cell line expressing conditional MyoD. As explained above, Table 5 in the examples shows that the cells treated with Ro 08-2750, ASO3, ASO1 and siRNA inhibitors reverted to normal phenotype when the miR-7 levels were increased up to 2.52, 1.68, 1.49 or 3.3-fold, respectively. This demonstrate that it is plausible to revert MD1 phenotype with the treatment of the inhibitor according to the present invention.

In an embodiment, the intracellular increase in miR-7 in the MD cell or the cell with the phenotype of a MD cell after treatment with the inhibitor may be of at least 1.2, 1.25, 1.3, 1.35, 1.4, 1.5, 1.6, 1.65, 1.7, 1.75, 1.8, 1.85, 1.9, or 1.95-fold increase of the intracellular miR-7 levels of the reference cell, wherein the reference cell is preferably a skin fibroblast cell line expressing conditional MyoD, as measured by a gene expression quantification technique. In a preferred embodiment, the increase is of at least 1.6-fold, more preferably at least 1.7-fold.

In a preferred embodiment, the increase in the intracellular miR-7 level is of at least 1.7-fold of the intracellular miR-7 levels of a reference cell and the MD cell or a cell with the phenotype of a MD cell is a myocyte or one of its precursor cell types.

By a "gene expression quantification technique" is meant any analytical method used to detect and quantify RNA expression levels or differential RNA expression within one or more cells. Northern blotting is a technique used to detect specific RNA molecules present within an RNA mixture, and it is employed in the analysis of an RNA sample from a cell type or tissue so as to determine the RNA expression of certain genes. Quantitative reverse transcription polymerase chain reaction (RT-qPCR) is one of the most sensitive techniques available for detecting and quantifying RNA. Using RT-PCR, extremely small sample sizes can be used in the quantification of RNA and the technique can in fact do this using just a single cell and in real time. RNA-sequencing (RNA-Seq) refers to methods used to measure the amount of RNA molecules. Examples include shotgun sequencing of cDNA molecules acquired from RNA through reverse transcription and technologies used to sequence RNA molecules from a biological sample so that the primary sequence and abundance of each RNA molecule can be determined.

The intracellular levels of miR-7 as measured by a gene expression quantification technique may be expressed or measured by an "absolute" quantification or by a "relative" or comparative quantification.

The inhibitor exerts its effects by causing a reduction in the MSI2 activity, by reducing intracellular transcript levels of MSI2, or by reducing intracellular protein levels of MSI2, as defined above. Preferably, the MSI2 inhibitor reduces the intracellular transcript levels of MSI2.

In an embodiment, the inhibitors according to the first aspect of the present invention are selected from the group comprising:
A) Oligonucleotides for RNA therapies
B) Proteins and small molecules
C) Compounds or systems for gene therapies

### A) Oligonucleotides for RNA therapies

Some examples of possible inhibitors may be oligonucleotide molecules, oligoribonucleotide molecules, hybrid DNA-RNA molecules, proteins and small molecules, and analogues or derivative thereof. Further, they may be formed of naturally occurring nucleic acids or synthetic or modified nucleic acids. In a preferred embodiment, the inhibitors may be oligonucleotides molecules. The MSI2 inhibitor may be an oligoribonucleotide molecule or an analogue thereof. The oligoribonucleotides (RNA) that are composed of a phosphate group, the nitrogenous bases adenine (A), cytosine (C), guanine (G) or uracil (U), and pentose known as ribose. Based on their widespread use, molecules whose units include nucleotide inosine are also considered within that definition.

The oligonucleotide molecules according to the first aspect of the present invention may be oligonucleotides or oligoribonucleotides molecules derived therefrom that include, among others, some of the usual chemical modifications that modify the oligonucleotide molecules to make them more resistant to degradation or bioavailable. Thus, as used in this invention, the term "oligonucleotide molecules" includes both oligonucleotides as such, as well as the "oligonucleotide analogues".

"Oligoribonucleotide analogues" are the molecules derived therefrom that incorporate some chemical modification in at least one of the ribonucleotide units that form them, either in the phosphate group, the pentose or one of the nitrogenous bases, the modifications consisting in the addition of non-nucleotide groups at the 5' and/or 3' ends are also included. A skilled person would know which type of chemical modifications are suitable for the purposes of the present invention. As reference, Figure 2 in Crooke et al. 2018 [Crooke, S. T., Witztum, J. L., Bennett, C. F., & Baker, B. F. (2018). RNA-Targeted Therapeutics. Cell Metabolism, 27(4), 714-739. doi:10.1016/j.cmet.2018.03.004], shows the different modification that can be performed in RNA and DNA analogues. Such modifications include 2'-Fluoro, 2'-O-methyl, 5-methylcytosine, 2'-MOE (2'-O-methoxiethyl), cEt ((S)-constrained ethyl), LNA (locked nucleic acid), PMO (Phosphorodiamidate morpholino), thiophosphoramidate, PS (phosphorothioate), among others. The oligoribonucleotide analogues may also be conjugated with other molecules, such as antibodies, lipids, lipid nanoparticles or GalNac (N-acetyl galactosamine) clusters, among others. By extension, for the purposes of this invention and as used herein, the terms "oligoribonucleotide molecule" and "oligoribonucleotide analogue" or "oligonucleotide analogue molecule" also include miRNA sponges, as it can be considered that the main constituent of the same are tandem repeats of oligonucleotides, characterized in that each of these oligonucleotides are in themselves or contain a binding site of a micro-RNA of interest.

Other chemical modifications in the oligonucleotides or oligoribonucleotides of the present invention are possible and known, which are also comprised within the possible modifications that give rise to oligonucleotide analogues. As can be deduced from the definition of "oligonucleotide molecules" and that of "oligonucleotide analogues", also included within these definitions are hybrid molecules, in which some units present modifications and others do not, as well as hybrids between analogues of nucleic acids and peptides or, even, hybrid molecules in which some of the nucleotide units are ribonucleotides (or analogues thereof) and others are deoxyribonucleotides (nucleotides in which the sugar is deoxyribose), as well as analogues of the latter, i.e. RNA-DNA hybrids and analogues thereof.

In a preferred embodiment, the MSI2 inhibitor for use in the treatment of myotonic dystrophy is an oligonucleotide molecule which comprises a fragment composed of one or more nucleotide unit sequence or a succession of nucleotide units. In an embodiment, the oligonucleotide molecule has an effect in the RNA interference pathway. By "RNA interference pathway" or "RNAi" is meant the biological process in which RNA molecules inhibit or alter gene expression or translation, by neutralizing targeted mRNA molecules. Particularly, the three types of oligonucleotide molecules that are central in the RNAi pathway are microRNAs (miRNA), small interfering RNAs (siRNA) and piwiRNAs.

In an embodiment, the oligonucleotide molecules or oligonucleotide analogues according to the first aspect of the present invention is selected from the group consisting of small interfering RNAs, antisense oligonucleotides, gapmers, morpholino oligomers, FANA oligonucleotides, agomiRs, miRNA mimics, antagomiRs, blockmiRs, PNAs, LNAs (locked nucleic acid antisense oligonucleotides), splice-switching oligonucleotides (SSOs), LNA-based splice-switching oligonucleotides (LNA SSOs), LNA/DNA mixmers or miRNA sponges. Also comprised within the concept of oligonucleotide molecules or oligonucleotide analogues useful for the purpose of the present invention and comprised within its scope are those mRNA inhibitors, blockers or antagonists that act on pre-mRNAs, usually altering mRNAs biogenesis or maturation and having a negative effect on mRNAs activity, mainly due to a decrease of the active available mRNA.

The design of oligonucleotides molecules as inhibitors/antagonists is usually based on a short basic sequence of nucleotides, which may be the sequence complementary to the RNA to be inhibited or an RNA-binding protein recognized. As used in this specification, it is understood that two chains of nucleotide molecules are 100% complementary (or, as is expressed in a more abbreviated way herein, that their sequences are complementary) when the nucleotide or nucleotide analogue sequence of one of them, read in the 5'-3' sense, is the sequence of nucleotides or nucleotide analogues that present the nitrogenous bases which pair with the nitrogenous bases of nucleotides or nucleotide analogues of the other sequence, read in the 3'-5' sense. That is to say, the sequence 5'-UAGC-3' would be complementary to the sequences 3'-AUCG-5' (in the case of being the ribonucleotide or ribonucleotide analogue units) and 3'-ATCG-5' (in the case of being the deoxyribonucleotide or deoxyribonucleotide analogue units), which would be, respectively, sequences 5'-GCUA-3' and 5'-GCTA-3' read in the 5'-3' sense.

To design the antagonist or inhibitor according to the first aspect, it is important to note that there should be sufficient complementarity with the endogenous molecules to which they must bind so that the desired effect of inhibition/antagonism/silencing is actually produced. In this sense, examples can be taken into account where the "typical" complementarity between a miRNA and its target may be 50% (see, for example, the reference http://mirtarbase.mbc.nctu.edu.tw/php/detail.php'?mirtid=MIRT000125#target), therefore it is advisable that the oligonucleotide molecule of the invention comprises a fragment of nucleotide or of nucleotide analogue unit sequence, in which the sequence of the nitrogenous bases of the nucleotide or nucleotide analogue units is at least 50% (or at least 55%, 60%, 70%, 80%, 85%, 90%, 91%, 92%, 93%, 94%, 95%, 96%, 97%, 98%, 99% or 99.5°/o, or 100%) identical to the sequence complementary to that of the fragment of the endogenous molecule with which it should pair, that is to say, to the sequence of endogenous RNA transcript to which it should bind. Overall, this is the criterion that has been followed for the design of the oligonucleotides of the present invention against human MSI2 transcript (SEQ ID: 1 or 2).

In the case of some oligonucleotides molecules, for instance agomiRs, the most important thing is that they comprise a fragment in which the sequence of nitrogenous bases of nucleotides or nucleotide analogues is complementary (preferably, 85%, 90%, 95% or 100% complementary) to the sequence of nitrogen bases of the nucleotides of the seed region of the molecule, for instance the mRNA of MSI2 protein, which is their target, so the complementarity in the rest of 25 nucleotides or nucleotide analogues that are present in the nucleotide molecule, if any, is less important. This is because some oligonucleotides are usually complementary to only a part, the so-called seed region, of their target mRNA, which spans around 7-10 nucleotides, but they bind to it with great affinity. In this case, the whole complementary between both molecules may not be more than 50%, but it should be considered that the seed region, comprised within the sequence of the oligonucleotides, comprises a sequence with high complementary of at least 80%, 90% or even 100% to the target mRNA where they hybridize.

In an embodiment, MSI2 transcription levels may be reduced according to the present invention by using agomiRs oligonucleotides. As defined above, agomiRs are micro-RNA that mimic endogenous RNAs, for instance microRNAs. An agomiR according to the present invention may be a molecule that mimics the endogenous micro-RNA-107, which negatively regulates MSI2 by directly binding to its 3' UTR. In a further embodiment, the inhibitor may be an oligonucleotide sequence that, alone or in combination with a protein activity, edits or modifies MSI2 transcripts, causing a decrease in their stability and therefore reducing the levels of intracellular MSI2 transcripts and/or intracellular MSI2 protein.

The oligonucleotide molecule which comprises a fragment composed of a succession of nucleotide units may comprise at least 5, 6, 7, 8, 9, 10 consecutive nucleotides in length that have at least 70%, 75%, 80%, 85%, 90%, 95%, 96%, 97%, 98%, 99% or 100% identity with the sequence target where they bind or to the complementary sequence thereof. In an embodiment, the target sequence where they bind is SED ID:1 or, preferably, SEQ ID NO: 2, or a complementary thereof. Any combination of consecutive nucleotides in length with the percentage of identity with the sequence target where they bind or to the complementary sequence thereof is included in the present invention. For instance, in an embodiment, the oligonucleotide molecule of the present invention may comprise at least 5 consecutive nucleotides in length that have at least 80% identity over the full length sequence of SEQ ID: 1 or, preferably SEQ ID NO: 2, or to the complementary sequence thereof. In a preferred embodiment, the oligonucleotide comprises at least 7 consecutive nucleotides in length that have at least 90% identity over the full length sequence of SEQ ID NO:1 or, preferably SEQ ID NO: 2, or to the complementary sequence thereof. In the context of the present invention, it is to be understood as a preferred embodiment that the inhibitor oligonucleotides molecules according to the present invention have at least 70%, 75%, 80%, 85%, 90%, 95%, 96%, 97%, 98%, 99% or 100% identity over the complementary sequence of the full sequence of SEQ ID NO:1 or, preferably SEQ ID NO: 2.

The length of oligonucleotide can be adjusted and may vary according to various criteria known by the skilled person in the art. The oligonucleotide molecule according to the present invention which comprises a fragment composed of a succession of nucleotide units may be at least 5-25, 10-25, 15-25, 20-50, or 25-50 etc. nucleotides in length. In an embodiment, the oligonucleotide molecule is at least 5, 6, 7, 8, 9, 10, 11, 12, 13, 14, 15, 16, 17, 18, 19, 20, 21, 22, 23 ,24, 25, 26, 27, 28, 29, 30 nucleotides in length. In another embodiment, the oligonucleotide molecule which comprises a fragment composed of a succession of nucleotide units may be more than 30 nucleotides in length. In another embodiment according to the first aspect, the oligonucleotide molecule is at least 5, 6, 7, 8, 9, 10, 11, 12, 13, 14, 15, 16, 17, 18, 19, 20, 21, 22, 23 ,24, 25, 26, 27, 28, 29, 30 nucleotides in length, of which at least 5, 6, 7, 8, 9, or 10 nucleotides are consecutive and have at least 70%, 75%, 80%, 85%, 90%, 95%, 96%, 97%, 98%, 99% or 100% identity with the sequence target where they bind or to the complementary sequence thereof. The sequence identity of the consecutive nucleotides comprised in the oligonucleotide molecule of the present invention may be at least 70%, 75%, 80%, 85%, 90%, 95%, 96%, 97%, 98%, 99% or 100% identical over the full-length sequence of the target sequence where they bind, which may be SEQ ID NO:1 or, preferably, SEQ ID NO: 2, or the complementary sequence thereof. Any combination between nucleotide length, consecutive nucleotides length and percentage of identity is included in the present invention. For instance, in a preferred embodiment, the oligonucleotide molecule comprises at least 10-25 nucleotides in length, from which at least 5 consecutive nucleotides in length have at least 80% identity over the full-length sequence of SEQ ID:1 or, preferably, SEQ ID NO: 2, or to the complementary sequence thereof. In a further preferred embodiment, the oligonucleotide molecule comprises at least 10-25 nucleotides in length, from which at least 7 consecutive nucleotides in length have at least 90% identity over the full-length sequence of SEQ ID: 1 or, preferably SEQ ID: 2, or the complementary sequences thereof.

In an embodiment, the nucleotide molecule according to the first aspect of the present invention has at least 65%, 70%, 75%, 80%, 85%, 90%, 95%, 96%, 97%, 98%, 99%, or 100% identify over the full sequence of any of the nucleotides of SEQ ID NO: 3 to SEQ ID NO: 37. In another embodiment according to the first aspect, the oligonucleotide molecule is at least 5, 6, 7, 8, 9, 10, 11, 12, 13, 14, 15, 16, 17, 18, 19, 20, 21, 22, 23 ,24, 25, 26, 27, 28, 29, 30 nucleotides in length, of which at least 5, 6, 7, 8, 9, or 10 nucleotides are consecutive and have at least 70%, 75%, 80%, 85%, 90%, 95%, 96%, 97%, 98%, 99% or 100% identity with the SEQ ID: 3 to 37. In a preferred embodiment, the oligonucleotide molecule has at least 90% identity over the full sequence of any of the oligonucleotides SEQ ID NO:3 to SEQ ID NO:37, or the complementary sequences thereof. In the context of the present invention, it is to be understood as a preferred embodiment that the inhibitor oligonucleotides molecules according to the present invention have at least 70%, 75%, 80%, 85%, 90%, 95%, 96%, 97%, 98%, 99% or 100% identity over the complementary sequence of the full sequence of SEQ ID NO:3 to SEQ ID NO: 37.

It is further preferred that the sequence of the nitrogenous bases of the nucleotide units of the fragment comprised in the oligonucleotide molecule has at least 80%, 85%, 90%, 91 %, 92%, 93%, 94%, 95%, 96%, 97%, 98%, 99%, 99.5%, 100% identity over the full sequence of the nitrogenous bases of the oligonucleotide of the AS01 (SEQ ID NO:38) or of the oligonucleotide sequence of ASO3 (SEQ ID NO:39), or of the siRNAs (SEQ ID NOs: 40 and 43), or the miR-107 analogue (agomiR-107) (SEQs ID NO:41 and 42). Preferably, the oligonucleotide sequence has at least 80% identity over the full sequence of any the oligonucleotides of SEQ ID NO: 38 to 43, preferably SEQ ID: 38 and 39. Also, preferably, the oligonucleotide sequence has at least 90% identity over the full sequence of any the oligonucleotides of SEQ ID NO:38 to 43, preferably SEQ ID: 38 and 39. Also preferably, the oligonucleotide sequence has at least 95%, 96%, 97%, 98%, 99%, or 100% identity over the full sequence of any the oligonucleotides of SEQ ID NO: 38 to 43, preferably SEQ ID: 38 and 39, or the complementary sequences thereof.

In another embodiment according to the first aspect, the oligonucleotide molecule is at least 5, 6, 7, 8, 9, 10, 11, 12, 13, 14, 15, 16, 17, 18, 19, 20, 21, 22, 23 ,24, 25, 26, 27, 28, 29, 30 nucleotides in length, of which at least 5, 6, 7, 8, 9, or 10 nucleotides are consecutive and have at least 70%, 75%, 80%, 85%, 90%, 95%, 96%, 97%, 98%, 99% or 100% identity over the full sequence of any of nucleotides of SEQ ID: 38 to 43.

Public and private tools can be used to sequence alignment for comparison between two nucleotide sequences. Some of them are the BLAST family programs, which are of public access (for example, through the webpage of the U.S. National Center for Biotechnological Information: http://blast.ncbi.nlm.nih.gov/Blast.cgi) and can be used to carry out searches and calculations of identity, especially for the purposes of the invention, BLAST TWO SEQ, dedicated to nucleotides.

Importantly, in all the SEQ IDs and sequences disclosed herein, "T" nucleotide is to be understood as interchangeable for "U" nucleotide if the oligonucleotides according any aspect of the present invention are produced as RNA instead of DNA, and the other way around ("U" can be "T" if an RNA oligonucleotide is produced as DNA). Similar applies to the complementary sequences thereof.

In a further embodiment, the inhibitor according to the first aspect of the present invention may be a molecule that acts as a decoy and hijacks MSI2 protein or transcripts interfering with their normal function. In an embodiment, the inhibitor hijacks MSI2 protein or transcript in the cytoplasm of the cell, reducing the normal activity of MSI2 protein within this cell. For instance, the inhibitor may be an RNA sequence with the consensus binding sites for the MSI2 protein so that the protein is sequestered by the decoy and depleted for its normal functions.

### B) Proteins and small molecules

In another preferred embodiment, the MSI2 inhibitor for use in the treatment of myotonic dystrophy according to the first aspect of the present invention reduces the activity of MSI2 protein. Proteins and small molecule inhibitors or modulators of MSI2 able to interact with MSI2 protein and inhibit its function are also included in the present invention. In a preferred embodiment, the inhibitor is a small molecule

It is known that there are key residues in MSI2 protein that are involved in its activity and therefore any inhibitor able to interact with them will potentially be an inhibitor of the activity of MSI2 protein. These residues were described in Minuesa et al. [Minuesa, G., Albanese, S.K., Xie, W. et al. Small-molecule targeting of MUSASHI RNA-binding activity in acute myeloid leukemia. Nat Commun 10, 2691 (2019). https://doi.org/10.1038/s41467-019-10523-3] and are K22, F66, F97 and R100. In an embodiment of the first aspect, the inhibitor is a protein or a small molecule that is able to interact with the residues K22, F66, F97 and R100 thereby reducing the activity of MSI2 protein.

In a further preferred embodiment, the MSI2 inhibitor that reduces the activity of the MSI2 protein is a small molecule such as 2,3,4,10-Tetrahydro-7,10-dimethyl-2,4-dioxobenzo[g]pteridine-8-carboxaldehyde (also called Ro-08-2750), analogues and derivatives thereof. Ro-08-2750 is an inhibitor of NGF that also binds directly and selectively to MSI2 and competes for its RNA binding in biochemical assays.

In another embodiment, the inhibitor according to the first aspect of the present invention may be directed to modify or target the DNA sequence of MSI2 gene, thereby reducing the expression of MSI2 gene and thus reducing the levels of MSI2 transcript and/or proteins. In a further embodiment, the inhibitor may modify the methylation status of the MSI2 gene, thereby reducing the expression of the MSI2 gene and thus reducing the levels of MSI2 transcript and proteins. In a further embodiment, the inhibitor may be an oligonucleotide sequence or a small molecule that edits or modifies MSI2 transcripts, alone or in combination with a protein activity, causing a decrease in their stability and therefore reducing the levels of intracellular MSI2 transcripts and/or intracellular MSI2 protein. Alternatively, in a further embodiment, inhibitors according to the present invention include molecules or proteins that target DNA regulatory elements, such as promoters or enhancers, wherein this targeting by the inhibitors induce a reduction of the expression of MSI2 gene.

### C) Compounds or systems of compounds for gene therapies

The inhibitor according to the first aspect may be a system comprising one or more compounds. Thus, also included in the present invention are compounds, systems, or complexes of proteins and/or oligonucleotides that are commonly used for gene therapies and that have an effect as inhibitors according to this aspect of the present invention.

In an embodiment, and as defined above, the inhibitor might consist of only one type of compound (i.e., oligonucleotides) or it may be comprised of a system of one or more than one compounds that work together and to modulate the expression of MSI2 gene, transcripts, protein or its activity. For instance, the inhibitor of the present invention may be accompanied or fused or administered together with a CRISPR/cas system. In an embodiment, the CRISPR/cas system comprises at least a gRNA, a Cas protein or the nucleotide sequence comprising the Cas protein, and, optionally, a donor DNA. In this case, the CRISPR/Cas system will provide the tools to modify the gene sequence of MSI2, or its regulatory sequences, such as its promoter, in order to eventually lead to a reduction in MSI2 protein or MSI2 transcripts levels, or a reduction in MSI2 activity.

In another embodiment, the CRISPR/Cas9 genetic system is designed to be used in therapeutic approaches consisting of targeting the removal of gene parts at the MSI2 DNA level, preventing transcription of the gene (e.g. by sterically hindering RNA pollI transcription), or targeting degradation of its RNA.

In another embodiment, the complex comprises at least a cas13 protein and a guide RNA that form a small complex (crRNA) that is able to bind to MSI2 RNA rather to the DNA and modify it, promoting or inhibiting the endogenous expression of target genes, for instance, MSI2 gene.

In an embodiment, a complex formed by Cas13 that specifically binds to RNA is used to effectively target the RNA of MSI2, thereby the overexpression of MSI2 in MD1 cells. This system is explained in detail in Cov et al. 2017 [Cox, D., Gootenberg, J. S., Abudayyeh, O. O., Franklin, B., Kellner, M. J., Joung, J., & Zhang, F. (2017). RNA editing with CRISPR-Cas13. Science (New York, N.Y.), 358(6366), 1019-1027. https://doi.org/10.1126/science.aaq0180].

In a further embodiment, engineered nuclease-deficient versions of Cas9 or Cas13, termed dCas9 or dCas13, when fused to effector domains with distinct regulatory functions, enables the repurposing of the CRISPR-Cas9 system to a general platform for RNA-guided DNA or RNA targeting for selective degradation or editing, among other possibilities.

### Treatment, uses, administration

In an embodiment according to the first aspect of the present invention, the inhibitor is for use in mammal. In a preferred embodiment, the mammal is a human being.

In a preferred embodiment, the MSI2 inhibitor is for use in the treatment of myotonic dystrophy type 1 or type 2, preferably type 1. As defined in the first aspect, treatment of MD1 is to be preferably understood as a palliative treatment of one or more symptoms of myotonic dystrophy type 1, preferably a palliative treatment of one or more of the muscular disorders that are part of the symptoms of myotonic dystrophy type 1. It is noted that the myotonic dystrophy type 1 which may be prevented or treated in a patient by the present invention is selected from asymptomatic, mild, severe or terminal MD1 or severe congenital myotonic dystrophy type 1, adult-onset MD1, infantile and juvenile MD1 and/or late-onset MD1.

Given the stability of the inhibitors of the present invention, direct administration to human beings and animals can be considered, for example via subcutaneous or systemic routes, preferably intravenously, for example dissolved or suspended in a pharmaceutically acceptable carrier, such as water or an aqueous solution such as saline or phosphate buffer. The composition in which they are administered may contain pharmaceutically acceptable excipients. As to the route of administration, the inhibitors of the present invention can be administered via different routes: intravenous (IV), intraperitoneal (IP), intradermal (ID), intramuscular (IM), intranasal (IN), intratracheal, hydrodynamic tail injection, inhalation, or local organ delivery.

In another embodiment, the inhibitor is in form of a prodrug such that, after administration, it is metabolized or transformed inside the body or cell where it is administrated and is converted into a pharmacologically active drug. The prodrug can be designed to improve the bioavailability, or to improve how the inhibitor according to the first aspect of the present invention is absorbed, distributed, metabolized and/or excreted.

In an embodiment, the present invention includes a method of integrating the inhibitor into the genome of a cell. In certain embodiments, the cell is a eukaryotic cell, e.g., a mammalian or human cell. In certain embodiments, the cell is a genetically engineered cell.

In a **second aspect,** this invention relates to a pharmaceutical composition comprising an inhibitor (or a system of one or more compounds) as defined in the first aspect of the present invention, or a mixture of two or more of them, optionally further comprising a carrier and/or one or more pharmaceutically acceptable excipients. In another embodiment of the second aspect, the pharmaceutical composition comprises an expression vector comprising the nucleotide sequence of at least one of the oligonucleotide molecules as defined in the first aspect of the present invention or a designed protein or peptide that recognizes the same sequences that the oligonucleotide sequences defined in the first aspect binds to. In a preferred embodiment, compatible with the preceding one, the composition comprises at least one of the oligonucleotide molecules described in the present invention, such oligonucleotides targeting the sequences represented by SEQ ID:3 to SEQ ID:37, preferably the nucleotides as set forth in sequences SEQ ID NO: 38 to 43, preferably SEQ ID: 38 and 39. In another possible embodiment, the composition comprises small molecules such as Ro-08-2750 (2,3,4,10-Tetrahydro-7,10-dimethyl-2,4-dioxobenzo[g]pteridine-8-carboxaldehyde), analogues or derivatives thereof.

The expressions "pharmaceutically acceptable" or "pharmacologically acceptable" refer to molecular entities and compositions that do not produce any adverse, allergic or other reactions when administered to an animal or human being. As used herein, "pharmaceutically acceptable vehicle" includes solvents, buffers, solutions, dispersion media, cells, coatings, antibacterial and antifungal agents, isotonic and absorption retarding agents and similar acceptable for use in pharmaceutical formulations, such as pharmaceutical products suitable for administration to human beings. The use of such media and agents for pharmaceutical-active substances is well known in the art. Except where any conventional medium or agent is incompatible with the active ingredients of this invention, their use in the pharmaceutical compositions of the invention is contemplated. Additional active ingredients may also be incorporated into the compositions, provided that they do not inactivate the molecules of this invention or their expression vectors. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the active ingredients of the present disclosure, its use in therapeutic compositions is contemplated. Supplementary active ingredients or other compounds, such as those directed to increase the uptake or the solubility of the inhibitor, also can be incorporated into the compositions, provided they do not inactivate the inhibitor of the compositions.

The pharmaceutical compositions provided herein in the second aspect may generally include one or more pharmaceutically acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like.

The composition is administrated in an effective dose. An effective dose of an inhibitor/antagonist of an oligonucleotide can be from approximately 1 mg/kg to approximately 100 mg/kg, from approximately 2.5 mg/kg to approximately 50 mg/kg, or from approximately 5 mg/kg to approximately 25 mg/kg. The precise determination of what would be considered an effective dose can be based on individual factors for each patient, including size, age, and the nature of the inhibitor or antagonist (for example, if it is an expression construct, an oligoribonucleotide analogue...). Therefore, the dosages can be easily determined by ordinary experts skilled in the art based on this description and the knowledge in the art. It may be necessary or convenient to administer multiple doses to the subject during a particular treatment period, administering doses daily, weekly, monthly, every two months, every three months or every six months. In certain embodiments, the subject receives an initial dose at the beginning which is larger than one or more subsequent doses or maintenance doses.

The composition of this aspect may be administered parenterally, subcutaneously, intravenously, intramuscularly, or intranasally, in particular subcutaneously or intramuscularly. Preferably, the pharmaceutical composition is administrated intramuscularly. In other embodiments, the said pharmaceutical composition is administered by any other path of administration known to the skilled practitioner. In a further preferred embodiment, the said pharmaceutical composition is administered intramuscularly, preferably the pharmaceutical composition is administered intramuscularly in a volume ranging between about 0.1 and 1.0 ml. Preferably, the pharmaceutical composition is administered in a volume ranging between 0.25 and 1.0 ml. More preferably, the said pharmaceutical composition is administered in a volume of about 0.5 ml.

In a **third aspect,** this invention relates to an inhibitor or to a pharmaceutical composition as defined in the first and the second aspect of the present invention, respectively, for use in the treatment of myotonic dystrophy, particularly myotonic dystrophy type 1 or myotonic dystrophy type 2, preferably myotonic dystrophy type 1. Preferably, the pharmaceutical composition is for use in the palliative treatment of one or more symptoms of myotonic dystrophy, more preferably type 1. In a preferred embodiment, the pharmaceutical composition is for use in the palliative treatment of one or more of the muscular disorders that are part of the symptoms of MD1.

In one more aspect, this invention relates to an inhibitor or to a pharmaceutical composition as defined in the first and the second aspect of the present invention, respectively, a mixture of two or more thereof, for the manufacture of a medicinal product for the treatment of myotonic dystrophy, preferably myotonic dystrophy type 1. In this aspect of the invention referred to the use for the manufacture of a medicinal product and, therefore, related to the use in the treatment of myotonic dystrophy type 1, a possible embodiment is that the inhibitor is, or the pharmaceutical composition comprises, a molecule that reduces the intracellular levels of MSI2 transcript, an inhibitor of MSI2 protein, or a molecule that reduces the intracellular protein levels of MSI2, a mixture thereof as defined in the first and second aspects of the present invention.

In a further aspect, the invention relates to a method of treating a subject for myotonic dystrophy, preferably myotonic dystrophy type 1, comprising administering to a mammal at least one an inhibitor of RNA-binding protein Musashi (MSI2) function or an analogue thereof, capable of reducing in a myotonic dystrophy (MD) cell or a cell with the phenotype of a MD cell, as defined in the first, second, and third aspects of the present invention.

According to this aspect of the present invention, the inhibitor used in the method of treating a subject for myotonic dystrophy, preferably myotonic dystrophy type 1, may be an oligonucleotide, a protein, a small molecule, a derivative or analogue thereof, or a system of one or more compounds, as defined in the first aspect of the present invention. In another embodiment, the invention relates to a method of treating a subject for myotonic dystrophy, preferably myotonic dystrophy type 1, comprising administering the composition of the second aspect of the present invention. Preferably, this aspect relates to the palliative treatment of one or more symptoms of myotonic dystrophy, more preferably type 1. In a preferred embodiment, this method is for the palliative treatment of one or more of the muscular disorders that are part of the symptoms of MD1. In a preferred embodiment, the subject is a mammal. In a further preferred embodiment, the subject is a human being.

### SEQUENCE LISTING

SEQ ID NO 1: MSI2 transcript (NCBI Reference Sequence: NM_138962.4)
Homo sapiens musashi RNA binding protein 2 (MSI2), transcript variant 1, mRNA
GTGCGAGGCAGCGGGGCTGAGCTAAGCCGAGCCCACGTGTGACGGCTCTCGCCGCTG
CCCCGGCTCCGCCGCTCGCAGAGAGATTCGGAGGAGCCCGGGCGGGGGGGAGGAGG
AGGGGGAGGAGGGAGCGGAGATCTCGGGGCTCGGAGCCGGCCGCCGCTCCGCTCCG
ATCGCTGTGGGGCTTGGTTTTTGGGGGTGGGGGGCGGGGGGCTCAGATATGGAG
GCAAATGGGAGCCAAGGCACCTCGGGCAGCGCCAACGACTCCCAGCACGACCCCGGT
AAAATGTTTATCGGTGGACTGAGCTGGCAGACCTCACCAGATAGCCTTAGAGACTATTT
TAGCAAATTTGGAGAAATTAGAGAATGTATGGTCATGAGAGATCCCACTACGAAACGCT
CCAGAGGCTTCGGTTTCGTCACGTTCGCAGACCCAGCAAGTGTAGATAAAGTATTAGGT
CAGCCCCACCATGAGTTAGATTCCAAGACGATTGACCCCAAAGTTGCATTTCCTCGTCG
AGCGCAACCCAAGATGGTCACAAGAACAAAGAAAATATTTGTAGGCGGGTTATCTGCGA
ACACAGTAGTGGAAGATGTAAAGCAATATTTCGAGCAGTTTGGCAAGGTGGAAGATGCA
ATGCTGATGTTTGATAAAACTACCAACAGGCACAGAGGGTTTGGCTTTGTCACTTTTGAG
AATGAAGATGTTGTGGAGAAAGTCTGTGAGATTCATTTCCATGAAATCAATAATAAAATG
GTAGAATGTAAGAAAGCTCAGCCGAAAGAAGTCATGTTCCCACCTGGGACAAGAGGCC
GGGCCCGGGGACTGCCTTACACCATGGACGCGTTCATGCTTGGCATGGGGATGCTGG
GATATCCCAACTTCGTGGCGACCTATGGCCGTGGCTACCCCGGATTTGCTCCAAGCTAT
GGCTATCAGTTCCCAGGCTTCCCAGCAGCGGCTTATGGACCAGTGGCAGCAGCGGCG
GTGGCGGCAGCAAGAGGATCAGGCTCCAACCCGGCGCGGCCCGGAGGCTTCCCGGG
GGCCAACAGCCCAGGACCTGTCGCCGATCTCTACGGCCCTGCCAGCCAGGACTCCGG
AGTGGGGAATTACATAAGTGCGGCCAGCCCACAGCCGGGCTCGGGCTTCGGCCACGG
CATAGCTGGACCTTTGATTGCAACGGCCTTTACAAATGGATACCATTGAGCAGGTGCTT
TCGTTGCCATCTCACTCTGAGAGCATACCTGGATGTCCAGGCAAGACTGGGCGAAGTTT
CTGAGTGGCCCTTTGTTTAGGTGATGTCCTCAGACCTGGACCCCCACCAGCCTCACTCC
CCATCCCAACCAGAGATGGCTCACTTCGGATCGAGGGTTGACTACATCTCATCATCTCA
CGAATCTGCTGTAATATAAGACAACAGCTTTTAAATGTGTATATAACCCATGATTTCGGTT
TTGTTTTGTTTTGTTTTTCTTGATGGTTTCCCTCTCCCTCCCTCTCTTCCCATTCTCCTTTT
AAATCTCTTTGAATCACATTTGGTAGTGATTTTGACTTAGTCCAGTAGTCACATAGCTTTA
ATATCTAGTTCAAAGCTAACCATAGTATAATTGTTATATTAAGGAGTTATTTTTTCTTAAAA
CATTTTTTTTTGCTTGTTTTGGTTCTGTTCTCACTTTTAAAGGATGCTGAGATGGTAATAT
GACTCTCCATATTTTGGTACCAATTCTGAGACTGTATGAATTTTCAGGTGGAACTTTAGC
ACACACTGAAGCAAAGTTGTGAAGTGCAGGGCGGGAGGTGGGCGTGAGCTTTCTATTT
TGCGTTGTAGAAGAAGTGAGATGTAGTAAGCTAATTAACAGACTTTCTAGCAGTTCTTTT
TGTGATGTCTCTTTGTTAATCTGAGTCTATCTATTTTCGGCAATAAGGTAAGGACGACAG

TGTTTTGAGTGTCCTCCTTTTCTATAAGTGCTTTTTTTCTGTTGAAAGAGGTGATATTATA
AGGTTTTTTGAAATTGTGAATTCTAAAAAAGAAATGTTGTAAATACAATTCCATTAACTAC
ATAGAAACTATTAAGAAAGAGAGAATCAAAAATATTTTTGTGAGGGAGTCGGTCCCAGG
CAGTTTGATGCTCTGTGGAAGGAGGCGGGAAGGAACGTTGGCCAAGTCAGTTACTGA
GATGAAGATCGCCCAGCTGCCAGGACCACCCCAGGACAAGTTAGAGCACTGTTTAGCT
CCTTTGTCTGTGTGATAGACCTAAGAACTGTATTAGTGTTGTACCAGCCTATTAACCTCT
TGTCTGTGCACAGCTTCAAATGTTACCGTCTAGTTAGATTTTATTTAAAATATGAAAAAC
TGCTTTTCCCAAGATGTTTTTAAAAACAAAGCTACAATTTTAATATTTAACATATTTAAAG
TTTCAAAGCACACCTGTTTGGCTTGGGTGGGGGTGGGGTGGGGGGACATTCTTTTTC
AGTCTTAATTTTTAAATATTTGATCATTTTCTATTGTCCAATCATTTCAGCACCTCCAAAGG
TCCCTAGGACACTTTGCCTCTCTTCTCCCCCTGCCCCCCACCCTGCTCCCACATCTGGG
GGCCCATGGGCCAGGAGTGGATAAGCCTGCATTAATACAACCTTTCTCCATTCACTTTC
TATTTACAAATTAGGAAAGCAACCTTTTGGTTTATATATATTTTTTTTAATACCTCAGTGCT
GCAAGTATCACCAGAGAGGCTATGGAAGAATTTTTTTTAATTTATTGTAGATGTAAACA
GAATTTTAAAAATAAAAAGTATAAACATCACTGCACTGTGACTGGTGGGAAAAACTGACA
GTTTCCTCTTTGCACATGTTTAACATTTGGCTGTTATAATATATGGTCCTCGGTTGGGGA
AAGATACTTATGATGAAGGATATTTTTTAATTTAACTTTTTTTAAATATTGGTAATAGGTC
GGCAACAGCAACTATAGAAGTACAACTCAATAGATGGCATTAAAACATATTGTAGTGTGG
ATATATATTTTTTCTTTTTTAAAATGTGATATTGACGTTTTATTAATATTTTTTAAATTGTTA
CGTTTATAAATTTGGTACTTAAGGCACAGCCAGTATGAGACACTGAATGCGACATTTATT
ATAAAGAGCTGCTGCACTCCTATTTTTATAAATTTTACTAACAAAGTAGACTAATGTAGAC
ATTCACAGACATGGTAGGGCAAAAGCATCTTCAAACTAAAGACTCCAAAATGCTAACTCA
GAAAGAAAGAAAAAACCCGTTTTCAATTCTAATGAAACAGCAACAACATTTTTTTTAATTA
AAAAAAAAATCATGTTCTTTGTTTTTCTAATAAAATGTTAGGTTGTTTGGTGAGGTTTTTTT
GTTGTTTTTTTCCTTTCTTTGTTTCTTTTCTTTTCCCCCAAACAACCAGATTAAATGCTGAG
CGCTTTTAAAATATCAAAACTTGTTCAAGTCATAAAACAACAAAACATAAGTTTTATTTAAA
AAAAAAAAAAGAAAGAAAAAATAGTAAATTCCCAGAAATTCAGTGTTTAGACGAGGAAT
TTAATTCCTATTTTGTCCATGTTGGTGATGTACTGTACTTCCCTTCCTTTTCTCTGCATCC
CCCATCACCTCATAGAAGACTCTTTGTTGATCATTGTATGTTAATAATGTATAAAATGGCT
ATCTTGTAAGCGTGCTGTCCTGGTACTAGTGTAGCGACTTTTTTTCTCCTCTTTCTTCTA
GTACATATTGATAGGTATAACATAATTAAGGTTTAAAAAAAATTAGACATAGTTATTCAGA
TTTAGGACCAGTAAGGATAGAACTTTCTCTTATTTATGAAAAAAAATGCTAATAATTTTGG
GGCAGTTTTTTCCTTTAATTATTTTTTTCAATTTCAAGTTTAATTTTATTTTAGCTGATCTGA
TGTGGTTTCAACTAACCCAAGGTCTCACCATGTTAAAATGCCGGCGGACTCTACGGCGT
TTTGTAGATCCCCCCCCCCCCACCCACTGTGAAGGGGTGCCATACTACCTTAAATGCTA
ATGCTAGATATGCAAAACTGGATTTTTTTAATTTATTTTTTAAAAGAGGAGGCATGGTAT
ATTAAAATGATTTTACTAAGAGAAAAAATATTTTTTTAAGAATGCTCAGAAGAAATTGATAA

TCTGTGTGAATATGTTTTAGATGTTTATATACCTTTTGAAGAGACCCAGTAGCCCATAGC
ACAAATCTTGTGGAAATCCGATATGTTTTAATGTGGCTACCTAGGTTTAAGGTTCACGTT
AGTCCCCCCATTCCATCTAGAAGTCCATTTTGAAAGATTTTTGTAAATTCTTTTAACACTG
ATGTTTCAGCCTCGTCTTTGTTTCAGTTAAGCTCAATGGCGAACATGGAACCACCTTTC
GCCTTCCCTGGGGGAGAAACCCTCTTGGCTGATGGCTTTTCCCCGGAATTATCAAACAG
CCACCGGGTGACTTTCTGGCTTCCAGATCCATCTGCCTGAGACCCCCGAACTCCTCTCC
TCCCAAGCAGAGGCGAGTGAGTGGCATTAGCTCCCGGACCCATTCCCGGTCCTAGCTG
GGCATGGGGCTGACGGAGATGACCAAGCCTTGGTCTGCTCTCTAGCAGCTTCCACAGA
CTTGGCTCGTGGCCTTCCTTATATCCACTGGGAACAAACAGCCTCGCGCTCTATACCAA
ACAGCCTCGCGCTCTATCCCAGTCGCCCATTAGCTTGATTCAAACAAAGCCCCAGCAGG
CCTTTGCGTTTTTATCCTTCATAACCTTCATCTTAATTTGAACTTGTAGCTTGGACTTTAA
GGTAGCATGGCTCTATTGCTGTCAATTTACTGTTTCACTGCACAGCAATCACAGCCAGT
GAATGTTACACACATCTTGCTAGACTAGTATAAAAATCATTGGGTAATTGTTGGTTCTAAT
GACCTGAAAGGTGTTCAGTTTTTGTTCTTGGTTTTGTTTTGTTTTTGATTTCTTGGGGGTG
GGTTTTGCTTGTTGTTCCTTTTCATTTGGGGGTTTTTGGGGAAAAAATTTATTTTTGGTTC
CAAATAGAAAAACAAAACCTATTTTGATCTTTAGTGCAAACGAGGGCTAGGGACTTAGCC
TCCTCCACCACCTCCACACTGCTTCATTCTGCCATTCACTCACTGCAGCATATTCAAGAA
TAAAGCAATATCGTTTACTACATTTTTTATTGAAGGTCAGCCATGCTTTCTGTATTATATT
GCATATGAAATTGTTTACAAAAGAAACACTAACTCATACTTCTCTTTATTGGTTGCAAGTG
GCACGCAGGAACAGAGGGAGAGTGGGGGGCTGGTGGGGGAGGGGAGATTTTTTTTTT
TTTTTCTTGAATGTGCTTCGCAAGCCAGGCTATCTTCCAAGGAAGGCAGACAGTGGGAG
AGCAGAGGGACTGACTGCAGGCAAGCACATTGAAGAAAGACACTGGCGGGTTTCCCCA
CCCTCACCCCAAAGCAGAAAACTAGCAGACGTCAGCTCAGCCCCGTCCTGGGCACAGA
CACTACACAAGGAGACGCTGGAAGTTAAGCAATACTTTAATACTGTAATATGTTTGTTTT
CTTTTTCTTTCTTTTTTTTCTACCAAAAAAAAGTAAGTAAACTAAAACACAAAAACATATAA
ATAAAATCCATCCCTCTTGTCGGGGACCTGCAGGGGGGCAACCTTAATCCAAACACCTG
GCTATCAAATAATCAGAATGTATTGTCTCAGACAGGATTTCAGTTCCGGGAGGCAGGGG
CATGATGGGGGAGGGGGCCGGAGGCTGAGAGACAAAAGTTCCAGAGCCTCCCTCGAA
GGTTCTCTACTACTGTATTCTGTACATAATGTACCATCCCATGTGGAATCTGTGAGTGTC
CTCTTAAGTAGCGTGGGCTAGCCAATCTGCCGTTCATGGTGTATTGTAAACTCCGAATT
CCATATGTAATAGGATGCAAGTCTAAGCGTTTCATGTGGACATAAATGTATCTAAATAAA
ACTTTCCCTAGCACTGTGGCTGACCTCACCCTTACTTTTATACTTTAGTATGAAACTGAT
GAGAACTTTGGTAGTGAGTATTTTTTTTATATATATACATATATATGTACTATCTATATATA
TATCTCAAGCATCTTTCAGGTCTTTGTGTGTGGCTTTCTTAAAGCCCTGTTGTAAAAAATT
ACTATGTGGATGGCAGTCTCTCACATCACAGATGTGGAAAGTATAATTTTATATTTGTATT
TTCAAATAAATAAGTTTGTGAAAGGTTTCCATCCTCTACTGTGGTCCAGAAATCAATGTG
TTTGTCTGACAAAAAAAAAAATAAAATAAAATAAACTGTTTTGAACAGA

SEQ ID NO 2: Partial MSI2 transcript (nucleotides 327-1618):
GCCTTAGAGACTATTTTAGCAAATTTGGAGAAATTAGAGAATGTATGGTCATGAGAGATC
CCACTACGAAACGCTCCAGAGGCTTCGGTTTCGTCACGTTCGCAGACCCAGCAAGTGT
AGATAAAGTATTAGGTCAGCCCCACCATGAGTTAGATTCCAAGACGATTGACCCCAAAG
TTGCATTTCCTCGTCGAGCGCAACCCAAGATGGTCACAAGAACAAAGAAAATATTTGTA
GGCGGGTTATCTGCGAACACAGTAGTGGAAGATGTAAAGCAATATTTCGAGCAGTTTGG
CAAGGTGGAAGATGCAATGCTGATGTTTGATAAAACTACCAACAGGCACAGAGGGTTTG
GCTTTGTCACTTTTGAGAATGAAGATGTTGTGGAGAAAGTCTGTGAGATTCATTTCCATG
AAATCAATAATAAAATGGTAGAATGTAAGAAAGCTCAGCCGAAAGAAGTCATGTTCCCAC
CTGGGACAAGAGGCCGGGCCCGGGGACTGCCTTACACCATGGACGCGTTCATGCTTG
GCATGGGGATGCTGGGATATCCCAACTTCGTGGCGACCTATGGCCGTGGCTACCCCGG
ATTTGCTCCAAGCTATGGCTATCAGTTCCCAGGCTTCCCAGCAGCGGCTTATGGACCAG
TGGCAGCAGCGGCGGTGGCGGCAGCAAGAGGATCAGGCTCCAACCCGGCGCGGCCC
GGAGGCTTCCCGGGGGCCAACAGCCCAGGACCTGTCGCCGATCTCTACGGCCCTGCC
AGCCAGGACTCCGGAGTGGGGAATTACATAAGTGCGGCCAGCCCACAGCCGGGCTCG
GGCTTCGGCCACGGCATAGCTGGACCTTTGATTGCAACGGCCTTTACAAATGGATACCA
TTGAGCAGGTGCTTTCGTTGCCATCTCACTCTGAGAGCATACCTGGATGTCCAGGCAAG
ACTGGGCGAAGTTTCTGAGTGGCCCTTTGTTTAGGTGATGTCCTCAGACCTGGACCCCC
ACCAGCCTCACTCCCCATCCCAACCAGAGATGGCTCACTTCGGATCGAGGGTTGACTA
CATCTCATCATCTCACGAATCTGCTGTAATATAAGACAACAGCTTTTAAATGTGTATATAA
CCCATGATTTCGGTTTTGTTTTGTTTTGTTTTTCTTGATGGTTTCCCTCTCCCTCCCTCTC
TTCCCATTCTCCTTTTAAATCTCTTTGAATCACATTTGGTAGTGATTTTGACTTAGTCCAG
TAGTCACATAGCTTTAATATCTAGTTCAAAGCTAACCATAGTATAATTGTTATATT

SEQ ID NO 3:
   GCCTTAGAGACTATTTTAGCAAA
SEQ ID NO 4:
   TAGAGACTATTTTAGCAAATTTG
SEQ ID NO 5:
   GACTATTTTAGCAAATTTGGAGA
SEQ ID NO 6:
   AGCAAATTTGGAGAAATTAGAGA
SEQ ID NO 7:
   AGCAAGTGTAGATAAAGTATTAG
SEQ ID NO 8:
   CACAAGAACAAAGAAAATATTTG
SEQ ID NO 9:
   AAGAACAAAGAAAATATTTGTAG
SEQ ID NO 10:
   GTGGAAGATGTAAAGCAATATTT
SEQ ID NO 11:
   TGGAAGATGTAAAGCAATATTTC
SEQ ID NO 12:
   TGCTGATGTTTGATAAAACTACC
SEQ ID NO 13:
   TGGCTTTGTCACTTTTGAGAATG
SEQ ID NO 14:
   GGCTTTGTCACTTTTGAGAATGA
SEQ ID NO 15:
   CACTTTTGAGAATGAAGATGTTG
SEQ ID NO 16:
   AAGATGTTGTGGAGAAAGTCTGT
SEQ ID NO 17:
   GAGAAAGTCTGTGAGATTCATTT
SEQ ID NO 18:
   TTCATTTCCATGAAATCAATAAT
SEQ ID NO 19:
   TTCCATGAAATCAATAATAAAAT
SEQ ID NO 20:
   TCCAAGCTATGGCTATCAGTTCC
SEQ ID NO 21:
   TTGATTGCAACGGCCTTTACAAA
SEQ ID NO 22:
   CACGAATCTGCTGTAATATAAGA
SEQ ID NO 23:
   AACAGCTTTTAAATGTGTATATA
SEQ ID NO 24:
   CAGCTTTTAAATGTGTATATAAC
SEQ ID NO 25
   AGCTTTTAAATGTGTATATAACC
SEQ ID NO 26:
   TCCCATTCTCCTTTTAAATCTCT
SEQ ID NO 27:
   CTCCTTTTAAATCTCTTTGAATC
SEQ ID NO 28:
   TCCTTTTAAATCTCTTTGAATCA
SEQ ID NO 29:
   CTCTTTGAATCACATTTGGTAGT
SEQ ID NO 30:
   ATCACATTTGGTAGTGATTTTGA
SEQ ID NO 31:
   TGGTAGTGATTTTGACTTAGTCC
SEQ ID NO 32:
   CACATAGCTTTAATATCTAGTTC
SEQ ID NO 33:
   TAGCTTTAATATCTAGTTCAAAG
SEQ ID NO: 34:
   AGCTTTAATATCTAGTTCAAAGC
SEQ ID NO: 35:
   TTCAAAGCTAACCATAGTATAAT
SEQ ID NO: 36:
   AGCTAACCATAGTATAATTGTTA
SEQ ID NO: 37:
   AACCATAGTATAATTGTTATATT
SEQ ID NO 38: ASO 1
   TGACTTCTTTCGGCTG
SEQ ID NO 39: ASO 3
   TTGGATTAAGGTTGCC
SEQ ID NO 40: siRNA 1
   TGACTTCTTTCGGCTGAGCTT
SEQ ID NO 41: Sense AgomiR-7 (mimic of miR-107)
   AGCAGCAUUGUACAGGGCUAUCA
SEQ ID NO 42: Antisense AgomiR-7 (mimic of miR-107)
   UGAUAGCCCUGUACAAUGCUGCU
SEQ ID NO 43: siRNA 2
   AGTTTTATCAAACATCAGCAT
SEQ ID NO 44: ASOS SCRAMBLE (control)
   TTCCCTGAAGGTTCCT
SEQ ID NO 45-68: See Table 2.

SEQ ID NO 69: AAV9-hDES-mMSI2(V4)-V5-T2A-Nanoluc
GCTAGCGCGGCCGCACCCATGCCTCCTCAGGTACCCCCTGCCCCCACAGCTCCTCTC
CTGTGCCTTGTTTCCCAGCCATGCGTTCTCCTCTATAAATACCCGCTCTGGTATTTGGG
GTTGGCAGCTGTTGCTGCCAGGGAGATGGTTGGGTTGACATGCGGCTCCTGACAAAAC
ACAAACCCCTGGTGTGTGTGGGCGTGGGTGGTGTGAGTAGGGGGATGAATCAGGGAG
GGGGCGGGGGACCCAGGGGGCAGGAGCCACACAAAGTCTGTGCGGGGGTGGGAGCG
CACATAGCAATTGGAAACTGAAAGCTTATCAGACCCTTTCTGGAAATCAGCCCACTGTTT
ATAAACTTGAGGCCCCACCCTCGAGATAACCAGGGCTGAAAGAGGCCCGCCTGGGGG
CTGCAGACATGCTTGCTGCCTGCCCTGGCGAAGGATTGGCAGGCTTGCCCGTCACAGG
ACCCCCGCTGGCTGACTCAGGGGCGCAGGCCTCTTGCGGGGGAGCTGGCCTCCCCGC
CCCCACGGCCACGGGCCGCCCTTTCCTGGCAGGACAGCGGGATCTTGCAGCTGTCAG
GGGAGGGGAGGCGGGGGCTGATGTCAGGAGGGATACAAATAGTGCCGACGGCTGGG
GGCCCTGTCTCCCCTCGCCGCATCCACTCTCCGGCCGGCCGCCTGCCCGCCGCCTCC
TCCGTGCGCCCGCCAGCCTCGCCCGCGCCGTCACCGGTTCGAACAGGTAAGCGCCCC
TAAAATCCCTTTGGCACAATGTGTCCTGAGGGGAGAGGCAGCGACCTGTAGATGGGAC
GGGGGCACTAACCCTCAGGGTTTGGGGTTCTGAATGTGAGTATCGCCATCTAAGCCCA
GTATTTGGCCAATCTCAGAAAGCTCCTGGCTCCCTGGAGGATGGAGAGAAAAACAAA
CAGCTCCTGGAGCAGGGAGAGTGTTGGCCTCTTGCTCTCCGGCTCCCTCTGTTGCCCT
CTGGTTTCTCCCCAGGTTCGAAGGATCCATGGAGGCAAATGGAGCCCAGGCACCTCG
GGCAGCGCCAACGACTCCCAGCACGACCCCGGTAAAATGTTTATCGGTGGACTGAGCT
GGCAGACCTCACCAGATAGCCTTAGAGACTATTTTAGCAAATTTGGAGAAATTAGAGAT
GTATGGTCATGAGAGATCCCACAACGAAACGCTCCAGAGGCTTCGGTTTCGTCACCTTC
GCAGACCCAGCAAGTGTAGATAAAGTATTAGGTCAGCCCCACCATGAGTTAGATTCCAA
GACGATTGACCCAAAAGTTGCATTTCCTCGTCGAGCGCAACCTAAGATGGTCACAAGAA
CAAAGAAAATCTTCGTAGGAGGATTGTCTGCAAACACAGTAGTGGAAGATGTAAAGCAG
TATTTCGAGCAGTTTGGCAAGGTAGAGGATGCGATGCTGATGTTCGACAAAACCACCAA
CAGGCACAGAGGGTTTGGCTTTGTCACCTTTGAGAATGAAGACGTTGTGGAGAAAGTCT
GTGAGATTCATTTCCATGAAATCAATAATAAAATGGTAGAATGTAAGAAAGCTCAGCCGA
AAGAAGTCATGTTCCCACCTGGGACAAGAGGCCGGGCCCGGGGGCTGCCATACACCAT

GGATGCGTTCATGCTTGGCATGGGGATGCTGGGCTACCCCAACTTTGTGGCAACCTAT
GGCAGAGGCTACCCCGGATTTGCTCCTAGCTATGGCTACCAGTTCCCAGGCTTCCCGG
CGGCAGCTTATGGACCAGTGGCAGCGGCAGCTGTGGCAGCGGCTCGAGGATCAGGCT
CCAACCCGGCGCGGCCCGGAGGCTTCCCGGGGGCCAACAGCCCAGGACCTGTCGCC
GATCTCTACGGCCCTGCCAGCCAGGACTCCGGAGTGGGGAATTACATAAGCGCGGCCA
GCCCACAGCCGGGCTCCGGCTTCGGCCACGGCATAGCTGGACCTTTGATTGCAACGG
CCTTTACAAATGGATACCACTGAGGTAAGCCTATCCCTAACCCTCTCCTCGGTCTCGATT
CTACGGAGGGCAGAGGAAGTCTGCTAACATGCGGTGACGTCGAGGAGAATCCTGGCC
CAGGATCCATGGTCTTCACACTCGAAGATTTCGTTGGGGACTGGCGACAGACAGCCGG
CTACAACCTGGACCAAGTCCTTGAACAGGGAGGTGTGTCCAGTTTGTTTCAGAATCTCG
GGGTGTCCGTAACTCCGATCCAAAGGATTGTCCTGAGCGGTGAAAATGGGCTGAAGAT
CGACATCCATGTCATCATCCCGTATGAAGGTCTGAGCGGCGACCAAATGGGCCAGATC
GAAAAAATTTTTAAGGTGGTGTACCCTGTGGATGATCATCACTTTAAGGTGATCCTGCAC
TATGGCACACTGGTAATCGACGGGGTTACGCCGAACATGATCGACTATTTCGGACGGC
CGTATGAAGGCATCGCCGTGTTCGACGGCAAAAAGATCACTGTAACAGGGACCCTGTG
GAACGGCAACAAAATTATCGACGAGCGCCTGATCAACCCCGACGGCTCCCTGCTGTTC
CGAGTAACCATCAACGGAGTGACCGGCTGGCGGCTGTGCGAACGCATTCTGGCGTAAG
TATAC

### EXAMPLES

### EXAMPLE 1

### Materials and Methods

### Chemically- modified oligonucleotides

siRNAs targeting *MSI2* transcripts and negative control were commercially available from Invitrogen (Madrid, Spain), *Silencer*^{®} Select siRNA. Cat# 4390843 for negative control and Cat#4392421 for siRNAs against MSI2 (ID S42755 and S42757). Gapmer oligonucleotides and scramble control were synthesized by biomers.net GmbH.

The ASOs sequences were as follows:
ASO1 (SEQ ID NO: 38) 5' +T+G+A*C*T*T*C*T*T*T*C*G*G+C+T+G 3'
ASO3 (SEQ ID NO: 39) 5' +T+T+G*G*A*T*T*A*A*G*G*T*T+G+C+C 3'
Scramble (SEQ ID NO: 44) 5' +T+T+C*C*C*T*G*A*A*G*G*T*T+C+C+T 3'
Where + denotes locked nucleic acid and * denotes phosphorothioate linkages.

Hsa-miR-107 agomiR (agomiR-107) (SEQ IDs 41 (sense) and 42 (antisense)) were synthesized by biomers.net GmbH according to the following sequences:
AgomiR-107 sense strand 5'AGCAGCAUUGUACAGGGCUAUCA 3'
AgomiR-107 antisense strand: 5'mU*mG*mAmUmAmGmCmCmCmUmGmU-mAmCmAmAmUmGmCm U*mG*mC*mU*3' chol

Where m denotes 2-O-methyl-modified phosphoramidites, * denotes phosphonothioate linkages, and "chol" denotes cholesterol groups

### Cells

MD1 cells are cells affected with MD1. In the present invention, these cells are obtained from patient-derived biopsies (Fig. 2 A), from primary cultures thereof (primary myoblasts, as shown in Fig. 2 B) or from immortalized skin fibroblast cell line expressing conditional MyoD (also referred to as "reference cells" as described in Arandel et al. 2017 and as explained above). These MD1 immortalized skin fibroblast cells expressing conditional MyoD are used in all figures except in Fig. 2A and B. The MD1 cells are the reference cells used to study the effects of the inhibitors described herein.
Skin fibroblasts transdifferentiated into multinucleated myotubes for 7 days are called herein TDM cells and are obtained as previously described¹

Non-DM control (Ctrl) cells (CNT), also referred in the present invention as healthy cells, are cells obtained as above but from a healthy or non-MD1 biopsies or from primary cultures thereof.

### Cell culture and treatment with oligonucleotides and small molecule

TDMs were cultured as previously described¹. After 4 days in differentiation medium (MDM), cells were transfected with a combination of two siRNAs (SEQ ID NO: 40 and 43) targeting *MSI2* transcripts (final concentration 100 nM) using Lipofectamine RNAiMAX transfection reagent (Invitrogen, Madrid, Spain)) following the manufacturer recommendations. After 48 h, the medium containing the siRNAs was replaced by fresh MDM. 24 h later, cells were collected. As a control, the same protocol was performed using 100 nM of a siRNA with a scrambled sequence (Cat# 4390843 for negative control). Gapmers against MSI2 and agomiR-107 were transfected following the same protocol using two different concentrations, 30 and 150 nM in the case of Gapmers and 100 and 300 nM in the case of agomiR-107. To treat cells with the small molecule Ro-08-2750 (Tocris Cat. No. 2272, Bristol, UK)), cells were cultured as before. Briefly, after 96 h in MDM, the medium was supplemented with Ro-08-2750 at a final concentration of 10 µM (0.8% DMSO) or with the vehicle, DMSO 0.8%, for 48 h. Then cells were collected for further analyses.

### Primary Human Skeletal Muscle Cultures

Primary human myoblasts from muscle biopsies of 3 MD1 and 3 control patients were obtained as previously described² by clinician collaborators from the La Fe Hospital (Valencia, Spain). In order to obtain highly purified myoblast cell cultures, positive selection for the CD56 surface marker was performed using CD56-coated microbeads (Milteny Biotec, Bergisch Gladbach, Germany) following manufacturer instructions². Purified myoblasts were seeded in 6 well plates (2.5 x 10⁵ cells/well) and the following day the medium was substituted with differentiation medium (DMEM + 2% horse serum). Medium was changed every 2 days, and pellets were collected at day 7 of differentiation.

### MD1 patients and skeletal muscle biopsies

All muscle biopsies were taken by clinician collaborators after informed consent from patients with approval by the institutional review boards (IRBs) of the University Hospital La Fe (Valencia, Spain experimentation ethics committee authorization 2014/0799). Muscle biopsies used for *miR-7* and MSI2 determinations were obtained at La Fe University Hospital. A detailed description of muscle type, and repeats length is provided in Table 1

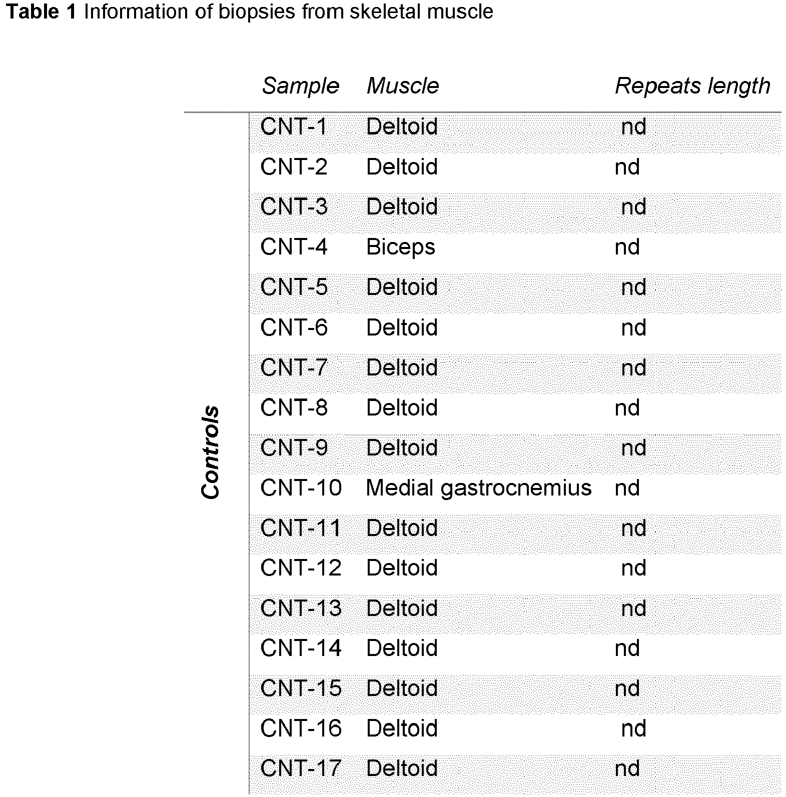

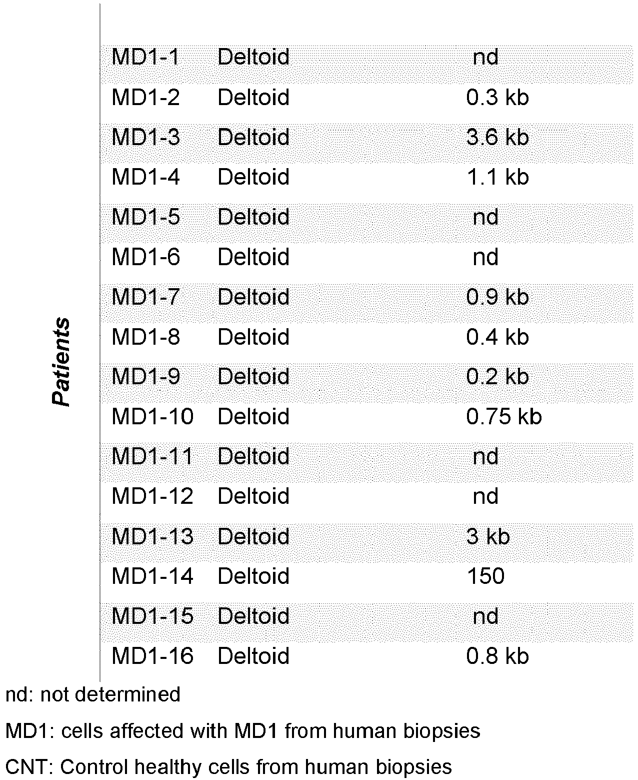

### Immunofluorescence methods

Desmin immunostaining and fusion index and diameter determination were performed as previously described³. For MSI2 immunodetection, 3.5x10⁴ fibroblasts/well were seeded in 24 well plates. After 7 days of differentiation into myotubes, cells were fixed with 4% paraformaldehyde (PFA) for 15 min. After 3 washes with PBS-T (0.1% X-TritonX-100 in PBS 1x) cells were blocked (PBS-T, 1% BSA, 5% normal goat serum) for 1 h and incubated with rabbit anti-MSI2 antibody (1:100, Abcam, EP1305Y, Cambridge, UK) in blocking buffer for 48 h at 4°C. After three washes with PBS-T cells were incubated for 1 h with goat anti-rabbit-FITC IgG (1:200, Sigma-Aldrich, St. Louis, Missouri) in blocking buffer. Finally, cells were washed thrice with PBS 1x and were counterstained and mounted with VECTASHIELD^{®} mounting medium containing DAPI (Vector Laboratories, London, UK) to detect the nuclei. Images were taken in a confocal microscope LSM800 (Zeiss) at 400x magnification.

### LysoTracker staining

2.5x10⁴ cells were seeded in a 24-well plate. After treatment with ASOs for 48 h, samples were incubated 30 min at 37°C with 100 nM LysoTracker RED-DND99 (Invitrogen, Madrid, Spain) and 5 µg/ml Hoechst 33258 (Sigma-Aldrich, St. Louis, Missouri). After two washes with warmed PBS, cells were fixed with 4% PFA for 15 min followed by washes in 1x PBS. Then, cells were mounted using fluorescence mounting medium Dako (Agilent Technologies, Santa Clara, California). Images were acquired in a LSM800 confocal microscope (Zeiss) at 400x magnification.

### Toxicity assay

Non-DM control (Ctrl) cells (CNT) were aliquoted in 96-well plates with 1.0x10⁵ cells per well. After 24 h cells were transfected as described with different ASOs (concentrations ranging from 1 nM to 1 µM) or treated with Ro-08-2750 (Tocris Bioscience, Bristol, UK) at concentrations ranging from 0.0195 up to 80 µM (in 1:2 serial dilutions). Cell viability was measured as previously described³.

### RNA extraction, semiquantitative PCR and real time PCR.

All the procedures were performed as previously described¹. Specific primers not previously reported are listed in Table 2.

**Table 2 Sequences of oligonucleotides used for RT-qPCR and semiquantitative RT-PCR**

| Primer name | Sequence (5' → 3') | technique | Species | SEQ ID NOs: |
|---|---|---|---|---|
| *GAPDH fwd* | CATCTTCCAGGAGCGAGATC | RT-qPCR/ RT-PCR | *H. sapiens* | 45 |
| *GAPDH rev* | GTTCACACCCATGACGAACAT | RT-qPCR | *H. sapiens* | 46 |
| *GPI fwd* | CAGGGCATCATCTGGGACAT | RT-qPCR | *H. sapiens* | 47 |
| *GPI rev* | TCTTAGCCAGCTGCTTTCCC | RT-qPCR | *H. sapiens* | 48 |
| *HPRT1 fwd* | TGACACTGGCAAAACAATGCA | RT-qPCR | *H. sapiens* | 49 |
| *HPRT1 REV* | GGTCCTTTTCACCAGCAAGCT | RT-qPCR | *H. sapiens* | 50 |
| *IGF1 fwd* | CTCTTCAGTTCGTGTGTGGAGAC | RT-qPCR | *H. sapiens* | 51 |
| *IGF1 rev* | CAGCCTCCTTAGATCACAGCTC | RT-qPCR | *H. sapiens* | 52 |
| *MSI2 fwd* | GCAGACCTCACCAGATAGCCTT | RT-qPCR | *H. sapiens* | 53 |
| *MSI2 rev* | AAGCCTCTGGAGCGTTTCGTAG | RT-qPCR | *H. sapiens* | 54 |
| *MSTN fwd* | TGAGAATGGTCATGATCTTGCTGT | RT-qPCR | *H. sapiens* | 55 |
| *MSTN rev* | TCATCACAGTCAAGACCAAAATCC | RT-qPCR | *H. sapiens* | 56 |
| *mTOR fwd* | AGCATCGGATGCTTAGGAGTGG | RT-qPCR | *H. sapiens* | 57 |
| *mTOR rev* | CAGCCAGTCATCTTTGGAGACC | RT-qPCR | *H. sapiens* | 58 |
| *NFIX fwd* | GAGCCCTGTTGATGACGTGTTCTA | RT-PCR | *H. sapiens* | 59 |
| *NFIX rev* | CTGCACAAACTCCTTCAGTGAGTC | RT-PCR | *H. sapiens* | 60 |
| *P21 fwd* | AGGTGGACCTGGAGACTCTCAG | RT-qPCR | *H. sapiens* | 61 |
| *P21 rev* | TCCTCTTGGAGAAGATCAGCCG | RT-qPCR | *H. sapiens* | 62 |
| *PKM fwd* | CTGAAGGCAGTGATGTCGCC | RT-PCR | *H. sapiens* | 63 |
| *PKM rev* | ACCCGGAGGTCCACGTCTC | RT-PCR | *H. sapiens* | 64 |
| *ATP2A fwd* | GATGATCTTCAAGCTCCGGGC | RT-PCR | *H. sapiens* | 65 |
| *ATP2A rev* | CAGCTCTGCCTGAAGATGTG | RT-PCR | *H. sapiens* | 66 |
| *TGFBR1 fwd* | GACAACGTCAGGTTCTGGCTCA | RT-qPCR | *H. sapiens* | 67 |
| *TGFBR1 rev* | CCGCCACTTTCCTCTCCAAACT | RT-qPCR | *H. sapiens* | 68 |

### Western blotting

Protein extraction, quantification and immunodetection was performed as previously described^{1,3}. Specifically, MSI2 detections were performed by incubating membranes overnight with a rabbit anti-MSI2 antibody (1:1000, Abcam, EP1305Y, Cambridge, UK). Goat horseradish peroxidase (HRP)-conjugated anti-rabbit-IgG (1:3500, Sigma-Aldrich, St. Louis, Missouri) was used as secondary antibody. Images were acquired with an ImageQuant LAS 4000 or AMERSHAM ImageQuant 800 (GE Healthcare). Quantification was performed using ImageJ software (NIH).

### Statistical analyses

In all molecular studies, for comparison on mean data, all parameters were assumed to follow a normal distribution, and the samples were compared using two-tailed t-tests (α= 0.05), applying Welch's correction when necessary. The statistical differences were estimated by the Student's t-tests (p< 0.05) on normalized data. Sample size (n) is included in the figure legends.

### AAV synthesis

Cloning and production of adeno-associated virus (AAVs) vectors was done by the Viral Vector Production Unit from Universitat Autònoma de Barcelona (UAB). The construct used for overexpressing isoform 4 of murine *Msi2* was AAV9-hDES-mMsi2(v4)-V5-T2A-Nanoluc.The specific construct used herein is disclosed above in SEQ ID NO: 69.

### Transgenic mice, AAV administration and blood and tissue collection

Mouse handling and experimental procedures conformed to the European law regarding laboratory animal care and experimentation (2003/65/CE) and were approved by *Conselleria de Agricultura, Generalitat Valenciana* (reference number 2019/VSC/PEA/0150). Homozygous transgenic HSA^{LR} (line 20 b) mice were provided by Prof. C. Thornton (University of Rochester Medical Center) and mice with the same genetic background (FVB) were used as controls. Experiments were performed in 4.5-month-old males. Animals received one intraperitoneal shot of 1x10¹² vg or 1.75x10¹² vg. Experimental groups were treated with PBS (n=5 FVB, n=3 HSA^{LR}) or MSI2-AAV (n=3 HSA^{LR}) and at six or ten weeks after the injection animals were sacrificed and quadriceps and gastrocnemius were harvested. Each muscle was divided into three parts, two were snap frozen in liquid nitrogen for protein and RNA isolation and the third was frozen in isopentane for histological analyses. Blood samples were collected from slightly anesthetized animals, previous euthanasia, by cardiac puncture. Samples were analyzed by Montoro Botella Laboratories.

### Electromyography studies and forelimb grip strength test.

Both studies were performed before AAV injection and before sacrifice. Analysis was done under general anaesthesia, as previously described³. Briefly, five needle insertions were performed in each quadriceps muscle of both hind limbs, and myotonic discharges were graded on a five-point scale: 0, no myotonia; 1, occasional myotonic discharge in ≤50% of the needle insertions; 2, myotonic discharge in >50% of the insertions; 3, myotonic discharge in nearly all of the insertions; and 4, myotonic discharge in all insertions. The forelimb grip strength was measured as previously described³.

### Fluorescent histological analysis of mouse muscle samples

For quantification of muscle fiber cross-sectional area, gastrocnemius and quadriceps were frozen in isopentane and 15 µm-sections were obtained with a Leica CM 1510S cryostat. For muscle fiber cross-sectional area quantification, muscle sections were washed with PBS1x and inmunostained with Wheat Germ Agglutinin, Alexa Fluor^{™} 488 Conjugate (1:200) for 45 min, washed three times with PBS1x and finally mounted with VECTASHIELD^{®} mounting medium containing DAPI (Vector Laboratories, London, UK)) to detect the nuclei. Images were taken in a confocal microscope LSM800 (Zeiss) at 400x magnification. Around 5000 fibers were analyzed in each condition by using a plugin "Muscle morphometry" of Fiji-Image J software (NIH).

Sections were stained with hematoxylin-eosin (H&E) and mounted with DPX (Sigma-Aldrich) according to standard procedures. Images were taken at a 100x magnification with a Leica DM2500 microscope. All parameters were analyzed in a total of 500 fibers in each mouse by using ImageJ software (NIH).

### References

1. Sabater-Arcis, M., Bargiela, A., Furling, D., and Artero, R. (2020). miR-7 Restores Phenotypes in Myotonic Dystrophy Muscle Cells by Repressing Hyperactivated Autophagy. Mol. Ther. - Nucleic Acids 19, 278-292.
2. de Luna N, Gallardo E, Soriano M, Dominguez-Perles R, de la Torre C, Rojas-García R, García-Verdugo JM, IIIa I. (2006). Absence of dysferlin alters myogenin expression and delays human muscle differentiation "in vitro". J Biol Chem. 28125:17092-8.
3. Bargiela, A., Sabater-Arcis, M., Espinosa-Espinosa, J., Zulaica, M., Lopez de Munain, A., and Artero, R. (2019). Increased Muscleblind levels by chloroquine treatment improve myotonic dystrophy type 1 phenotypes in in vitro and in vivo models. Proc. Natl. Acad. Sci. 116, 25203-25213.

### EXAMPLE 2

The transcript and protein levels of MSI2 were measured and compared between human biopsies of healthy people and patients suffering from MD1. Table 3 below shows the fold change values of MD1 patients vs healthy (used herein as reference), where it can be observed that the MSI2 RNA levels are increased in patients suffering from MD in a fold change of 1.358, and MSI2 protein levels are increased in patients suffering from MD in a fold change of 1.594. Further, the miR-7 levels were also compared in healthy controls versus MD1 biopsies, showing a decrease in miR-7 intracellular levels in patients of 0.58 fold relative to controls. Lastly, the levels of RNA TGFBR1 were also measured, since TGFBR1 is a gen whose mRNA is directly increased by MSI2 activity. The results showed that the TGFBR1 levels were increased in 1.4 fold respect to healthy people.

**Table 3. Fold change values MD1 versus healthy patients**

| | | **Human Biopsies** |
|---|---|---|
| **MSI2** | RNA | 1.358 |
| | Protein | 1.594 |
| **Mir-7** | | 0.58 |
| **TGFBR1** | RNA | 1.4 |

Similar comparison was performed in MD1 primary myoblasts in comparison with healthy control cells, where the fold change values are shown in Table 4 below:

**Table 4. Fold change values MD1 cells versus control cells**

| | | **MD1 primary myoblasts** |
|---|---|---|
| **MSI2** | RNA | 1.8 |
| | Protein | 2 |
| **Mir-7** | | 0.51 |

### EXAMPLE 3

Relative expression levels of HuR and MSI2 transcripts in healthy control and MD1 cells were analysed, and the results showed that, while no significant differences were found in HuR at the transcript or protein levels, MSI2 was strongly upregulated in two cell models of disease, in muscle biopsies, and in RNA-seq data that were accessed through data mining. GAPDH expression was used as internal reference (Figure 1B and C). Further, Western blot quantification of total levels of MSI2 in control and MD1 cells confirmed these results, as shown in Figure 1D.

The upregulation of MSI2 and downregulation of miR-7 was further confirmed in MD1 samples, as shown in Figure 2, where the relative expression of miR-7, MSI2 transcripts and MSI2 protein levels where quantified in patient-derived biopsies (Figure 2 A), primary myoblasts (Figure 2 B) and TDM (Figure 2 C). In all cases, significant lower levels of miR-7 in MD1 samples were detected. When the levels of MSI2 at both transcript and protein levels were studied, a MSI2 upregulation in muscle biopsies was found, but this upregulation was spectacularly increased in TDMs (near 60 times at protein levels when compared MD1 TDM cells to control cells).

Figure 2G shows MSI2 transcripts per million in RNA-seq experiments from biopsies from 40 MD1 patients and 10 controls according to Wang et al. 2019, where it can be observed that MSI2 presents higher transcripts per million in comparison with control (CNT) biopsies.

Next, MSI2 was immunostained in healthy control (CNT) and MD1 myotubes (TDM), as shown in the confocal images of Figure 2E-F, where it can be observed that MSI2 is overexpressed in MD1 cells.

Lastly, Figure 2D shows the Pearson's correlation between miR-7 relative expression and MSI2 protein levels, which confirmed a negative correlation in biopsies and TDMs consistent with our findings that increased MSI2 in MD1 inhibits mature miR-7 and thus downregulate levels in MD1 (Fig. 1D). This correlation was not observed when primary myoblasts were analysed. To further validate MSI2 overexpression in MD1 and that this alteration was relevant in the disease, publicly available RNA-Seq and ankle dorsiflexion strength (ADS) data from 40 MD1 patients and ten controls (obtained from Wang et al. 2019) were resorted, and it was found that MSI2 was significantly overexpressed (over 35%) in MD1 patients compared to controls in full agreement with results obtained in our own muscle samples. The analysis also supported that MSI2 levels negatively correlated to ankle dorsiflexion strength (Fig. 2H; r=-0.48, P=0.0017). Data according to Wang et al. 2019 [Wang, E. T. et al. Transcriptome alterations in myotonic dystrophy skeletal muscle and heart. Hum Mol Genet 28, 1312-1321, doi:10.1093/hmg/ddy432 (2019)]

In view of these results, the inventors found a tight inverse correlation between miR-7 and MSI2: the more MSI2 the less miR-7.

### EXAMPLE 4

Next, the inventors evaluated potential targets within the transcript sequence of MSI2 (SEQ ID NO: 1) that could be targeted by oligonucleotides or siRNA candidates, with the aim of impairing the function or activity or levels of the MSI2 transcript, and eventually reducing the activity or the protein levels of MSI2. First, in order to stablish potential regions that could be targeted, the inventors performed a bioinformatics analysis that consisted in selection of highly functional siRNA using the siRNA design software, siDirect 2.0 siDirect2.0 (http://siDirect2.RNAi.jp/). This software advocates the Ui-Tei rules, which satisfy the following four conditions simultaneously: (1) A or U at position 1 from 5' terminus of siRNA guide strand, (2) G or C at position 19, (3) AU richness (AU ≥4) in positions 1-7, and (4) no long GC stretch > 10. To avoid seed-dependent off-target effects, selecting the siRNAs with low Tm of the seed-target duplex should minimize seed-dependent off-target silencing. The Tm of 21.5°C may serve as the benchmark, which discriminates the almost off-target-free seed sequences from the off-target-positive ones. In the third step, siRNAs that have near-perfect matches to any other non-targeted transcripts were eliminated.

The analysis resulted in several target regions (SEQ ID NO: 3 to 37) within the MSI2 transcript that could be potentially targeted by oligonucleotide candidates, and it was found that these target regions are mainly concentrated between the nucleotides 327-1618 (SEQ ID NO: 2) of the MSI2 full transcript.

In view of this, the inventors designed several oligonucleotides comprised within this region (nucleotides 327-1618 (SEQ ID NO: 2)) but also one oligonucleotide that falls outside this region (from nucleotides 1618 to the end of the transcript), in order to test their effect in reducing MSI2 function. Thus, the oligonucleotides were designed and synthetized to be tested *in vitro.* Two gapmer antisense oligonucleotides (ASO1 and ASO3, SEQ IDS NO: 38 and 39, respectively) against MSI2 transcript were designed to trigger RNAse-H mediated degradation of the target where they bind. ASO1 falls within the region detected by the bioinformatics analysis, while ASO3 hybridized outside this region

Initially, ASOS' toxicity was assayed in control TDMs and it was observed that they present low toxicity as near 1000 nM the cell viability was still higher than 50%. Two different concentrations were used in subsequent experiments: 30 nM, at which around 90% of cells were viable, and 150 nM, at which between 70%-80% of the cells were viable when transfected with any of the two ASOs (Fig. 3 A). To confirm the activity of the ASOs, *MSI2* relative expression was measured upon transfection in DM1 TDMs. A significant reduction of MSI2 levels were observed with ASO1 and 3, but ASO1 was better at both tested concentrations. Similar results were observed quantifying MSI2 protein levels (Fig. 3B and C). *miR-7* was derepressed in ASO-treated MD1 cells (Fig. 3 D). The effect of reduced MSI2 levels on P21 and TGFBR1 targets was also studied (Fig. 3 E). P21 was strongly derepressed in muscle cells treated with ASO3 at the lowest concentration, while *TGFBR1* behaved the opposite at 150 nM of ASO1. After seven days of differentiation, TDMs were stained with an antibody against Desmin. Quantification of fusion capacity and myotube diameter showed that reducing *MSI2* levels in DM1 TDMs restored the fusion index dramatically, in a dose-dependent manner in the case of ASO3, and the size of the myotubes (Fig. 3F and G). Figure 3H, I and J show representative confocal images of Desmin-immunostained human fibroblasts transdifferentiated for 7 days after ASOs transfection into MD1 cells and their respective scramble control at 150 nM.

Thus, as shown in Figure 3, both ASO1 and 3 significantly lowered the amount of MSI2 transcripts in MD1-derived myoblasts at two concentrations (30 nM and 150 nM). The inventors quantified a reduction in 27% of MSI2 transcript after 150 nM ASO1 treatment, and in 46% when cells were transfected with 30 nM ASO3, that were the conditions at which the highest effect was observed.

Concomitant with reduced MSI2 levels, a strong rescue in two atrophy-related myotube parameters was found: the fusion index (percentage of nuclei within myotubes, indicating a normal or pathogenic ability to fuse of MD muscle cells) and the myotube diameter, which are very low in MD1 cells and the treatment brings back to close to normal levels.

### Reducing MSI2 boosts MBNL1 levels in DM1 TDMs.

Considering the key role of MBNL and CELF1 proteins in the pathogenesis of DM1 and previous results where inhibition of autophagy in DM1 promoted MBNL1 activity, we quantified MBNL1 and MBNL2, and CELF1 levels, after ASO treatments. Our data show that when DM1 muscle cells were treated with ASOs at 150 nM, MBNL1 expression was enhanced, achieving levels 50% higher than those obtained in cells treated with scrambled ASO (Fig. 4 A). In contrast, MBNL2 and CELF1 levels remained unchanged after ASOs treatment (Fig 4B-C). These results were confirmed by immunofluorescence staining to detect MBNL1 and MBNL2 in ASO-treated DM1 TDMs. For MBNL1, a low concentration of ASOs did not change the intensity or the pattern of the signal compared to scrambled-treated controls but at the high ASO concentration, a boost in the green signal became evident both in the cytoplasm and in the cell nucleus (Fig 4D-I). An antibody against MBNL2 showed results consistent with the western blot quantification, except for a slight increase with 150 nM ASO1, although the signal was very weak given the low expression of MBNL2 (Fig. 4J-O). To confirm that the additional protein detected was functional, we studied splicing defects regulated by MBNL proteins (Fig. 4P-R). ASO-induced MBNL1 increase was sufficient to reduce the inclusion of pyruvate kinase (PK) exon 10 (fetal PKM2 isoform) in all the experimental conditions evaluated. The inclusion of SERCA1 exon 22 (ASO3) or NFIX exon 7 (both tested ASOs) further confirms the relevance of the extra MBNL1 detected.

Further, cells were transfected with a combination of two siRNAs (SEQ ID NO: 40 and 43) targeting *MSI2* transcripts. The results showed that the siRNAs similarly reduce MSI2 relative expression (Fig. 5 A), enhances miR-7 relative expression (Fig. 5 B), and rescues the fusion index in patient cells, as shown in Figure 5C-E.

Thus, it is shown herein that oligonucleotides directed to target several regions within the SEQ ID NO: 1 (full transcript), but especially within the region of SEQ ID NO: 2 (partial transcript, nucleotides 327-1618) of MSI2, are able to reduce the levels of MSI2 mRNA, causing an increase in the intracellular levels of miR-7. Overall, the level of silencing achieved with ASOs was higher than that obtained with siRNAs, but both oligonucleotides led to significant changes in direct MSI2 targets.

### EXAMPLE 5

Additionally, other inhibitors and strategies were tested to reduce MSI2 levels with mechanisms very different to those presented in Figures 3 - 5. In this case, the small molecule Ro-08-2750 that has been shown to bind directly and selectively to MSI2 and competes for its RNA binding was used (Figure 6). Further, another mechanism was explored to achieve MSI2 reduction, which consisted in the treatment of muscle cells with an oligonucleotide that mimics miR-107, named AgomiR-107 (SEQ ID NO: 41). miR-107 regulates negatively MSI2 by directly binding to its 3'UTR region (Figure 7). The inventors observed that MSI2 levels were significantly reduced at transcript and protein levels when cells were treated with the small molecule Ro-08-2750 at a concentration of 10 µM or the agomiR (Figure 6A and G and 7A and G). Downregulation of MSI2 correlated with significant derepression of miR-7 (Figure 6B and 7B) and downregulation of autophagy markers (Figure 6C, D, H-J and Figure 7C, D, H-J) and autophagic flux (Fig 6K and Figure 7K). Next, expression levels of P21 and TGFBR1, two direct targets of MSI2 were analysed and it was found that treatment with the small molecule was sufficient to reduce TGFBR1 levels but not to produce a significant effect on P21 (Figure 6E and F). However, addition of agomiR-107 resulted in significant modulation of both targets (Figure 7E and F).

Another parameter analysed was the fusion capacity by Desmin immunostaining of the cells after treatment with Ro-08-2750 or agomiR-107 (SEQ ID NO: 41). It was found that the fusion index was significantly improved after MSI2 reduction with any of the treatments (Figure 6 L and M and Figure 7L and M).

### EXAMPLE 6

The increase in intracellular miR-7 expression in the MD treated cell with the inhibitor at different concentrations was quantified. Table 5 below shows the fold-change increase of miR-7 expression in comparison to reference MD1 cells.

**Table 5: Increase of miR-7 levels in treated cells.**

| **FC *miR-7* expression respect to MD1** | **Low concentration** | **High concentration** |
|---|---|---|
| ASO 1(30 and 150 nM) | 1.49 | 1.72* |
| ASO 3 (30 and 150 nM) | 1.68* | 2.25* |
| Ro 08-2750 (10 µM) | 2.52* | |
| siRNA (100 nM) | 3.3 | |

A significant miR-7 increase relative to MD1 cells is observed when treating MD1 cells with ASO3 at the lowest concentration (30 nM). Under these conditions, a significant 1.68-fold increase (68%) in miR-7 was obtained with respect to MD1 cells treated with the scramble sequence control at 30 nM. Importantly, this increase was sufficient to significantly rescue the fusion capacity of the MD1 myotubes, both fusion index and diameter, and some gene splicing events related to the disease (PKM2, SERCA and N-FIX), as shown in Figs. 3 and 4.

Further, the reduction in MSI2 that leads to a lower increase in miR-7 but that does have a positive effect on MD1 cells is the treatment with ASO1 at a concentration of 30 nM. In this case, a 1.49-fold (49%) increase in miR-7 levels was obtained, which translated into a significant rescue in the fusion capacity (fusion index and diameter) (Fig. 3). Lastly, in the case of the small molecule Ro 08-2750 used at 10 µM, the increase in intracellular miR-7 levels achieved is of 2.52 times (252%). Phenotype rescues are observed in terms of the fusion index and diameter of the MD1 myoblasts after treatment with the inhibitors, as shown for ASOs in Fig. 3 or for small molecule Ro 08-2750 in Fig. 6.

Altogether, these results indicate that the use of inhibitors of MSI2 function of different nature (oligonucleotides, small compounds) are able to rescue the phenotype of treated cells. The effects of these inhibitors can be measured by the increase in miR-7 levels in comparison to MD1 untreated cells, where an increase of about 50% already produces a phenotype change in MD1 cells in terms of the fusion index and diameter.

### EXAMPLE 7

Finally, miR-7 levels in different tissues (quadriceps, gastrocnemius, diaphragm) were measured in a MD1 mouse model, known as HSA^{LR} and, surprisingly, no significant differences were found in comparison to control mice (FVB) (Figure 9 C) MSI2 levels were quantified (RNA and protein), and it also did not display significant alterations compared to normal controls (Figure 9A and B). Also, consistent with the hypothesis that overactivation of autophagy causes muscle atrophy, it was observed that the activity of this pathway was normal in HSA^{LR} model mice, which was assessed by quantification at the protein level of the ratio LC3I/LC3II, Atg4a, Atg7, and P62 (see Figure 9 D).

Thus, combining patient-derived cell data and *in vivo* observations, it was hypothesized that the ultimate reason why the HSA^{LR} model mice fails to show strong muscle degeneration is its failure to increase MSI2 protein levels. Therefore, if artificially overexpressed, MSI2 may interfere with miR-7 biogenesis, and reduced levels of mature miR-7 will release repression of several autophagy-related genes, which in turn will enhance muscle catabolism beyond normal homeostasis and lead to muscle degradation. Considering this hypothesis, a pilot study was carried out with a reduced number of animals in which the inventors overexpressed murine Msi2 by using AAV. The tested doses were 1x10¹² and 1.75x10¹² vg/mouse and animals were sacrificed at 6 and 10 weeks after the infection. Significantly increased Msi2 levels were observed in mice treated at the highest dose and sacrificed 10 weeks after the injection (Figure 8A and B). Moreover, miR-7 was reduced in this experimental group (Figure 8 C). At the functional level, forelimb grip strength test was performed before starting the treatment, in an intermediate time point, and after euthanasia (Figure 8 D). It was found that mice overexpressing Msi2 showed reduced force in their forelimbs in a dose and time-dependent manner. Additionally, stained cryosections of quadriceps with WGA (wheat germ agglutinin) were performed to visualize cell membranes and quantify the cross-sectional area of the muscle fibers (Figure 8E, F). Data showed that there was not a significant difference between control mice (FVB) and model (PBS). However, the area was strongly reduced in MD1 mice treated with the highest AAV dose. Similar results were observed when the analysis was performed on gastrocnemius. Finally, RT-qPCR in treated muscle samples revealed increased levels of autophagy-related genes Atg7 and Atg4 (Figure 8 G) suggesting increased activity of this pathway. Moreover, treated mice showed overexpression of the atrogenes Fbxo32 (Atrogin-1) and Trim63 (MuRF1), thus indicating increased activity of the UPS system.

Table 6 below shows the fold change values in mice treated with AAV versus control mice caused by the overexpression of MSI2 by the AAV, showing that the overexpression of MSI2 induces a reduction in miR-7 and an increase in TGFBR1 levels.

**Table 6: Fold change values in mice treated with AAV versus control mice**

| | | **AAV GASTROCNEMIUS** | **AAV QUADRICEPS** |
|---|---|---|---|
| **MSI2** | RNA | 1.31 | 1.96 |
| | Protein | 1.51 | 1.56 |
| **Mir-7** | | 0.78 | 0.7 |
| **TGFBR1** | RNA | 1.22 | 1.43 |
| **CSA (% reduction)** | | 45.44% | 35.66% |

Taken together these results reveal that MSI2 overexpression is sufficient to trigger activation of molecular mechanisms that contribute to muscle atrophy in MD1.

## Claims

1. An inhibitor of RNA-binding protein Musashi (MSI2) function or an analogue thereof for use in the treatment of myotonic dystrophy in a mammal, wherein the inhibitor is capable of reducing in a myotonic dystrophy (MD) cell or in a cell with the phenotype of a MD cell, at least one of
i) MSI2 protein activity,
ii) intracellular transcript levels of MSI2, and/or
iii) intracellular protein levels of MSI2,
thereby causing in the same MD cell an increase in the intracellular miR-7 levels of at least 1.5-fold of the intracellular miR-7 levels of a reference cell, as measured by a gene expression quantification technique such as quantitative reverse transcription PCR (RT-qPCR), Northern blotting, or RNA-sequencing (RNA-Seq) techniques,
wherein the cell with the phenotype of a MD cell is selected from the group of muscular cells, nervous cell, or cardiac cells, and
wherein the reference cell is immortalized skin fibroblasts expressing conditional MyoD.

2. The MSI2 inhibitor for use according to claim 1, wherein the increase in the intracellular miR-7 level is of at least 1.7-fold of the intracellular miR-7 levels of a reference cell.

3. The MSI2 inhibitor for use according to any of claims 1 and 2, wherein the MD cell or a cell with the phenotype of a MD cell is a myocyte.

4. The MSI2 inhibitor for use according to any of claims 2 and 3, wherein the increase in the intracellular miR-7 level is of at least 1.7-fold of the intracellular miR-7 levels of a reference cell and wherein the MD cell or a cell with the phenotype of a MD cell is a myocyte.

5. The MSI2 inhibitor for use according to any of the claims 1 to 4, wherein said inhibitor is for use in the treatment of myotonic dystrophy type 1.

6. The MSI2 inhibitor for use according to any of claims 1 to 5, wherein the mammal is a human being.

7. The MSI2 inhibitor for use according to claims 1 to 6, wherein the inhibitor reduces the intracellular transcript levels of MSI2, and wherein the inhibitor is an oligonucleotide molecule which comprises a fragment composed of a succession of nucleotide units, wherein the oligonucleotide is selected from the group consisting of small interfering RNAs, antisense oligonucleotides, gapmers, morpholino oligomers, FANA oligonucleotides, agomiRs, miRNA mimics, antagomiRs, blockmiRs, PNAs, LNAs (locked nucleic acid antisense oligonucleotides), splice-switching oligonucleotides (SSOs), LNA-based splice-switching oligonucleotides (LNA SSOs), LNA/DNA mixmers or miRNA sponges.

8. The oligonucleotide molecule for use according to claim 7, wherein said oligonucleotide comprises at least 7 consecutive nucleotides in length that have at least 90% identity over the complementary sequence of the full length sequence of SEQ ID: 1 or, preferably, SEQ ID: 2.

9. The oligonucleotide molecule for use according to claim 8, wherein said oligonucleotide comprises at least 10-25 nucleotides in length, from which at least 7 consecutive nucleotides in length have at least 90% identity over the complementary sequence of the full length sequence of SEQ ID: 1 or, preferably SEQ ID: 2.

10. The oligonucleotide molecule for use according to any of previous claims, wherein said oligonucleotide comprises at least 10-25 nucleotides in length, from which at least 7 consecutive nucleotides in length have at least 90% identity over the complementary sequence of the full sequence of any of the oligonucleotides of SEQ ID NO:3 to SEQ ID NO:37.

11. The oligonucleotide molecule for use according to any of previous claims, wherein said oligonucleotide comprises at least 10-25 nucleotides in length, from which at least 7 consecutive nucleotides in length have at least 95% identity over the full sequence of any of the oligonucleotides SEQ ID NO: 38 to 43.

12. The MSI2 inhibitor for use according to any of claims 1 to 6, wherein the inhibitor reduces the activity of MSI2 protein, and wherein the inhibitor is a molecule such as 2,3,4,10-Tetrahydro-7,10-dimethyl-2,4-dioxobenzo[g]pteridine-8-carboxaldehyde, analogues and derivatives thereof.

13. A pharmaceutical **composition** comprising an inhibitor as defined in any of claims 1 to 12, or a mixture of two or more of them, optionally further comprising a carrier and/or one or more pharmaceutically acceptable excipients.

14. A pharmaceutical composition according to claim 13, wherein the pharmaceutical composition comprises an expression vector comprising the oligonucleotide sequence of at least one of the oligonucleotide molecules as defined in any of the claims 1 to 11.

15. A pharmaceutical composition according to any of claims 13 and 14, for use according to any of the claims 1 to 12.
